# EUROPEAN PATENT SPECIFICATION

(11) **EP 2 818 551 B1**
(45) Date of publication and mention of the grant of the patent: **05.04.2017**
(21) Application number: 14174763.4
(22) Date of filing: 27.06.2014
(51) Int. Cl.: C12P 7/16, C12P 7/18, C12N 15/77, C12N 1/00

(54) **Corynebacterium, in which a gene encoding CoA independent succinate semialdehyde dehydrogenase and a gene encoding L-lactate dehydrogenase are attenatued and method of producing C4 compounds, e.g. 4-hydroxybutyrate**
Corynebakterium, in dem die für Koenzym A unabhängige Succinat-Semialdehyddehydrogenease und L-Laktat Dehydrogenase kodierenden Gene attenuiert sind und ein Verfahren zur Herstellung von C4-Verbindungen, z.B. 4-Hydroxybutyrat
Corynebacterium, dans lequel le gène succinate-semialdehyde déshydrogénase indépendant de CoA et le gène L-lactate déshydrogénase sont supprimés et un procédé pour produire des composés C4, par exemple le 4-hydroxybutyrate

(30) Priority: 28.06.2013 KR 20130075940; 30.07.2013 KR 20130090441
(43) Date of publication of application: 31.12.2014
(73) Proprietor: Samsung Electronics Co., Ltd, Gyeonggi-do 443-742 (KR)
(72) Inventor: Rhee, Hong-soon, 443-803 Gyeonggi-do (KR); Lee, Young-min, 443-803 Gyeonggi-do (KR); Kim, Jin-woo, 443-803 Gyeonggi-do (KR); Park, Eun-young, 443-803 Gyeonggi-do (KR); Song, Yeo-ju, 443-803 Gyeonggi-do (KR); Park, Jin-hwan, 443-803 Gyeonggi-do (KR); Cho, Kwang-myung, 443-803 Gyeonggi-do (KR)
(74) Representative: Grünecker Patent- und Rechtsanwälte PartG mbB

(56) References cited:
- WO-A1-2009/023493
- WO-A1-2012/177943
- WO-A2-2009/031766
- US-A1- 2014 030 781

## Description

### BACKGROUND

### 1. Field

The present disclosure relates to microorganisms and methods of producing metabolites such as C4 compounds by using the same.

### 2. Description of the Related Art

Bioplastic is a polymer synthesized by using biomass as a raw material, which is derived from a renewable plant instead of a conventional fossil fuel. Since the biomass is a raw material that requires carbon dioxide in the air during a biosynthesis by a photosynthesis process, bioplastic is a very useful material to reduce carbon emission. Bioplastic replaces fossil fuel, reduces carbon dioxide, and does not cause environmental problems, and thus, bioplastic may be widely used in the low carbon green growth industry.

The biodegradable polymer material is being suggested as an alternative material that may replace a synthetic polymer material. 4-hydroxybutyrate shows a wide range of properties from crystalline plastic to highly elastic rubber and thus, lots of research is being conducted into a microorganism degradable plastic. 4-hydroxybutyrate may be converted into 1,4-butanediol and 1,4-butanediol is used in the whole chemical industry such as in the synthesis of a polymer, a solvent, and an intermediate material used in fine chemistry. However, considering the increase in production cost according to the increase in the price of oil, there is a need for a biological production method that may supplement or substitute chemical processes.

To this end, a method of producing C4-compound such as 4-hydroxybutyrate by using a transformed microorganism and a highly concentrated chemical material converted therefrom is

WO 2009/031766 discloses a mutant microorganism with a high production of 1,4-butanediol. The mutant may contain an inactive lactate dehydrogenase. The mutant may contain the genes encoding succinyl-CoA transferase (Cat1), succinate semialdehyde dehydrogenase (SucD), 4-hydroxybutyrate dehydrogenase (4hbD or GHB) and an alcohol dehydrogenase. Moreover, the mutant may have an inactive gene encoding a succinic semialdehyde dehydrogenase (GabD). The genes encoding for Cat1, SucD and 4hbD may be introduced into the mutant microorganism on an expression vector.

WO 2009/023493 discloses that in a non-naturally occurring microorganism which is deficient in both succinate to hydroxybutyrate pathways (i.e. deficient in both CoA-dependent and CoA-independent succinate semialdehyde dehydrogenase), expressible nucleic acids encoding CoA-dependent succinate semialdehyde dehydrogenase and 4-hydroxybutanoate dehydrogenase are to be exogeneously expressed in the host microorganism.

WO 2012/177943 discloses a strain which contains a deletion in IdhA and contains the genes, E.coli sucCD, P.gingivalis sucD, 4hbd and Cat2 on a plasmid. D3 also discloses a strain, which is designed for the production of 4-HB. In ECKh-761, the genes sad and gabD which encode succinate semialdehyde dehydrogenase enzymes have been deleted. In addition, ECKh-761 contains a deleted ldhA gene and contains the P.gingivalis sucD and 4hbd genes integrated into the chromosome

### SUMMARY

The invention is defined by the claims.

### BRIEF DESCRIPTION OF THE DRAWINGS

These and/or other aspects will become apparent and more readily appreciated from the following description of the embodiments, taken in conjunction with the accompanying drawings in which:
FIG. 1 is a cleavage map of a pGSK+ vector;
FIG. 2 is a cleavage map of a pGT1 vector;
FIG. 3 is a cleavage map of a pG3G vector;
FIG. 4 is a cleavage map of a pGSX1 vector;
FIG. 5 is a cleavage map of a pG2G vector;
FIG. 6 shows productivity of 4-hydroxybutyrate (4HB) in B039 pG3G and B039 pG2G;
FIG. 7 shows productivity of 4HB of the strains disclosed in Table 2;
FIG. 8 shows productivity of 4HB and 1,4-BDO in D003, D003 pC2E2, D001, and D001 pC2E2;
FIG. 9 is a cleavage map of a pcat1 vector;
FIG. 10 is a cleavage map of a psucD vector;
FIG. 11 is a cleavage map of a p4hbD vector;
FIG. 12 shows productivity of 4HB in the strains disclosed in Table 4;
FIG. 13 shows productivity of 4HB in D003 and D003 pG2G;
FIG. 14 shows productivity of 4HB according to culturing conditions;
FIG. 15 shows an example of a biosynthetic pathway for producing 4HB from glucose;
FIG. 16 is a cleavage map of a pC2E2 vector.

### DETAILED DESCRIPTION

Provided is a microorganism producing a C4 compound (4-carbon compound), in which the microorganism expresses a CoA-dependent succinate semialdehyde dehydrogenase from a gene in a vector.

The microorganism is from the *Corynebacterium* genus. The *Corynebacterium* genus microorganism may be a microorganism selected from the group consisting of *Corynebacterium glutamicum, Corynebacterium thermoaminogenes, Brevibacterium flavum*, and *Brevibacterium lactofermentum.*

The CoA-dependent succinate semialdehyde dehydrogenase may be an enzyme having catalytic activity for converting succinyl-CoA into succinic semialdehyde. The enzyme may be categorized as EC.1.2.1.b. For example, the enzyme may be SucD. SucD may have an amino acid sequence of SEQ ID NO: 1 derived from *Porphyromonas gingivalis.* A polynucleotide coding for SucD may code for an amino acid sequence of SEQ ID NO: 1. The polynucleotide coding for SucD may have a nucleotide sequence of SEQ ID NO: 2 derived from *Porphyromonas gingivalis.*

The term "vector" refers to a DNA construct including a DNA sequence that is operably linked to a suitable regulatory sequence that may express DNA in a suitable host. The vector is physically separated from chromosomal DNA (may also be referred to as 'genomic DNA' hereinafter) of a subject and in some cases, the vector may be replicated independently from the chromosome. The vector may include a plasmid vector, a bacteriophage vector, and a cosmid vector. To operate as a vector expressing a gene, the vector may include an origin of replication, a promoter, a multiple cloning site (MCS), and a selection marker. The origin of replication enables the vector to be replicated separately from a chromosome of a host cell, the promoter acts in transcription of a gene to be inserted, and the MCS provides various restriction sites in which exotic genes may be inserted. The selection marker acts to confirm the insertion of a vector into a host cell. The selection marker includes an antibiotic resistant gene that is generally used in the art. The selection marker may have a resistance gene for, for example, ampicillin, gentamicin, carbenicillin, chloramphenicol, streptomycin, kanamycin, geneticin, neomycin, or tetracycline.

When a prokaryotic cell is used as a host, the vector may include a strong promoter, for example, a lambda PL promoter, a trp promoter, a lac promoter, a T7 promoter, or a tac promoter. When a eukaryotic cell is used as a host, the vector may include a promoter derived from a genome of a mammalian cell, for example, a metalllothionein promoter, or a promoter derived from a mammalian virus, for example, an adenovirus late promoter, a vaccinia virus 7.5K promoter, an SV40 promoter, a cytomegalovirus promoter, and a tk promoter of HSV. The promoter may be a lambda PL promoter, a trp promoter, a lac promoter, a T7 promoter, or a tac promoter. The promoter is operably linked to a sequence coding for a gene.

Also, the promoter is operably linked to a sequence coding for a gene. The expression, "operably linked" refers to a functional linkage between a nucleic acid expression regulatory sequence, for example, a promoter, a signal sequence, an array at a binding location of a transcription factor, a terminator, or an enhancer, and another nucleotide sequence and thus, the regulatory sequence may regulate transcription and/or translation of the nucleotide sequence coding for the gene.

The vector may include a CoA-dependent succinate semialdehyde dehydrogenase gene that is operably linked to a regulatory sequence that is different from the CoA-dependent succinate semialdehde dehydrogenase gene in the chromosome.

The microorganism producing the C4 compound may be a microorganism having inactivated or reduced activity of a pathway for synthesizing lactate from pyruvate. The microorganism may have an inactivated or attenuated gene coding an L-lactate dehydrogenase (LDH). The LDH may have a catalytic activity for converting pyruvate into lactate. The LDH may be an enzyme categorized as EC.1.1.1.27. The LDH may have an amino acid sequence of SEQ ID NO: 3. The gene coding for LDH may be a gene coding for an amino acid sequence of SEQ ID NO: 3.

The term "inactivation" or "inactivated" may mean that a gene is not expressed or a gene is expressed but a product of the expressed gene is not active. The term "attenuation" or "attenuated" may mean that the expression of a gene is decreased to a level lower than an expression level of a wild type strain, a strain which is not genetically engineered, or a parent strain. Alternatively, "attenuation" may mean that a product of the expression of the gene has an attenuated activity. Inactivation and attenuation encompasses deletion of a gene in whole or in part. The inactivation or attenuation may be performed, for example, by homologous recombination, site directed mutagenesis etc. The inactivation or attenuation may include changing a expression regulatory sequence such as the sequence of promoter, transcription terminator, operator, and the like by a site specific mutation method, or homologous recombination method. The inactivation or attenuation may include change the coding sequence of a gene by a site specific mutation method, or homologous recombination method. The inactivation or attenuation may be caused by insertion, substitution, conversion, or addition of nucleotide(s).The inactivation or attenuation may be performed, for example, by transforming a vector including a part of the sequence of the genes into a cell, culturing the cell so that homologous recombination of the sequence may occur with a homologous gene of the cell, and then using a selection marker to select a cell in which homologous recombination occurred. Due to inactivation or attenuation of the gene, activity of the enzyme coded from the gene may be eliminated or decreased. The term "decreased" may refer to relative activity of a manipulated microorganism compared with an unmanipulated microorganism, i.e. a parent cell.

The microorganism producing a C4 compound may have an inactivated or attenuated gene for a CoA independent succinic semialdehyde dehydrogenase (SSADH). The SSADH may have activity for catalyzing a reaction that converts succinic semialdehyde into succinate. The SSADH may be an enzyme categorized as EC.1.2.1.16 or EC.1.2.1.39. The SSADH may be NAD+ or NADP+ dependent. The SSADH may include an amino acid sequence of SEQ ID NO: 4, SEQ ID NO: 5, or SEQ ID NO: 6. A gene coding for the SSADH may include a nucleotide sequence of SEQ ID NO: 7, SEQ ID NO: 8, or SEQ ID NO: 9. The microorganism may be a microorganism in which the gene coding for the SSADH is inactivated or attenuated.

Also, the microorganism producing a C4 compound in which a CoA-dependent succinate semialdehyde dehydrogenase is expressed from a gene in a vector may additionally include a polynucleotide coding for a 4-hydroxybutyrate dehydrogenase in a vector.

The 4-hydroxybutyrate dehydrogenase may be an enzyme having a catalytic activity for converting succinic semialdehyde into 4-hydroxybutyrate. The enzyme may be an enzyme categorized as EC.1.1.1.a. For example, the enzyme may be 4hbD. The 4hbD may have an amino acid sequence of SEQ ID NO: 10 derived from *Porhyromonas gingivalis.* A polynucleotide coding for 4hbD may code for an amino acid sequence of SEQ ID NO: 10. The polynucleotide coding for the 4hbD may have a nucleotide sequence of SEQ ID NO: 11.

Also, the microorganism may include a polynucleotide coding for a CoA-dependent succinate semialdehyde dehydrogenase and a polynucleotide coding for a 4-hydroxybutyrate dehydrogenase in a chromosome of the microorganism, apart from the CoA-dependent succinate semialdehyde dehydrogenase and the 4-hydroxybutyrate dehydrogenase expressed each gene present in a vector.

Also, the microorganism may additionally include a polynucleotide coding for a succinyl-CoA:CoA transferase. The polynucleotide coding for a succinyl-CoA:CoA transferase may exist in a chromosome or a vector.

The succinyl-CoA:CoA transferase may be an enzyme having an activity for catalyzing a reaction that converts succiinate into succinyl-CoA. The enzyme may be an enzyme categorized as EC.2.8.3.a. For example, the enzyme may be Cat1. The Cat1 may have an amino acid sequence of SEQ ID NO: 12 derived from Clostridium kluyveri. A polynucleotide coding for Cat1 may code for an amino acid sequence of SEQ ID NO: 12. The polynucleotide coding for Cat1 may have a nucleotide sequence of SEQ ID NO: 13.

The polynucleotide coding for the succinyl-CoA:CoA transferase may be located in a chromosome of the microorganism.

Accordingly, the microorganism may include all of the polynucleotide coding for the succinyl-CoA:CoA transferase, the polynucleotide coding for the CoA-dependent succinate semialdehyde dehydrogenase, and the polynucleotide coding for the 4-hydroxybutyrate dehydrogenase in the chromosome. Thus, the microorganism may have the ability to produce 4-hydroxybutyrate (4-HB).

The C4 compound may be an organic acid and may be succinate, fumaric acid, malic acid, or a C4 compound derived therefrom. Also, a material derived from succinate may be 4-hydroxybutyrate (4-HB), 1,4-butanediol (1,4-BDO), y-butyrolactone (GBL), or a C4 compound derived therefrom, but it is not limited thereto.

The microorganism producing the C4 compound may further include various enzymes. The microorganism may further include a polynucleotide coding for a 4-hydroxybutyryl CoA:acetyl-CoA transferase and a polynucleotide coding for an alcohol dehydrogenase. The microorganism including these enzymes may produce 1,4-butanediol.

The 4-hydroxybutyryl CoA:acetyl-CoA transferase may be an enzyme having an activity for catalyzing a reaction that converts 4-hydroxybutyrate into 4-hydroxybutyryl-CoA. The 4-hydroxybutyryl CoA:acetyl-CoA transferase may be an enzyme categorized as EC.2.8.3.a. The enzyme may be Cat2. Cat2 may have an amino acid sequence of SEQ ID NO: 14 derived from *Porphyromonas gingivalis.* The polynucleotide coding for the Cat2 may have a nucleotide sequence of SEQ ID NO: 15.

The alcohol dehydrogenase may be an enzyme having an activity for catalyzing a reaction that converts 4-hydroxybutyryl-CoA into 1,4-butanediol. The alcohol dehydrogenase may be an enzyme categorized as EC.1.1.1.c. The enzyme may be AdhE2. The AdhE2 may have an amino acid sequence of SEQ ID NO: 16 derived from *Clostridium acetobutylicum.* The polynucleotide coding for the AdhE2 may have a nucleotide sequence of SEQ ID NO: 17.

According to another embodiment, provided is a method of producing the C4 compound, the method including: culturing the microorganism to obtain a culture and recovering the C4 compound from the culture.

The microorganism may be a microorganism producing the C4 compound, in which the CoA-dependent succinate semialdehyde dehydrogenase is expressed from a gene in a vector is as described above. Also, the microorganism may additionally include the 4-hydroxybutyrate dehydrogenase in the vector. Also, the microorganism may include a polynucleotide coding for a CoA-dependent succinate semialdehyde dehydrogenase and a polynucleotide coding for a 4-hydroxybutyrate dehydrogenase in a chromosome, apart from the CoA-dependent succinate semialdehyde dehydrogenase and the 4-hydroxybutyrate dehydrogenase expressed in a vector. Also, the microorganism may further include a polynucleotide coding for the 4-hydroxybutyryl CoA:acetyl-CoA transferase and a polynucleotide coding for the alcohol dehydrogenase.

The culturing may be performed in a suitable culture medium and culturing conditions known in the art. One of ordinary skill in the art may easily adjust the culture medium and the culturing conditions according to the selected microorganism. A culturing method may include a batch mode, a continuous mode, a fed-batch mode, or a combination mode thereof.

The culture medium may include various carbon sources, nitrogen sources, and trace element components.

The carbon source may include a carbohydrate such as glucose, sucrose, lactose, fructose, maltose, starch, and cellulose; a lipid such as soybean oil, sunflower oil, castor oil, and coconut oil; a fatty acid such as palmitic acid, stearic acid, and linoleic acid; an alcohol such as glycerol and ethanol; an organic acid such as acetic acid; or a combination thereof. The culturing may be performed by using glucose as a carbon source. The nitrogen source may include an organic nitrogen source such as peptone, yeast extract, meat extract, malt extract, corn steep liquid, and soybean; an inorganic nitrogen source such as urea, ammonium sulfate, ammonium chloride, ammonium phosphate, ammonium carbonate, and ammonium nitrate; or a combination thereof.

The culture medium may include as a phosphorous source, for example, potassium dihydrogen phosphate, dipotassium phosphate, a sodium-containing salt corresponding to potassium dihydrogen phosphate, and dipotassium phosphate, and a metal salt such as magnesium sulfate and iron sulfate. The culture medium or an individual component may be added to the culture in a batch mode or a continuous mode.

In addition, the pH of the culture may be adjusted during the culturing by adding a compound such as ammonium hydroxide, potassium hydroxide, ammonia, phosphoric acid, or sulfuric acid to the culture medium in an appropriate mode. In addition, bubble formation may be repressed by using an anti-foaming agent such as fatty acid polyglycol ester.

The microorganism may be cultured under anaerobic or microaerobic conditions. The wording "microaerobic condition" refers to a state in which the oxygen concentration in the culture medium is lower than that obtained when the culture medium is contacted with the normal atmospheric air. For example, a low level of oxygen can be less than about 10%, 5%, 1%, 0.1%, or 0.01 %, about 0.01% to about 10%, about 0.01% to about 5%, about 0.01% to about 1%, about 0.01% to about 0.1 %, about 0.1 % to about 10%,_about 1% to about 9%, about 2% to about 8%, about 3% or 7%, or about 4% to about_6% of the oxygen level in the culture medium obtained when the culture medium allowed to be contacted with the normal atmospheric air.

The microaerobic condition may be formed, for example, by supplying carbon dioxide or nitrogen at a flow rate range from about 0.1 vvm (aeration volume/medium volume/minute) to about 0.4 vvm, from about 0.2 vvm to about 0.3 wm, or at a flow rate of about 0.25 vvm. Also, the anaerobic or micraerobic condition may have an aeration rate of about 0 vvm (i.e., anaerobic) to about 0.4 vvm, about 0.1 vvm to about 0.3 vvm, and about 0.15 vvm to about 0.25 vvm.

The method includes recovering the produced C4-compound from the culture. The produced C4 compound may be succinate, fumaric acid, malic acid, or a C4 compound derived therefrom. According to an embodiment of the present invention, the microorganism has an enhanced ability to produce the C4 compound of TCA cycle and materials derived therefrom. Examples of succinate derived material may include 4-HB, 1,4-BDO, GBL, or a C4 compound derived therefrom.

Recovering the 4-hydroxybutyrate may be performed by a well-known separation and purification method. The recovering may be performed by a well-known separation and purification method. The recovering may be performed by centrifugation, ion exchange chromatography, filtration, precipitation, or a combination thereof.

The recovered produced C4 compound may be converted into another organic compound to further include a process of obtaining various C4 compounds. The 4-hydroxybutyrate produced according to the method described above may be chemically converted to synthesize a substrate that is structurally related thereto. 4-hydroxybutyrate may be incubated in the presence of a strong acid at a temperature of about 100 °C to about 200 °C and then distilled to obtain GBL. The obtained GBL may be converted into N-methylpyrrolidone (NMP) through an amination reaction by using an amination agent, for example, methylamine. Also, the obtained GBL may be selectively converted into tetrahydrofuran (THF), 1,4-butanediol, or butanol through hydrogenation by using a metal-containing catalyst, for example, ruthenium (Ru) or palladium (Pd).

Also, the produced 4-hydroxybutyrate may be biologically converted to obtain poly-4-hydroxybutyrate. The biological conversion may be caused by a polyhydroxyalkanoate synthase, 4HB-CoA:CoA transferase, or a combination thereof.

Provided is a microorganism in which a gene coding for CoA independent SSADH is inactivated or attenuated. The gene coding for CoA independent SSADH may be located in the chromosome.

The Corynebacterium genus microorganism may be a microorganism selected from the group consisting of *Corynebacterium glutamicum, Corynebacterium thermoaminogenes*, *Brevibacterium flavum, and Brevibacterium lactofermentum.*

The CoA independent SSADH may have a catalytic activity that converts succinic semialdehde into succinate. The SSADH may be an enzyme categorized as EC.1.2.1.16 or EC.1.2.1.39. The SSADH may be NAD+ dependent or NADP+ dependent. The SSADH may be a polypeptide having an amino acid sequence of SEQ ID NO: 4, SEQ ID NO: 5, or SEQ ID NO: 6. A gene coding for the SSADH may have a nucleotide sequence of SEQ ID NO: 7, SEQ ID NO: 8, or SEQ ID NO: 9.

The microorganism may have at least one inactivated or attenuated polynucleotide selected from a polynucleotide having a nucleotide sequence of SEQ ID NO: 7, a polynucleotide having a nucleotide sequence of SEQ ID NO: 8, and a polynucleotide having a nucleotide sequence of SEQ ID NO: 9. At least one selected from a polynucleotide having a nucleotide sequence of SEQ ID NO: 7 and a polynucleotide having a nucleotide sequence of SEQ ID NO: 8 may be inactivated or attenuated.

The microorganism may be devoid of CoA-independent SSADH activity or may have reduced CoA-independent SSADH activity due to inactivation or attenuation of the gene. The expression "decreased" refers to the activity of a manipulated microorganism in comparison with the activity of an unmanipulated organism, i.e., parent organism.

The microorganism may have an inactivated or attenuated pathway for synthesizing lactate from pyruvate. The microorganism may be devoid of or have decreased activity of L-lactate dehydrogenase (LDH). The LDH may have a catalytic activity for converting pyruvate into lactate. The LDH may be an enzyme categorized as EC.1.1.1.27. For example, the LDH ma have an amino acid sequence of SEQ ID NO: 3. A gene coding for the LDH in the microorganism may be inactivated or may have attenuated activity.

The microorganism may include at least one selected from a polynucleotide coding for succinyl-CoA:coenzyme A transferase (Cat1), a polynucleotide coding for acetyl-CoA hydrolase (ActA and ActB), and a polynucleotide coding for CoA-dependent succinate semialdehyde dehydrogenase (SucD).

The Cat1, ActA, or ActB may have a catalytic activity for converting succinate into succinyl-CoA. The Cat1 may be an enzyme classified as EC.2.8.3.a. For example, the Cat1 may have an amino acid sequence of SEQ ID NO: 12. The polynucleotide coding for Cat1 may have a nucleotide sequence of SEQ ID NO: 13. The ActA may be an enzyme classified as EC.2.8.3.-. For example, the ActA may have an amino acid sequence of SEQ ID NO: 82. The polynucleotide coding for ActA may have a nucleotide sequence of SEQ ID NO: 83. The ActB may be an enzyme classified as EC.2.8.3.8. For example, the ActB may have an amino acid sequence of SEQ ID NO: 84. The polynucleotide coding for ActB may have a nucleotide sequence of SEQ ID NO: 85.

The SucD may have a catalytic activity for converting succinyl-CoA into succinic semialdehyde. The SucD may be an enzyme classified as EC.1.2.1.b. For example, the SucD may have an amino acid sequence of SEQ ID NO: 1. The polynucleotide coding for SucD may have a nucleotide sequence of SEQ ID NO: 2.

The microorganism may further include a polynucleotide coding for 4-hydroxybutyrate dehydrogenase (4hbD). The microorganism may have 4HB productivity.

The 4hbD may have a catalytic activity for converting succinic semialdehyde into 4HB. The 4hbD may be an enzyme classified as EC.1.1.1.a. For example, the 4hbD may have an amino acid sequence of SEQ ID NO: 10. The polynucleotide coding for 4hbD may have a nucleotide sequence of SEQ ID NO: 11.

The microorganism for producing 4HB may be used for producing a convertible material from 4HB. The convertible material may be gamma-butyrolactone (GBL), 1,4-butanediol, poly-4-hydroxybutyrate, tetrahydrofuran (THF), or N-methylpyrrolidone (NMP).

The microorganism may further include a polynucleotide coding for 4-hydroxybutyrate dehydrogenase, a polynucleotide coding for 4-hydroxybutyryl CoA:acetyl-CoA transferase (Cat2), and a polynucleotide coding for alcohol dehydrogenase (AdhE2). The microorganism may capble of producing 1,4-butanediol.

The Cat2 may have a catalytic activity for converting 4HB into 4-hydroxybutyryl-CoA. The Cat2 may be an enzyme classified as EC.2.8.3.a. For example, the Cat2 may have an amino acid sequence of SEQ ID NO: 14. The polynucleotide coding for Cat2 may have a nucleotide sequence of SEQ ID NO: 15 of nucleotide sequence.

The AdhE2 may have a catalytic activity for converting 4-hydroxybutyryl-CoA into 1,4-butanediol. The AdhE2 may be an enzyme classified as EC.1.1.1.c. For example, the AdhE2 may have an amino acid sequence of SEQ ID NO: 16. The polynucleotide coding for AdhE2 may have a nucleotide sequence of SEQ ID NO: 17.

Provided is a method of producing a C4 compound using the microorganism in which a gene coding for a CoA-independent succinic semialdehyde dehydrogenase is inactivated or attenuated. The microorganism in which a gene coding for a CoA-independent succinic semialdehyde dehydrogenase is inactivated or attenuated is as described above.

According to another embodiment, provided is a method of producing 4HB, the method including: culturing a microorganism producing 4HB to obtain a culture; and recovering 4HB from the culture.

The 4HB producing microorganism is the same as described above.

The culturing may be performed as described above.

The culturing may occur in anaerobic or a microaerobic condition as described above. The recovering of 4HB may be performed by a separation and purification method known in the art. The recovering may be performed by centrifugation, ion-exchange chromatography, filtration, immersion, or a combination thereof.

According to another embodiment, provided is a method of producing 1,4-butanediol, the method comprising: culturing a microorganism 1,4-butanediol to obtain cultured products; and recovering 1,4-butanediol from the culture.

The microorganism producing 1,4-butanediol is the same as described above.

The culturing is the same as described above. The culturing may occur in anaerobic or a microaerobic condition. The microaerobic condition is the same as described above.

The recovering of 1,4-butanediol may be performed by a separation and purification method known in the art. The recovering may be performed by centrifugation, ion-exchange chromatography, filtration, immersion, or a combination thereof.

Hereinafter, the present invention will be described in further detail with reference to examples. It will be obvious to a person having ordinary skill in the art that these examples are illustrative purposes only.

### Example 1: Preparation of a strain with an inactivated L-lactate dehydrogenase gene

### 1.1. Preparation of a replacement vector

An L-lactate dehydrogenase gene (ldh) of *Corynebacterium glutamicum* (*C. glutamicum*, CGL) (ATCC 13032, hereinafter the same) was inactivated through a homologous recombination by using a pK19 mobsacB(ATCC 87098) vector. Two homologous regions for removing the ldh gene were obtained by PCR amplification using a genomic DNA of CGL. The homologous regions were upstream and downstream regions of the ldh gene, each of which was obtained by PCR amplification using a primer set of ldhA_5'_HindIII (SEQ ID NO: 18) and ldhA_up_3'_Xhol (SEQ ID NO: 19) and a primer set of ldhA_dn_5'_Xhol(SEQ ID NO: 20) and ldhA_3'_EcoRI) (SEQ ID NO: 21). The PCR amplification was performed by repeating 30 cycles each including denaturation at a temperature of 95 °C for 30 seconds, annealing at 55 °C for 30 seconds, and extension at 72 °C for 30 seconds. All the PCR amplifications hereinafter were performed under the same conditions. The amplification products were cloned in the Hindlll and EcoRl restriction sites of a pK19 mobsacB vector to prepare a pK19_Δldh vector.

### 1.2. Preparation of a CGL (Δldh) strain

The pK19_Δldh vector was introduced to CGL through electroporation. The introduced strain was streaked on a Luria Brain Heart Infusion Supplemented (LBHIS) culture medium containing 25 *µg*/ml of kanamycin and then cultured at a temperature of 30 °C. The LBHIS culture medium included 18.5 g/L of a brain-heart infusion broth, 0.5 M of sorbitol, 5 g/L of bacto-tryptone, 2.5 g/L of bacto-yeast extract, 5 g/L of NaCl, and 18 g/L of bacto agar. Hereinafter, the composition of the LBHIS agar plate was the same. Colonies formed were streaked on an LB-sucrose culture medium, cultured at a temperature of 30 °C, and only the colonies in which double crossing-over occurred were selected. Genomic DNA was separated from the selected colonies, a primer set of ldhA up (SEQ ID NO: 22) and ldhA down (SEQ ID NO: 23) was used to confirm the deletion of the ldh gene through PCR. As a result, a CGL (Δldh) strain was obtained.

### Example 2: Preparation of a strain for removing a succinic semialdehyde dehydrogenase (ssadh) gene

### 2.1. Preparation of a substitution vector

A succinic semialdehyde dehydrogenase gene (ssadh) of *Corynebacterium glutamicum* was inactivated by homologous recombination using a pK19 mobsacB vector. Homologous regions for removing the ssadh gene, NCgl0049 (SEQ ID NO: 27), NCgl0463 (SEQ ID NO: 28), or NCgl2619 (SEQ ID NO: 29) were obtained by PCR amplification using genomic DNA of *Corynebacterium glutamicum* as a template. Two homologous regions for removing the NCgl0049 gene were upstream and downstream regions of the NCgl0049 gene, which were obtained by PCR amplification using a primer set of 0049-1 for(SEQ ID NO: 24) and 0049-1 rev(SEQ ID NO: 25) and a primer set of 0049-2 for(SEQ ID NO: 26) and 0049-2 rev(SEQ ID NO: 27). The amplification products were cloned in HindIII and Pstl restriction sites of the pK19 mobsacB vector to prepare a pK19_Δn0049 vector. Two homologous regions for removing the NCgl0463 gene were upstream and downstream regions of the NCgl0463 gene, which were obtained by PCR amplification using a primer set of 0463-1 for(SEQ ID NO: 28) and 0463-1 rev(SEQ ID NO: 29) and a primer set of 0463-2 for(SEQ ID NO: 30) and 0463-2 rev(SEQ ID NO: 31). The amplification products were cloned in HindIII and Pstl restriction sites of the pK19 mobsacB vector to prepare a pK19_An0463 vector. Two homologous regions for removing the NCgl2619 gene were upstream and downstream regions of the NCgl2619 gene, which were obtained by PCR amplification using a primer set of 2619-1 for(SEQ ID NO: 32) and 2619-1 rev(SEQ ID NO: 33) and a primer set of 2619-2 for(SEQ ID NO: 34) and 2619-2 rev(SEQ ID NO: 35). The amplification products were cloned in HindIII and Pstl restriction sites of the pK19 mobsacB vector to prepare a pK19_Δn2619 vector.

### 2.2. Preparation of a CGL (Δldh Δn0049) strain, a CGL (Δldh Δ0463) strain, a CGL (Δldh Δn2619) strain, and a B039 strain

The pK19_Δn0049, pK19_Δn0463, and pK19_Δn2619 vectors were introduced separately or altogether to the CGL (Δldh) strain prepared in Example 1 through electroporation. The CGL (Δldh) strain to which the pK19_An0049, pK19_An0463, and pK19_Δn2619 vectors were introduced was streaked in an LBHIS medium containing 25 *µg*/ml of kanamycin to culture the same at a temperature of 30 °C. The colony formed was streaked in an LB-sucrose medium to culture the same at a temperature of 30 °C and only the colonies in which double crossing-over occurred were selected. Genomic DNA was separated from the selected colonies and PCR was performed by using a primer set of 0049for(SEQ ID NO: 36) and 0049rev(SEQ ID NO: 37), a primer set of 0463for(SEQ ID NO: 38) and 0463rev(SEQ ID NO: 39), or a primer set of 2619for(SEQ ID NO: 40) and 2619rev(SEQ ID NO: 41) to confirm the deletion of NCgl0049, NCgl0463, and NCgl2619. Table 1 below shows names and genotypes of the prepared strains.

**[Table 1]**

| Strain name | Genotype |
|---|---|
| CGL (Δldh Δn0049) | ATCC 13032 Δldh ΔNCgl0049 |
| CGL (Δldh Δn0463) | ATCC 13032 Δldh ΔNCgl0463 |
| CGL (Δldh Δn2619) | ATCC 13032 Δldh ΔNCgl2619 |
| B039 | ATCC 13032 Δldh ΔNCgl0049 ΔNCgl0463 ΔNCgl2619 |

### Example 3. Changes in production of 4HB depending on the deletion of a succinic semialdehyde dehydrogenase gene

### 3.1. Preparation of a B039 pG3G strain and evaluation of 4HB productivity

### 3.1.1. Preparation of a pG3G vector

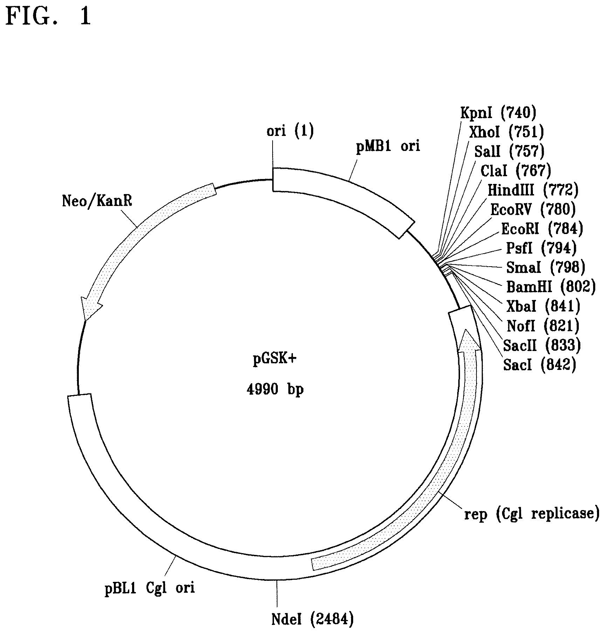

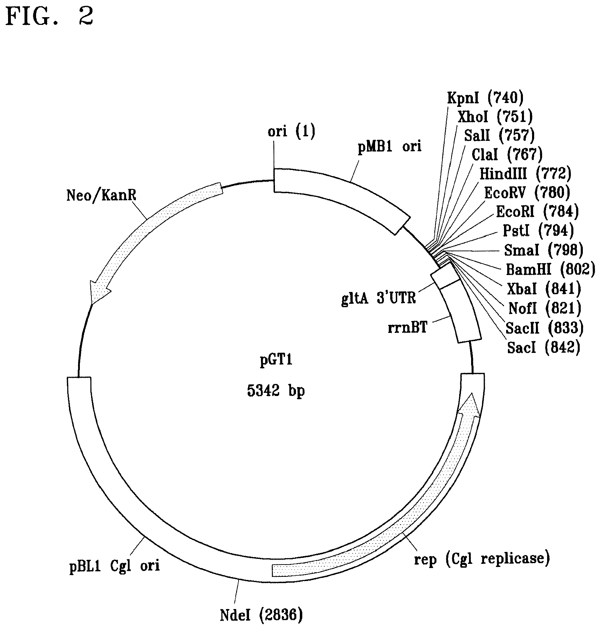

From a pET2 vector (GenBank accession number: AJ885178.1), which is a *E.coli-Corynebacterium glutamicum* shuttle vector, a region disposed between a replication origin of E.coli and a replication origin of *Corynebacterium glutamicum,* which is a region including a chloramphenicol acetyltransferase gene and an rrnB transcription terminator, was removed. Also, an aph gene, which is a kanamycin resistance gene, was substituted with a neo gene (nptll), which is a Tn5-derived kanamycin resistance gene, to remove a restriction site and remove an Xbal restriction site in a replicase (rep) gene located in the replication origin of *Corynebacterium glutamicum.* Thereafter, a multi-cloning site (MCS) derived from a pBlueScript II SK(+) phagemid vector including 14 restriction sites was inserted into the region to prepare a pGSK+ vector(SEQ ID NO: 42). FIG. 1 is a cleavage map of the pGSK+ vector. Based on the pGSK+ vector, 3'UTR and an rrnB transcription terminator of a gltA gene were inserted into a Sall restriction site of pGSK+ to prepare a pGT1 vector (SEQ ID NO: 43). FIG. 2 is a cleavage map of the pGT1 vector.

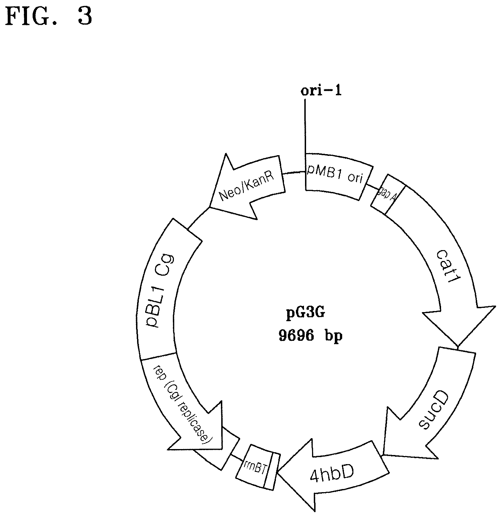

Based on the pGT1 vector, a pG3G, which is an expression vector for cat1, sucD, and 4hbD genes, was prepared. FIG. 3 is a cleavage map of the pG3G vector. A gene cassette (SEQ ID NO: 44), which sequentially includes a gapA promoter, and cat1, sucD, 4hbD, and cat2 genes were prepared. By using the cassette as a template, a sequence between a gapA promoter, which is a promoter for a gapA gene (NCgl1526) derived from Corynebacterium glutamicum, and a 4hbD gene was obtained by PCR amplification using a primer set of 3G up(SEQ ID NO: 45) and 3G down(SEQ ID NO: 46). The amplification product was cloned in Pstl and Xbal restriction sites of the pGT1 vector by using an In-fusion HD cloning kit (#PT5162-1, Clontech).

### 3.1.2. Preparation of a pG2G vector

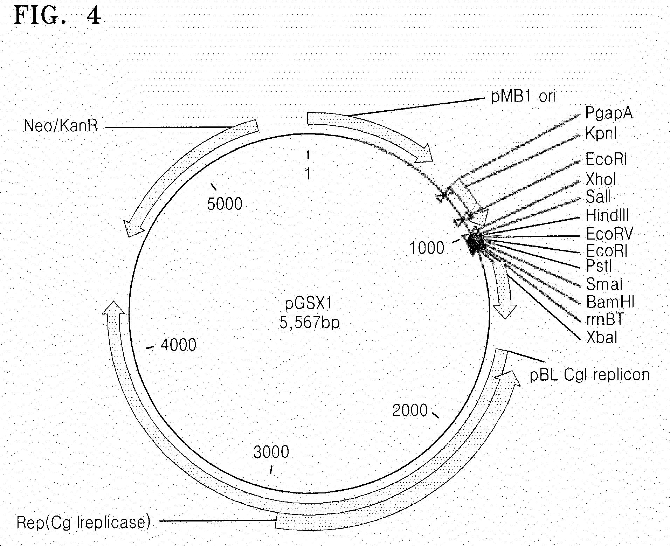

A pGSX1(SEQ ID NO: 47), which is a plasmid vector for expressing cat1, sucD, and 4hbD genes, including a gapA promoter was prepared in the following manner. FIG. 4 is a cleavage map of the pGSX1 vector. The gapA promoter was obtained by PCR amplification using a genomic DNA of *Corynebacterium glutamicum* ATCC 13032 and a primer set of MD-625 (SEQ ID NO: 48) and MD-626 (SEQ ID NO: 49). The amplification product was cloned by using the In-fusion HD cloning kit, such that a 270 bp DNA fragment was disposed in Kpnl and Xhol restriction sites of the pGT1 1 vector.

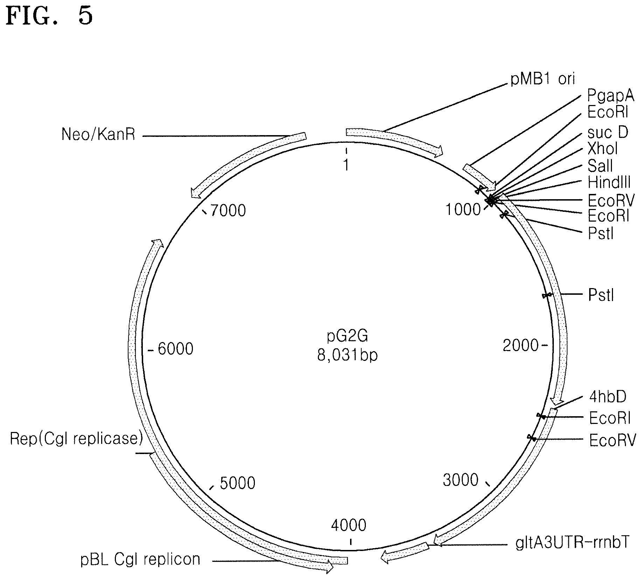

Based on the pGSX1 vector, a pG2G, which is an expression vector for sucD and 4hbD genes, was prepared. FIG. 5 is a cleavage map of the pG2G vector. In the gene cassette, a sucD-4hbD sequence was obtained by PCR amplification using a primer set of sucD-up (SEQ ID NO: 64) and 4hbD-down (SEQ ID NO: 67). The amplification product obtained therefrom was cloned in Pstl and Xbal restriction sites of the pGSX1 vector by using the In-fusion HD cloning kit.

### 3.1.3. Preparation of a strain

The pG2G and pG3G vectors were introduced into the CGL (Δldh) strain or the strain disclosed in Table 1 through electroporation. The introduced strain was streaked in an LBHIS medium containing 25 µg/ml of kanamycin to culture the same at a temperature of 30 °C, and strains having kanamycin resistance were selected. Table 2 below shows names and genotypes of the prepared strains.

**[Table 2]**

| Strain name | Genotype |
|---|---|
| CGL (Δldh pG3G) | ATCC 13032 Δldh pGT1_cat1, sucD, 4hbD |
| CGL (Δldh Δn0049 pG3G) | ATCC 13032 Δldh ΔNCgl0049 pGT1_cat1, sucD, 4hbD |
| CGL (Δldh Δn0463 pG3G) | ATCC 13032 Δldh ΔNCgl0463 pGT1_cat1, sucD, 4hbD |
| CGL (Δldh Δn2619 pG3G) | ATCC 13032 Δldh ΔNCgl2619 pGT1_cat1, sucD, 4hbD |
| B039 pG2G | ATCC 13032 Δldh ΔNCgl0049 ΔNCgl0463 ΔNCgl2619 pGT1_sucD, 4hbD |
| B039 pG3G | ATCC 13032 Δldh ΔNCgl0049 ANCgl0463 ΔNCgl2619 pGT1_cat1, sucD, 4hbD |

### 3.1.4. 4HB production

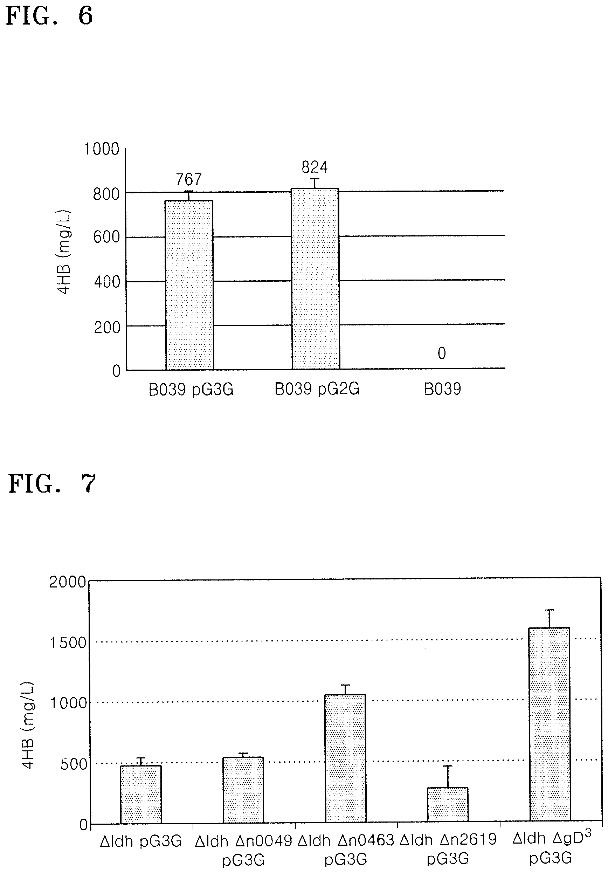

Strains disclosed in Table 2 were cultured in a flask to compare 4HB productivities thereof. The strains were cultured in a brain heart infusion (BHI) medium (Becton, Dickinson and Company) at a temperature of 30 °C and at a rotation speed of 230 rpm for 8 hours. A culture medium solution obtained therefrom was inoculated in a CgXll medium (Keilhauer et al. J. Bacteriol. 1993, 175:5595) at a concentration of 1/50, and then cultured at 30 °C and at 230 rpm for 16 hours. Cap and neck portions of the flask were sealed to create a microaerobic condition and then cultured at 30 °C and at 230 rpm for 8 hours (FIG. 6) or 24 hours (FIG. 7).

FIG. 6 shows 4HB production of B039 pG3G and B039 pG2G. FIG. 7 shows measurement results of 4HB production after introducing a pG3G or a pG2G into 4HB production of the strains described in Table 2. FIG. 6 shows measurement results of 4HB production after introducing the pG3G or the pG2G vector in which all three types of ssadh genes were partially or completely deleted. FIG. 7 shows measurement results of 4HB production after introducing the pG3G vector in which all three types of ssadh genes were partially or completely deleted. The B039 pG3G strain, in which all three types of ssadh genes present in the chromosome were deleted, showed at least a 230% increase in 4HB production, compared to the CGL (Δldh pG3G) strain in which all the three types of ssadh genes present in the chromosome were not deleted.

### 3.2. Preparation of a D003 strain and evaluation of 4HB productivity

### 3.2.1. Preparation of a pK19 gapA::3G vector

Based on the pK19 mobsacB, a pK19 gapA::3G vector for inserting cat1, sucD, and 4hbD genes of a Corynebacterium chromosome was prepared. Two homologous regions for homologous recombination were obtained by PCR amplification using a genomic DNA of *Corynebacterium glutamicum* ATCC 13032 and a primer set of ncgl0049_up F(SEQ ID NO: 50) and ncgl0049_up R(SEQ ID NO: 51) and a primer set of ncgl0049_down F(SEQ ID NO: 52) and ncgl0049_down R(SEQ ID NO: 53), respectively. A DNA fragment, including a gapA promoter and cat1, sucD and 4hbD genes, was obtained by PCR amplification using a pG3G vector as a template and a primer set of pG3G F(SEQ ID NO: 54) and pG3G R(SEQ ID NO: 55). In HindIII and EcoRI restriction sites of the pK19 mobsacB vector, the amplification product was cloned by using the In-fusion HD cloning kit, such that the DNA fragment including the gapA promoter and cat1, sucD and 4hbD genes was disposed between the two homologous regions, to prepare a pK19 gapA::3G vector.

### 3.2.2. Preparation of a strain

The pK19 gapA::3G vector was introduced to a CGL (Δldh) strain or a CGL (Aldh ΔgD3) strain through electroporation. The introduced strain was streaked in an LBHIS medium containing 25 *µg*/ml of kanamycin to culture the same at a temperature of 30 °C. Then, the colonies formed were streaked in an LB-sucrose medium to culture the same at 30°C and then only the colonies, in which a double crossing-over occurred, were selected. Genomic DNA was separated from the selected colonies and introduction of cat1, sucD, and 4hbD genes were confirmed by using a primer set of cat1-seq1F(SEQ ID NO: 56) and cat1-seq2R(SEQ ID NO: 57), a primer set of sucD-seq1F(SEQ ID NO: 58) and sucD-seq2R(SEQ ID NO: 59), and a primer set of 4hbD-seq1F(SEQ ID NO: 60) and 4hbD-seq2R(SEQ ID NO: 61), respectively. Table 3 below shows strain names and genotypes of the prepared strains.

**[Table 3]**

| Strain name | Genotype |
|---|---|
| D001 | ATCC 13032 Δldh, gapA::cat1, sucD, 4hbD |
| D003 | ATCC 13032 Δldh ΔNCgl0049 ΔNCgl0463 ΔNCgl2619, gapA::cat1, sucD, 4hbD |

### 3.2.3. 4HB production

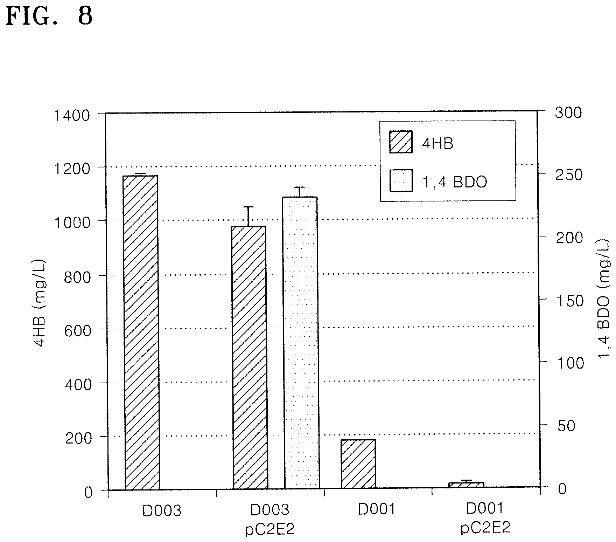

The strains disclosed in Table 3 were cultured in the same manner as in Example 3.1. The final culture medium solution was centrifuged, filtered, and then analyzed for 4HB by using ultra performance liquid chromatography (UPLC). The UPLC analysis was performed with reference to a conventional method (J Chromatogra.B 885-886, 2012, 37-42). An apparatus used for the UPLC analysis was an Acquity UPLC (Waters) loaded with an HSS T3 column (2.1 mm id x 100 mm, dp = 1.7 *µ*m). As a mobile phase, 0.2% formic acid (A) and methanol (B) were used, the HSS T3 column was maintained at a temperature of 65 °C, and chromatography was performed at a flow rate of 0.5 ml/min. As shown in FIG. 8, D003 strain, in which all three types of ssadh genes present in the chromosome were deleted, produced about 1160 mg/L and thus, showed 4HB production that is about 6.4 times as great as D001 strain in which the genes were not deleted.

### 3.3. Preparation of a D003 pG3G strain and evaluation of 4HB productivity

### 3.3.1. Preparation of a vector

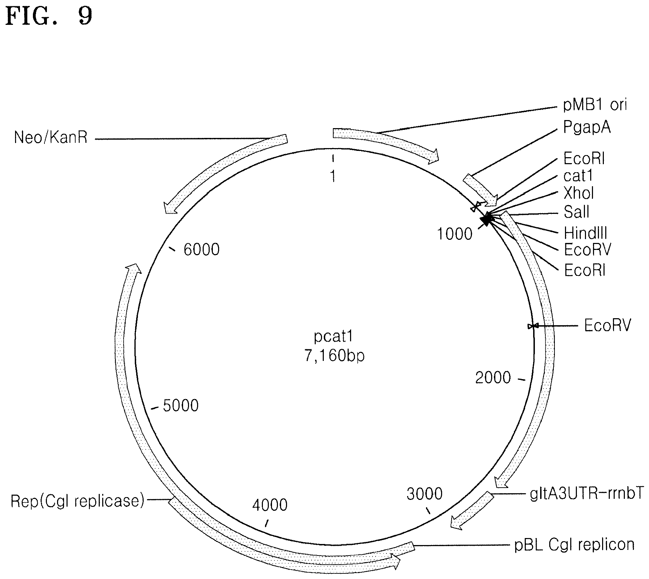

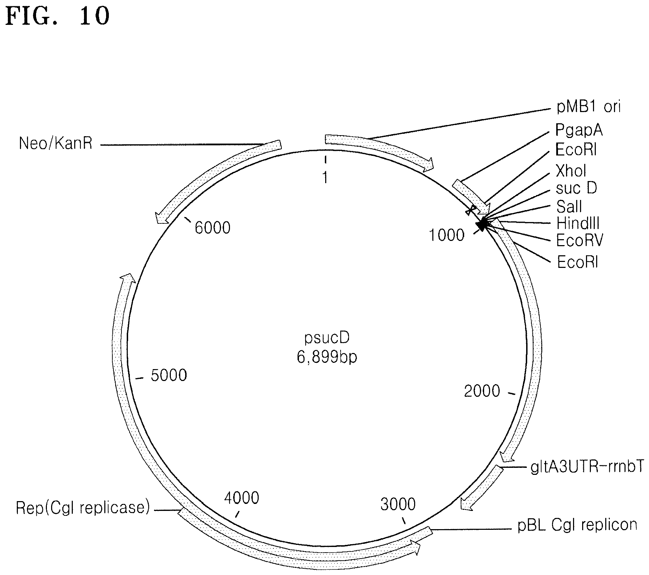

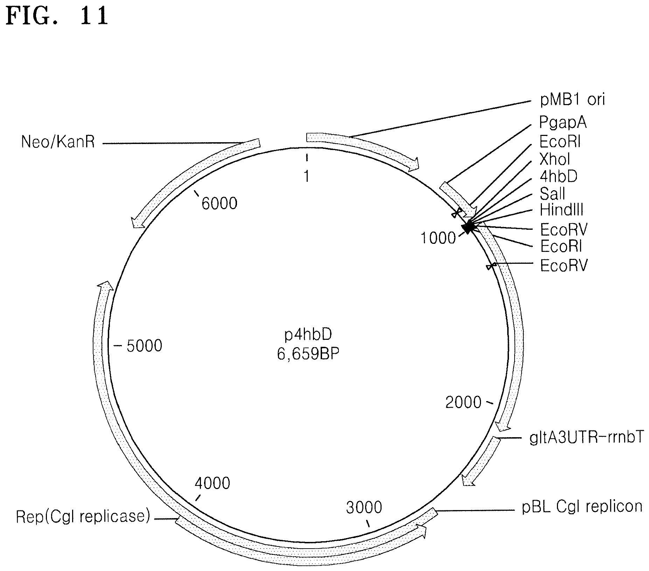

Based on the pGSX1 vector, expression vectors, pcat1, psucD, and p4hbD were prepared for cat1, sucD, and 4hbD genes, respectively. FIGS. 9, 10, and 11 show cleavage maps of pcat1, psucD, and p4hbD vectors. In the gene cassette, the cat1 sequence was obtained by PCR amplification using a primer set of cat1-up (SEQ ID NO: 62) and cat1-down (SEQ ID NO: 63). The sucD sequence was obtained by PCR amplification using a primer set of sucD-up(SEQ ID NO: 64) and sucD-down(SEQ ID NO: 65). The 4hbD sequence was obtained by PCR amplification using a primer set of 4hbD-up (SEQ ID NO: 66) and 4hbD-down (SEQ ID NO: 67). The amplification products obtained therefrom were cloned in Pstl and Xbal restriction sites of the pGSX1 vector by using the In-fusion HD cloning kit.

### 3.3.2. Preparation of a strain

The pcat1, psucD, p4hbD, pG2G, and pG3G vectors were introduced to the D003 strain through electroporation. Table 4 shows the genotypes of the prepared strains. The introduced strains were cultured in an LB medium and then freeze-stored.

**[Table 4]**

| Strain names | Genotypes |
|---|---|
| D003 | ATCC13032 Δldh ΔNCgl0049 ΔNCgl0463 ΔNCgl2619, gapA::cat1:sucD:4hbD |
| D003 pcat1 | ATCC13032 Δldh ΔNCgl0049 ΔNCgl0463 ΔNCgl2619, gapA::cat1:sucD:4hbD, pcat1 |
| D003 psucD | ATCC13032 Δldh ΔNCgl0049 ΔNCgl0463 ΔNCgl2619, gapA::cat1:sucD:4hbD, psucD |
| D003 p4hbD | ATCC13032 Δldh ΔNCgl0049 ΔNCgl0463 ΔNCgl2619, gapA::cat1:sucD:4hbD, p4hbD |
| D003 pG2G | ATCC13032 Δldh ΔNCgl0049 ΔNCgl0463 ΔNCgl2619, gapA::cat1:aucD:4hbD, pG2G |
| D003 pG3G | ATCC13032 Δldh ΔNCgl0049 ΔNCgl0463 ΔNCgl2619, gapA::cat1:sucD:4hbD, pG3G |

### 3.3.3. Production of 4HB

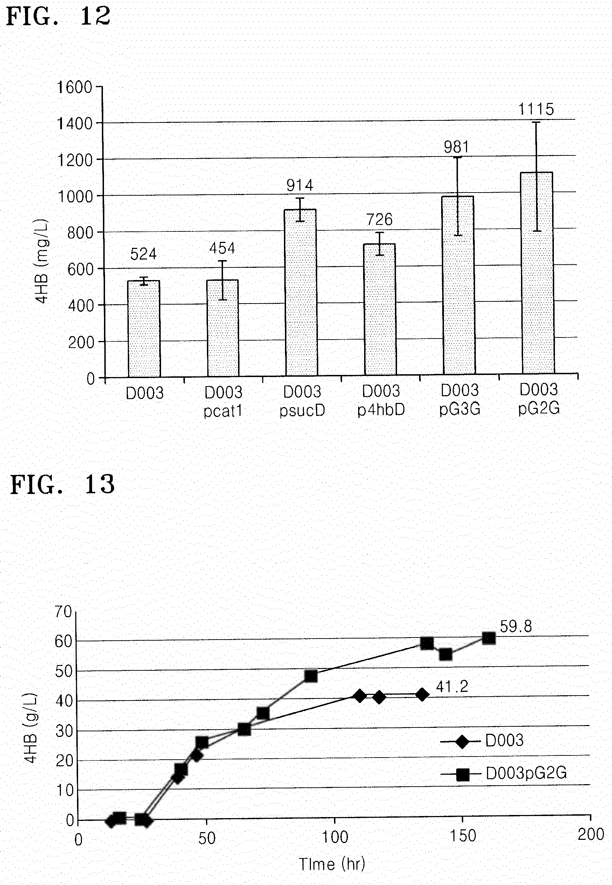

The strains disclosed in Table 4 were cultured for 24 hours in a flask in the same method as in Example 3.1. to compare 4HB productivity. FIG. 12 shows an amount of 4HB produced in a culture using the strains disclosed in Table 4. As shown in FIG. 12, 4HB productivity of the D003 pG2G was the greatest.

With respect to the strains, 4HB productivities thereof were compared through a fermentation experiment. The fermentation strains were cultured in a medium including 4% of glucose, 1% of polypeptone, 5 g/L of yeast extract, 2 g/L of (NH₄)₂SO4, 4 g/L of potassium phosphate monobasic, 8 g/L of potassium phosphate dibasic, 0.5 g/L of MgSO4·7H₂O, 1 mg/L of thiamine-HCl, 0.1 mg/L of D-biotin, and trace elements (0.1-10 ppm of iron, manganese, zinc, copper) at a temperature of 30°C and at a speed of 230 rpm for about 8 hours to about 12 hours. The fermentation medium included 1% of CSL, 2 g/L of (NH₄)₂SO₄, 1g/L of potassium phosphate monobasic, 0.5 g/L of MgSO4·7H2O, 3 mg/L of thiamine-HCl, 0.3 mg/L of D-biotin, and trace elements (0.1∼10 ppm of iron, manganese, zinc, and copper), and glucose was added according to an amount of glucose consumption during the fermentation. The strains were cultured under conditions of culturing temperature at about 30 °C to about 35 °C, stirring speed of about 500 rpm to about 800 rpm, amount of air current of about 0.5 vvm to about 2 vvm, pH during culturing of about 6.5 to about 8.0 in an aerobic culture for about 17 hours to about 35 hours, and then switched to anaerobic or microaerobicconditions under the same culturing temperature and pH during culturing, to culture the strains at a stirring speed of about 100 rpm to about 500 rpm and amount of air current of about 0 vvm to about 0.4 vvm for about 24 hours to about 150 hours. FIG. 13 shows a production amount of 4HB in D003 and D003 pG2G. As shown in FIG. 13, it was confirmed that D003 pG2G, which increased the expression of sucD and 4hbD, increased 4HB productivity to a level that is about 1.5 times as great as that of D003. For FIG. 13, D003 and D003 pG2G were cultured at 30 °C for 24 hours in aerobic conditions and then cultured in a microaerobic conditions, i.e., with an aeration rate of 0.1 vvm.

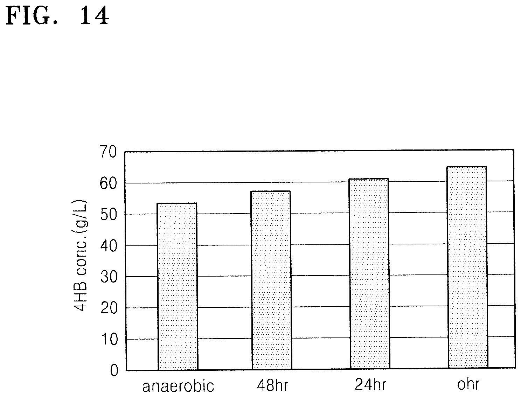

Also, during the fermentation process of D003 pG2G, 4HB productivities according to changes in air flow conditions were compared after performing the aerobic culturing as described above. After the completion of aerobic growth, 4HB productivities were measured for a case in which the strains were maintained in a microaerobic state immediately thereafter at 0.1 vvm and a case in which the strains were maintained in an anaerobic condition without aeration, and a case in which the strains were maintained in anaerobic conditions for 24 hours to 48 hours and then maintained in an microaerobic state at 0.1 wm. FIG. 14 shows 4HB productivities according to aeration conditions after growth by aerobic culturing. In FIG. 14, 'Ohr' represents the case in which the microaerobic state was maintained at 0.1 vvm immediately after the completion of the aerobic growth for 114 hours. The expression, 'anaerobic' represents the case in which the strains were maintained without aeration, i.e., the strains were maintained in a closed vessel without aeration, thus the oxygen is only provided from the head space of the vessel and the expressions '48 hr' and '24 hr' represent the case in which the strains were maintained in anaerobic conditions for 24 hours to 48 hours and then maintained in an microaerobic state at 0.1 vvm to the end. As a result, as shown in FIG. 14, '0hr' showed 4HB productivity that is 1.2 times as great as that of the case in which the strains were maintained in an anaerobic condition.

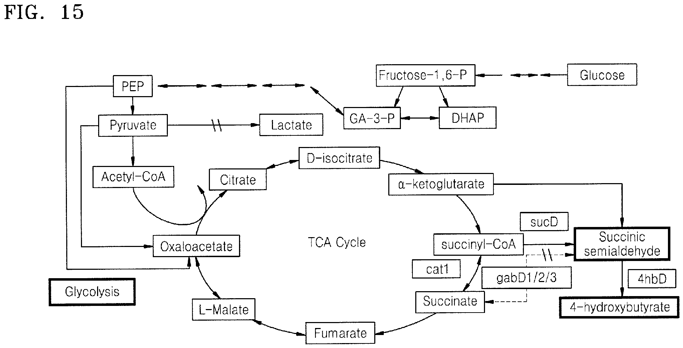

FIG. 15 shows an example of a biosynthetic pathway for producing 4-hydroxybutyrate from glucose

### Example 4. Changes in 1,4-BDO production due to deletion of a succinic semialdehyde dehydrogenase gene

### 4.1. Preparation of a D003 pC2E2 strain

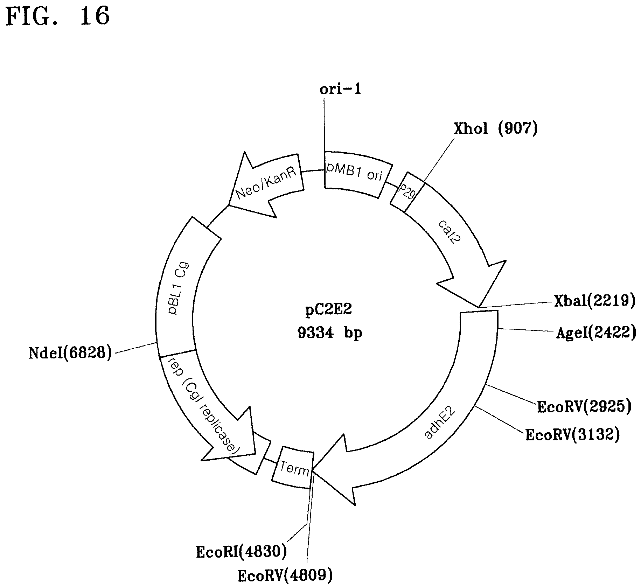

Based on the pGSK+ vector, cat2 and adhE2 gene expression vector pC2E2 (SEQ ID NO: 68) was prepared. FIG. 16 is a cleavage map of a pC2E2 vector. A P29 promoter (P29) was obtained by PCR amplification using a genomic DNA of *Corynebacterium glutamicum* ATCC 13032 as a template and a primer set of P1 (SEQ ID NO: 69) and P2(SEQ ID NO: 70). The cat2 gene was obtained by PCR amplification using a vector MD0294(SEQ ID NO: 71) as a template and a primer set of P3(SEQ ID NO: 72) and P4(SEQ ID NO: 73). The adhE2 gene was obtained by PCR amplification using a vector MD0295(SEQ ID NO: 74) as a template and a primer set of P5(SEQ ID NO: 75) and P6(SEQ ID NO: 76). A transcription terminator region (Term) was obtained by using a vector pGSX1 as a template and a primer set of P7(SEQ ID NO: 77) and P8(SEQ ID NO: 78). The amplification products obtained therefrom were cloned in Kpnl and Sacl restriction sites of the pGSK+ vector by using the In-fusion PCR cloning kit.

The pC2E2 vector was introduced into the strains D001 and D003 prepared in Example 3.1. by using electroporation. The introduced strains were streaked in an LBHIS medium containing 25 *µ*g/ml of kanamycin to culture the same at a temperature of 30 °C, and strains having kanamycin resistance were selected. Table 5 below shows strain names and genotypes of the prepared strains.

**[Table 5]**

| Strain name | Genotype |
|---|---|
| D001 pC2E2 | ATCC 13032 Δldh, gapA::cat1, sucD, 4hbD, pGSK+_cat2, adhE2 |
| D003 pC2E2 | ATCC 13032 Δldh ΔNCgl0049 ΔNCgl0463 ΔNCgl2619, gapA::cat1, sucD, 4hbD, pGSK+_cat2, adhE2 |

### 4.3. Production of 1,4-BDO

The D001 pC2E2 and D003 pC2E2 strains disclosed in Table 5 were cultured under the same conditions as described in Example 3.1, and then 1,4-BDO was analyzed by using UPLC. As shown in FIG. 8, three types of ssadh genes were deleted and the D003 pC2E2 strain in which cat2 and adhE2 genes were additionally introduced produced about 232.5 mg/L of 1,4-BDO. On the contrary, the D001 pC2E2 strain in which the ssadh gene was not deleted did not show 1,4-BDO production. The D003 pC2E2 strain in which three types of ssadh genes were deleted may be effectively used for 1,4-BDO production.

As described above, according to the one or more of the above embodiments of the present invention, a microorganism producing a C4 compound in which a CoA-dependent succinate semialdehyde dehydrogenase is expressed in a vector may produce the C4 compound more effectively. By using the microorganism, the efficiency of the C4 compound production may increase to produce the C4 compound that may be used for various industrial purposes.

The microorganism in which SSADH gene present in the chromosome is inactivated or attenuated may be used for the production of useful metabolites.

According to a method of producing 4-hydroxybutyrate according to another embodiment may effectively produce 4-hydroxybutyrate.

According to a method of producing 1,4-butanediol according to another embodiment may effectively produce 1,4-butanediol.

## Claims

1. A microorganism comprising a vector encoding a CoA-dependent succinate semialdehyde dehydrogenase, wherein the microorganism expresses the CoA-dependent succinate semialdehyde dehydrogenase from the vector and produces a C4 compound; wherein the microorganism belongs to a *Corynebacterium* genus and comprises a gene coding for an L-lactate dehydrogenase that is inactivated or attenuated, and a gene coding for a genomic CoA-independent succinic semialdehyde dehydrogenase that is inactivated or attenuated; and wherein the microorganism further comprises a vector encoding a 4-hydroxybutyrate dehydrogenase, and the microorganism expresses 4-hydroxybutyrate dehydrogenase from the vector.

2. The microorganism of claim 1, wherein the gene coding for the inactivated or attenuated genomic CoA-independent succinic semialdehyde dehydrogenase has an amino acid sequence of SEQ ID NO: 4, SEQ ID NO: 5, or SEQ ID NO: 6.

3. The microorganism of claim 1, wherein the microorganism further comprises a polynucleotide coding for a CoA-dependent succinate semialdehyde dehydrogenase and a polynucleotide coding for a 4-hydroxybutyrate dehydrogenase in a chromosome of the microorganism.

4. The microorganism of any one of claims 1 to 3, wherein the microorganism further comprises a polynucleotide coding for a succinyl-CoA:CoA transferase, preferably in a chromosome of the microorganism.

5. The microorganism of any one of claims 1 to 4, wherein the C4 compound is 4-hydroxybutyrate (4-HB).

6. The microorganism of any one of claims 1 to 5, wherein the vector is a plasmid.

7. The microorganism of any one of claims 1 to 6, wherein the microorganism further comprises a polynucleotide coding for a 4-hydroxybutyrate dehydrogenase, a polynucleotide coding for a 4-hydroxybutyryl CoA:acetyl-CoA transferase, and a polynucleotide coding for an alcohol dehydrogenase.

8. A method of producing a C4 compound, the method comprising: culturing the microorganism of any one of claims 1 to 7 with a carbon source; and recovering the C4 compound from the culture.

9. The method of claim 8, wherein the C4 compound is succinate, fumaric acid, malic acid, 4-hydroxybutyrate, 1,4-butanediol, γ-butyrolactone, or C4 compounds derived therefrom.

10. The method of claim 8 or 9, wherein the microorganism is cultured under aerobic conditions and subsequently cultured under anaerobic conditions, or the microorganism is cultured under aerobic conditions and subsequently cultured under microaerobic conditions.

11. The method of claim 10, wherein culturing under microaerobic conditions comprises providing carbon dioxide or nitrogen to the culture at a rate of about 0.1 vvm to about 0.4 vvm.

12. The method of claim 10, further comprising culturing the microorganism under microaerobic conditions 0 to 24 hours after culturing the microorganism under anaerobic conditions.

13. A Corynebacterium genus microorganism in which a gene coding for a CoA-independent succinic semialdehyde dehydrogenase is inactivated or attenuated, and L-lactate dehydrogenase activity is eliminated or attenuated; wherein the microorganism comprises at least one polynucleotide selected from a polynucleotide coding for a succinyl CoA transferase, a polynucleotide coding for acetyl-CoA hydrolase, and a polynucleotide coding for a CoA-dependent succinate semialdehyde dehydrogenase; and wherein the microorganism further comprises a polynucleotide coding for a 4-hydroxybutyrate dehydrogenase (4hbD).

14. A method of producing 4-hydroxybutyrate, the method comprising: culturing the microorganism of claim 13 with a carbon source; and recovering 4-hydroxybutyrate from the culture.

## Patentansprüche

1. Mikroorganismus umfassend einen Vektor, der eine CoA-abhängige Succinat-Semialdehyd-Dehydrogenase kodiert, wobei der Mikroorganismus die CoA-abhängige Succinat-Semialdehyd-Dehydrogenase von dem Vektor exprimiert und eine C4-Verbindung produziert; wobei der Mikroorganismus zu der *Corynebacterium-Gattung* gehört und ein Gen, das für eine L-Lactat-Dehydrogenase kodiert, welche inaktiviert oder abgeschwächt ist, und ein Gen, das für eine genomische CoA-unabhängige Succinat-Semialdehyd-Dehydrogenase kodiert, welche inaktiviert oder abgeschwächt ist, umfasst; und wobei der Mikroorganismus außerdem einen Vektor umfasst, der eine 4-Hydroxybutyrat-Dehydrogenase kodiert, und der Mikroorganismus 4-Hydroxybutyrat-Dehydrogenase von dem Vektor exprimiert.

2. Mikroorganismus nach Anspruch 1, wobei das Gen, das für die inaktivierte oder abgeschwächte genomische CoA-unabhängige Succinat-Semialdehyd-Dehydrogenase kodiert, eine Aminosäuresequenz von SEQ ID NO: 4, SEQ ID NO: 5 oder SEQ ID NO: 6 aufweist.

3. Mikroorganismus nach Anspruch 1, wobei der Mikroorganismus außerdem ein Polynukleotid, das für eine CoA-abhängige Succinat-Semialdehyd-Dehydrogenase kodiert, und ein Polynukleotid, das für eine 4-Hydroxybutyrat-Dehydrogenase kodiert, in einem Chromosom des Mikroorganismus umfasst.

4. Mikroorganismus nach einem der Ansprüche 1 bis 3, wobei der Mikroorganismus außerdem ein Polynukleotid, das für eine Succinyl-CoA:CoA-Transferase kodiert, vorzugsweise in einem Chromosom des Mikroorganismus umfasst.

5. Mikroorganismus nach einem der Ansprüche 1 bis 4, wobei die C4-Verbindung 4-Hydroxybutyrat (4-HB) ist.

6. Mikroorganismus nach einem der Ansprüche 1 bis 5, wobei der Vektor ein Plasmid ist.

7. Mikroorganismus nach einem der Ansprüche 1 bis 6, wobei der Mikroorganismus außerdem ein Polynukleotid, das für eine 4-Hydroxybutyrat-Dehydrogenase kodiert, ein Polynukleotid, das für eine 4-Hydroxybutyryl-CoA:Acetyl-CoA-Transferase kodiert, und ein Polynukleotid, das für eine Alkohol-Dehydrogenase kodiert, umfasst.

8. Verfahren zum Herstellen einer C4-Verbindung, wobei das Verfahren umfasst: die Kultivierung des Mikroorganismus nach einem der Ansprüche 1 bis 7 mit einer Kohlenstoffquelle; und die Gewinnung der C4-Verbindung aus der Kultur.

9. Verfahren nach Anspruch 8, wobei es sich bei der C4-Verbindung um Succinat, Fumarsäure, Äpfelsäure, 4-Hydroxybutyrat, 1,4-Butandiol, γ-Butyrolacton oder davon abgeleitete C4-Verbindungen handelt.

10. Verfahren nach Anspruch 8 oder 9, wobei der Mikroorganismus unter aeroben Bedingungen kultiviert wird und anschließend unter anaeroben Bedingungen kultiviert wird, oder der Mikroorganismus unter aeroben Bedingungen kultiviert wird und anschließend unter mikroaeroben Bedingungen kultiviert wird.

11. Verfahren nach Anspruch 10, wobei die Kultivierung unter mikroaeroben Bedingungen das Bereitstellen von Kohlendioxid oder Stickstoff für die Kultur mit einer Rate von ungefähr 0,1 vvm bis ungefähr 0,4 vvm umfasst.

12. Verfahren nach Anspruch 10, außerdem umfassend die Kultivierung des Mikroorganismus unter mikroaeroben Bedingungen 0 bis 24 Stunden nach der Kultivierung des Mikroorganismus unter anaeroben Bedingungen.

13. Mikroorganismus der Gattung *Corynebacterium,* in welchem ein Gen, das für eine CoA-unabhängige Succinat-Semialdehyd-Dehydrogenase kodiert, inaktiviert oder abgeschwächt ist, und die Aktivität der L-Lactat-Dehydrogenase eliminiert oder abgeschwächt ist; wobei der Mikroorganismus wenigstens ein Polynukleotid, ausgewählt aus einem Polynukleotid, das für eine Succinyl-CoA-Transferase kodiert, einem Polynukleotid, das für Acetyl-CoA-Hydrolase kodiert, und einem Polynukleotid, das für eine CoA-abhängige Succinat-Semialdehyd-Dehydrogenase kodiert, umfasst; und wobei der Mikroorganismus außerdem ein Polynukleotid, das für eine 4-Hydroxybutyrat-Dehydrogenase (4hbD) kodiert, umfasst.

14. Verfahren zum Herstellen von 4-Hydroxybutyrat, wobei das Verfahren umfasst: die Kultivierung des Mikroorganismus nach Anspruch 13 mit einer Kohlenstoffquelle; und die Gewinnung von 4-Hydroxybutyrat aus der Kultur.

## Revendications

1. Microorganisme comprenant un vecteur codant pour une succinate-semialdéhyde déshydrogénase CoA-dépendante, le microorganisme exprimant la succinate semi-aldéhyde déshydrogénase CoA-dépendante à partir du vecteur et produisant un composé en C4 ; le microorganisme appartenant à un genre *Corynebacterium* et comprenant un gène codant pour une L-lactate déshydrogénase qui est inactivée ou atténuée, et un gène codant pour une succinate-semialdéhyde déshydrogénase CoA-indépendante génomique qui est inactivée ou atténuée ; et le microorganisme comprenant, en outre, un vecteur codant pour une 4-hydroxybutyrate déshydrogénase, et le microorganisme exprimant la 4-hydroxybutyrate à partir du vecteur.

2. Microorganisme selon la revendication 1, dans lequel le gène codant pour la succinate semi-aldéhyde déshydrogénase CoA-indépendante génomique inactivée ou atténuée posède une séquence d'acides aminés parmi SEQ ID N° : 4, SEQ ID N° 5 ou SEQ ID N° : 6.

3. Microorganisme selon la revendication 1, le microorganisme comprenant, en outre, un polynucléotide codant pour une succinate-semialdéhyde déshydrogénase CoA-dépendante et un polynucléotide codant pour une 4-hydroxybutyrate déshydrogénase dans un chromosome du microorganisme.

4. Microorganisme selon l'une quelconque des revendications 1 à 3, le microorganisme comprenant, en outre, un polynucléotide codant pour une succinyl-CoA:CoA transférase, de préférence dans un chromosome du microorganisme.

5. Microorganisme selon l'une quelconque des revendications 1 à 4, le composé en C4 étant le 4-hydroxybutyrate (4-HB).

6. Microorganisme selon l'une quelconque des revendications 1 à 5, le vecteur étant un plasmide.

7. Microorganisme selon l'une quelconque des revendications 1 à 6, le microorganisme comprenant, en outre, un polynucléotide codant pour une 4-hydroxybutyrate déshydrogénase, un polynucléotide codant pour une 4-hydroxybutyryl-CoA:acétyl-CoA transférase et un polynucléotide codant pour une alcool déshydrogénase.

8. Procédé de production d'un composé en C4, le procédé comprenant : la culture d'un microorganisme selon l'une quelconque des revendications 1 à 7 avec une source de carbone ; et la récupération du composé en C4 de la culture.

9. Procédé selon la revendication 8, le composé en C4 étant du succinate, acide fumarique, acide malique, 4-hydroxybutyrate, 1,4-butanediol, γ-butyrolactone ou des composés en C4 dérivés de ceux-ci.

10. Procédé selon la revendication 8 ou 9, dans lequel le microorganisme est cultivé dans des conditions aérobies puis ensuite cultivé dans des conditions anaérobies, ou le microorganisme est cultivé dans des conditions aérobies puis ensuite cultivé dans des conditions microaérobies.

11. Procédé selon la revendication 10, dans lequel la culture dans des conditions microaérobies comprend un apport de dioxyde de carbone ou d'azote à la culture à un débit d'environ 0,1 vvm à environ 0,4 vvm.

12. Procédé selon la revendication 10, comprenant en outre la culture du microorganisme dans des conditions microaérobies 0 à 24 heures après la culture du microorganisme dans des conditions anaérobies.

13. Microorganisme du genre *Corynebacterium* dans lequel un gène codant pour une semialdéhyde succinique déshydrogénase CoA-indépendante est inactivé ou atténué, et l'activité d'une L-lactate déshydrogénase est supprimée ou atténuée ; le microorganisme comprenant au moins un polynucléotide choisi parmi un polynucléotide codant pour une succinyl-CoA transférase, un polynucléotide codant pour une acétyl-CoA hydrolase et un polynucléotide codant pour une succinate-semialdéhyde déshydrogénase CoA-dépendante ; et le microorganisme comprenant, en outre, un polynucléotide codant pour une 4-hydroxybutyrate déshydrogénase (4hbD).

14. Procédé de production de 4-hydroxybutyrate, le procédé comprenant : la culture du microorganisme de la revendication 13 avec une source de carbone ; et la récupération du 4-hydroxybutyrate de la culture.


### SEQUENCE LISTING

<110> SAMSUNG ELECTORNICS CO., LTD.
<120> Corynebacterium for producing C4-chemicals and method for producing C4
   chemicals using them
<130> EP94733FZ
<160> 85
<170> BiSSAP 1.3
<210> 1
   <211> 451
   <212> PRT
   <213> Porphyromonas gingivalis
<400> 1
   Met Glu Ile Lys Glu Met Val Ser Leu Ala Arg Lys Ala Gln Lys Glu
   1               5                   10                  15
   Tyr Gln Ala Thr His Asn Gln Glu Ala Val Asp Asn Ile Cys Arg Ala
               20                  25                  30
   Ala Ala Lys Val Ile Tyr Glu Asn Ala Ala Ile Leu Ala Arg Glu Ala
           35                  40                  45
   Val Asp Glu Thr Gly Met Gly Val Tyr Glu His Lys Val Ala Lys Asn
       50                  55                  60
   Gln Gly Lys Ser Lys Gly Val Trp Tyr Asn Leu His Asn Lys Lys Ser
   65                  70                  75                  80
   Ile Gly Ile Leu Asn Ile Asp Glu Arg Thr Gly Met Ile Glu Ile Ala
                   85                  90                  95
   Lys Pro Ile Gly Val Val Gly Ala Val Thr Pro Thr Thr Asn Pro Ile
               100                 105                 110
   Val Thr Pro Met Ser Asn Ile Ile Phe Ala Leu Lys Thr Cys Asn Ala
           115                 120                 125
   Ile Ile Ile Ala Pro His Pro Arg Ser Lys Lys Cys Ser Ala His Ala
       130                 135                 140
   Val Arg Leu Ile Lys Glu Ala Ile Ala Pro Phe Asn Val Pro Glu Gly
   145                 150                 155                 160
   Met Val Gln Ile Ile Glu Glu Pro Ser Ile Glu Lys Thr Gln Glu Leu
                   165                 170                 175
   Met Gly Ala Val Asp Val Val Val Ala Thr Gly Gly Met Gly Met Val
               180                 185                 190
   Lys Ser Ala Tyr Ser Ser Gly Lys Pro Ser Phe Gly Val Gly Ala Gly
           195                 200                 205
   Asn Val Gln Val Ile Val Asp Ser Asn Ile Asp Phe Glu Ala Ala Ala
       210                 215                 220
   Glu Lys Ile Ile Thr Gly Arg Ala Phe Asp Asn Gly Ile Ile Cys Ser
   225                 230                 235                 240
   Gly Glu Gln Ser Ile Ile Tyr Asn Glu Ala Asp Lys Glu Ala Val Phe
                   245                 250                 255
   Thr Ala Phe Arg Asn His Gly Ala Tyr Phe Cys Asp Glu Ala Glu Gly
               260                 265                 270
   Asp Arg Ala Arg Ala Ala Ile Phe Glu Asn Gly Ala Ile Ala Lys Asp
           275                 280                 285
   Val Val Gly Gln Ser Val Ala Phe Ile Ala Lys Lys Ala Asn Ile Asn
       290                 295                 300
   Ile Pro Glu Gly Thr Arg Ile Leu Val Val Glu Ala Arg Gly Val Gly
   305                 310                 315                 320
   Ala Glu Asp Val Ile Cys Lys Glu Lys Met Cys Pro Val Met Cys Ala
                   325                 330                 335
   Leu Ser Tyr Lys His Phe Glu Glu Gly Val Glu Ile Ala Arg Thr Asn
               340                 345                 350
   Leu Ala Asn Glu Gly Asn Gly His Thr Cys Ala Ile His Ser Asn Asn
           355                 360                 365
   Gln Ala His Ile Ile Leu Ala Gly Ser Glu Leu Thr Val Ser Arg Ile
       370                 375                 380
   Val Val Asn Ala Pro Ser Ala Thr Thr Ala Gly Gly His Ile Gln Asn
   385                 390                 395                 400
   Gly Leu Ala Val Thr Asn Thr Leu Gly Cys Gly Ser Trp Gly Asn Asn
                   405                 410                 415
   Ser Ile Ser Glu Asn Phe Thr Tyr Lys His Leu Leu Asn Ile Ser Arg
               420                 425                 430
   Ile Ala Pro Leu Asn Ser Ser Ile His Ile Pro Asp Asp Lys Glu Ile
           435                 440                 445
   Trp Glu Leu
       450
<210> 2
   <211> 1356
   <212> DNA
   <213> Porphyromonas gingivalis
<400> 2
   atggagatta aagagatggt cagtcttgcg cgcaaagctc agaaggagta tcaggccacc      60
   cataaccaag aagctgtgga caacatctgc cgagcagcag cgaaggttat ttacgaaaat     120
   gcagcaattc tggcacgcga ggcagtggac gaaaccggca tgggtgttta cgagcacaag     180
   gtggccaaga atcaaggcaa gtccaaaggt gtttggtaca acctgcataa caagaagtcg     240
   attggcatcc tcaatatcga cgagcgtacc ggcatgatcg agatcgcaaa acctatcggg     300
   gttgtaggcg ccgttacgcc aaccaccaac cctatcgtta ctccgatgag caacatcatc     360
   tttgctctta agacctgcaa cgccatcatt atcgccccac acccgcgctc caaaaagtgc     420
   tctgcccacg cagttcggct gatcaaagag gctatcgctc cgttcaacgt gcccgaaggt     480
   atggttcaga tcatcgagga gcctagcatc gagaagacgc aggaattgat gggcgccgta     540
   gacgtggtcg ttgctaccgg gggcatgggc atggtcaagt ctgcctactc ctcagggaag     600
   ccttctttcg gtgtcggagc cggcaatgtt caggtgatag tggacagcaa catcgacttc     660
   gaagcggcag cagaaaagat catcaccgga cgtgccttcg acaacggtat catctgctca     720
   ggcgaacagt ccatcatcta caacgaggct gacaaggaag cagttttcac agcattccgc     780
   aaccacggtg cgtacttttg cgacgaggcc gagggagatc gggctcgtgc agcgatcttc     840
   gaaaatggag ccatcgcgaa agatgttgtg ggccagtccg ttgcctttat tgcaaagaag     900
   gcgaacatta atatccccga gggtactcgt attctcgtgg tcgaagctcg cggagtaggc     960
   gccgaagatg tcatctgtaa agaaaagatg tgtccagtca tgtgcgccct ctcctacaag    1020
   cacttcgaag agggggtaga gatcgcaagg acgaacctcg caaacgaagg caatggccat    1080
   acctgtgcta tccactccaa caaccaagca cacatcatct tggcaggctc ggagctgacc    1140
   gtgtctcgca tcgtggtcaa cgcgccaagt gctaccacag caggcggtca catccagaac    1200
   ggtcttgccg tcaccaatac tctaggctgc ggctcttggg gtaacaactc gatctccgaa    1260
   aacttcactt ataaacacct gctcaacatt tcacgcatcg ccccgttgaa ctccagcatt    1320
   catatcccag atgataagga aatctgggaa ctctaa                              1356
<210> 3
   <211> 314
   <212> PRT
   <213> Corynebacterium glutamicum
<400> 3
   Met Lys Glu Thr Val Gly Asn Lys Ile Val Leu Ile Gly Ala Gly Asp
   1               5                   10                  15
   Val Gly Val Ala Tyr Ala Tyr Ala Leu Ile Asn Gln Gly Met Ala Asp
               20                  25                  30
   His Leu Ala Ile Ile Asp Ile Asp Glu Lys Lys Leu Glu Gly Asn Val
           35                  40                  45
   Met Asp Leu Asn His Gly Val Val Trp Ala Asp Ser Arg Thr Arg Val
       50                  55                  60
   Thr Lys Gly Thr Tyr Ala Asp Cys Glu Asp Ala Ala Met Val Val Ile
   65                  70                  75                  80
   Cys Ala Gly Ala Ala Gln Lys Pro Gly Glu Thr Arg Leu Gln Leu Val
                   85                  90                  95
   Asp Lys Asn Val Lys Ile Met Lys Ser Ile Val Gly Asp Val Met Asp
               100                 105                 110
   Ser Gly Phe Asp Gly Ile Phe Leu Val Ala Ser Asn Pro Val Asp Ile
           115                 120                 125
   Leu Thr Tyr Ala Val Trp Lys Phe Ser Gly Leu Glu Trp Asn Arg Val
       130                 135                 140
   Ile Gly Ser Gly Thr Val Leu Asp Ser Ala Arg Phe Arg Tyr Met Leu
   145                 150                 155                 160
   Gly Glu Leu Tyr Glu Val Ala Pro Ser Ser Val His Ala Tyr Ile Ile
                   165                 170                 175
   Gly Glu His Gly Asp Thr Glu Leu Pro Val Leu Ser Ser Ala Thr Ile
               180                 185                 190
   Ala Gly Val Ser Leu Ser Arg Met Leu Asp Lys Asp Pro Glu Leu Glu
           195                 200                 205
   Gly Arg Leu Glu Lys Ile Phe Glu Asp Thr Arg Asp Ala Ala Tyr His
       210                 215                 220
   Ile Ile Asp Ala Lys Gly Ser Thr Ser Tyr Gly Ile Gly Met Gly Leu
   225                 230                 235                 240
   Ala Arg Ile Thr Arg Ala Ile Leu Gln Asn Gln Asp Val Ala Val Pro
                   245                 250                 255
   Val Ser Ala Leu Leu His Gly Glu Tyr Gly Glu Glu Asp Ile Tyr Ile
               260                 265                 270
   Gly Thr Pro Ala Val Val Asn Arg Arg Gly Ile Arg Arg Val Val Glu
           275                 280                 285
   Leu Glu Ile Thr Asp His Glu Met Glu Arg Phe Lys His Ser Ala Asn
       290                 295                 300
   Thr Leu Arg Glu Ile Gln Lys Gln Phe Phe
   305                 310
<210> 4
   <211> 490
   <212> PRT
   <213> Corynebacterium glutamicum
<400> 4
   Met Thr Ile Asn Val Ser Glu Leu Leu Ala Lys Val Pro Thr Gly Leu
   1               5                   10                  15
   Leu Ile Gly Asp Ser Trp Val Glu Ala Ser Asp Gly Gly Thr Phe Asp
               20                  25                  30
   Val Glu Asn Pro Ala Thr Gly Glu Thr Ile Ala Thr Leu Ala Ser Ala
               35                  40                  45
   Thr Ser Glu Asp Ala Leu Ala Ala Leu Asp Ala Ala Cys Ala Val Gln
           50                  55                  60
   Ala Glu Trp Ala Arg Met Pro Ala Arg Glu Arg Ser Asn Ile Leu Arg
   65                  70                  75                  80
   Arg Gly Phe Glu Leu Val Ala Glu Arg Ala Glu Glu Phe Ala Thr Leu
                   85                  90                  95
   Met Thr Leu Glu Met Gly Lys Pro Leu Ala Glu Ala Arg Gly Glu Val
               100                 105                 110
   Thr Tyr Gly Asn Glu Phe Leu Arg Trp Phe Ser Glu Glu Ala Val Arg
           115                 120                 125
   Leu Tyr Gly Arg Tyr Gly Thr Thr Pro Glu Gly Asn Leu Arg Met Leu
       130                 135                 140
   Thr Ala Leu Lys Pro Val Gly Pro Cys Leu Leu Ile Thr Pro Trp Asn
   145                 150                 155                 160
   Phe Pro Leu Ala Met Ala Thr Arg Lys Val Ala Pro Ala Ile Ala Ala
                   165                 170                 175
   Gly Cys Val Met Val Leu Lys Pro Ala Arg Leu Thr Pro Leu Thr Ser
               180                 185                 190
   Gln Tyr Phe Ala Gln Thr Met Leu Asp Ala Gly Leu Pro Ala Gly Val
           195                 200                 205
   Leu Asn Val Val Ser Gly Ala Ser Ala Ser Ala Ile Ser Asn Pro Ile
       210                 215                 220
   Met Glu Asp Asp Arg Leu Arg Lys Val Ser Phe Thr Gly Ser Thr Pro
   225                 230                 235                 240
   Val Gly Gln Gln Leu Leu Lys Lys Ala Ala Asp Lys Val Leu Arg Thr
                   245                 250                 255
   Ser Met Glu Leu Gly Gly Asn Ala Pro Phe Ile Val Phe Glu Asp Ala
               260                 265                 270
   Asp Leu Asp Leu Ala Ile Glu Gly Ala Met Gly Ala Lys Met Arg Asn
           275                 280                 285
   Ile Gly Glu Ala Cys Thr Ala Ala Asn Arg Phe Leu Val His Glu Ser
       290                 295                 300
   Val Ala Asp Glu Phe Gly Arg Arg Phe Ala Ala Arg Leu Glu Glu Gln
   305                 310                 315                 320
   Val Leu Gly Asn Gly Leu Asp Glu Gly Val Thr Val Gly Pro Leu Val
                   325                 330                 335
   Glu Glu Lys Ala Arg Asp Ser Val Ala Ser Leu Val Asp Ala Ala Val
               340                 345                 350
   Ala Glu Gly Ala Thr Val Leu Thr Gly Gly Lys Ala Gly Thr Gly Ala
           355                 360                 365
   Gly Tyr Phe Tyr Glu Pro Thr Val Leu Thr Gly Val Ser Thr Asp Ala
       370                 375                 380
   Ala Ile Leu Asn Glu Glu Ile Phe Gly Pro Val Ala Pro Ile Val Thr
   385                 390                 395                 400
   Phe Gln Thr Glu Glu Glu Ala Leu Arg Leu Ala Asn Ser Thr Glu Tyr
                   405                 410                 415
   Gly Leu Ala Ser Tyr Val Phe Thr Gln Asp Thr Ser Arg Ile Phe Arg
               420                 425                 430
   Val Ser Asp Gly Leu Glu Phe Gly Leu Val Gly Val Asn Ser Gly Val
           435                 440                 445
   Ile Ser Asn Ala Ala Ala Pro Phe Gly Gly Val Lys Gln Ser Gly Met
       450                 455                 460
   Gly Arg Glu Gly Gly Leu Glu Gly Ile Glu Glu Tyr Thr Ser Val Gln
   465                 470                 475                 480
   Tyr Ile Gly Ile Arg Asp Pro Tyr Ala Gly
                   485                 490
<210> 5
   <211> 453
   <212> PRT
   <213> Corynebacterium glutamicum
<400> 5
   Met Ser Leu Thr Phe Pro Val Ile Asn Pro Ser Asp Gly Ser Thr Ile
   1               5                   10                  15
   Thr Glu Leu Glu Asn His Asp Ser Thr Gln Trp Met Ser Ala Leu Ser
               20                  25                  30
   Asp Ala Val Ala Ala Gly Pro Ser Trp Ala Ala Lys Thr Pro Arg Glu
           35                  40                  45
   Arg Ser Val Val Leu Thr Ala Ile Phe Glu Ala Leu Thr Glu Arg Ala
       50                  55                  60
   Gln Glu Leu Ala Glu Ile Ile His Leu Glu Ala Gly Lys Ser Val Ala
   65                  70                  75                  80
   Glu Ala Leu Gly Glu Val Ala Tyr Gly Ala Glu Tyr Phe Arg Trp Phe
                   85                  90                  95
   Ala Glu Glu Ala Val Arg Leu Pro Gly Arg Tyr Gly Gln Ser Pro Ser
               100                 105                 110
   Gly Ile Gly His Ile Ala Val Thr Arg Ala Pro Val Gly Pro Val Leu
           115                 120                 125
   Ala Ile Thr Pro Trp Asn Phe Pro Ile Ala Met Ala Thr Arg Lys Ile
       130                 135                 140
   Ala Pro Ala Leu Ala Ala Gly Cys Pro Val Leu Val Lys Pro Ala Ser
   145                 150                 155                 160
   Glu Thr Pro Leu Thr Met Val Lys Val Gly Glu Ile Ile Ala Ser Val
                   165                 170                 175
   Phe Asp Thr Phe Asn Ile Pro Gln Gly Leu Val Ser Ile Ile Thr Thr
               180                 185                 190
   Thr Arg Asp Ala Glu Leu Ser Ala Glu Leu Met Ala Asp Pro Arg Leu
           195                 200                 205
   Ala Lys Val Thr Phe Thr Gly Ser Thr Asn Val Gly Arg Ile Leu Val
       210                 215                 220
   Arg Gln Ser Ala Asp Arg Leu Leu Arg Thr Ser Met Glu Leu Gly Gly
   225                 230                 235                 240
   Asn Ala Ala Phe Val Ile Asp Glu Ala Ala Asp Leu Asp Glu Ala Val
                   245                 250                 255
   Ser Gly Ala Ile Ala Ala Lys Leu Arg Asn Ala Gly Gln Val Cys Ile
               260                 265                 270
   Ala Ala Asn Arg Phe Leu Val His Glu Ser Arg Ala Ala Glu Phe Thr
           275                 280                 285
   Ser Lys Leu Ala Thr Ala Met Gln Asn Thr Pro Ile Gly Pro Val Ile
       290                 295                 300
   Ser Ala Arg Gln Arg Asp Arg Ile Ala Ala Leu Val Asp Glu Ala Ile
   305                 310                 315                 320
   Thr Asp Gly Ala Arg Leu Ile Ile Gly Gly Glu Val Pro Asp Gly Ser
                   325                 330                 335
   Gly Phe Phe Tyr Pro Ala Thr Ile Leu Ala Asp Val Pro Ala Gln Ser
               340                 345                 350
   Arg Ile Val His Glu Glu Ile Phe Gly Pro Val Ala Thr Ile Ala Thr
           355                 360                 365
   Phe Thr Asp Leu Ala Glu Gly Val Ala Gln Ala Asn Ser Thr Glu Phe
       370                 375                 380
   Gly Leu Ala Ala Tyr Gly Phe Ser Asn Asn Val Lys Ala Thr Gln Tyr
   385                 390                 395                 400
   Met Ala Glu His Leu Glu Ala Gly Met Val Gly Ile Asn Arg Gly Ala
                   405                 410                 415
   Ile Ser Asp Pro Ala Ala Pro Phe Gly Gly Ile Gly Gln Ser Gly Phe
               420                 425                 430
   Gly Arg Glu Gly Gly Thr Glu Gly Ile Glu Glu Tyr Leu Ser Val Arg
           435                 440                 445
   Tyr Leu Ala Leu Pro
       450
<210> 6
   <211> 521
   <212> PRT
   <213> Corynebacterium glutamicum
<400> 6
   Met Ile Lys Arg Leu Pro Leu Gly Pro Leu Pro Lys Glu Leu His Gln
   1               5                   10                  15
   Thr Leu Leu Asp Leu Thr Ala Asn Ala Gln Asp Ala Ala Lys Val Glu
               20                  25                  30
   Val Ile Ala Pro Phe Thr Gly Glu Thr Leu Gly Phe Val Phe Asp Gly
           35                  40                  45
   Asp Glu Gln Asp Val Glu His Ala Phe Ala Leu Ser Arg Ala Ala Gln
       50                  55                  60
   Lys Lys Trp Val His Thr Thr Ala Val Glu Arg Lys Lys Ile Phe Leu
   65                  70                  75                  80
   Lys Phe His Asp Leu Val Leu Lys Asn Arg Glu Leu Leu Met Asp Ile
                   85                  90                  95
   Val Gln Leu Glu Thr Gly Lys Asn Arg Ala Ser Ala Ala Asp Glu Val
               100                 105                 110
   Leu Asp Val Ala Ile Thr Thr Arg Phe Tyr Ala Asn Asn Ala Gly Lys
           115                 120                 125
   Phe Leu Asn Asp Lys Lys Arg Pro Gly Ala Leu Pro Ile Ile Thr Lys
       130                 135                 140
   Asn Thr Gln Gln Tyr Val Pro Lys Gly Val Val Gly Gln Ile Thr Pro
   145                 150                 155                 160
   Trp Asn Tyr Pro Leu Thr Leu Gly Val Ser Asp Ala Val Pro Ala Leu
                   165                 170                 175
   Leu Ala Gly Asn Ala Val Val Ala Lys Pro Asp Leu Ala Thr Pro Phe
               180                 185                 190
   Ser Cys Leu Ile Met Val His Leu Leu Ile Glu Ala Gly Leu Pro Arg
           195                 200                 205
   Asp Leu Met Gln Val Val Thr Gly Pro Gly Asp Ile Val Gly Gly Ala
       210                 215                 220
   Ile Ala Ala Gln Cys Asp Phe Leu Met Phe Thr Gly Ser Thr Ala Thr
   225                 230                 235                 240
   Gly Arg Ile Leu Gly Arg Thr Met Gly Glu Arg Leu Val Gly Phe Ser
                   245                 250                 255
   Ala Glu Leu Gly Gly Lys Asn Pro Leu Ile Val Ala Lys Asp Ala Asp
               260                 265                 270
   Leu Asp Lys Val Glu Ala Glu Leu Pro Gln Ala Cys Phe Ser Asn Ser
           275                 280                 285
   Gly Gln Leu Cys Val Ser Thr Glu Arg Ile Tyr Val Glu Glu Asp Val
       290                 295                 300
   Tyr Glu Glu Val Ile Ala Arg Phe Ser Lys Ala Ala Lys Ala Met Ser
   305                 310                 315                 320
   Ile Gly Ala Gly Phe Glu Trp Lys Tyr Glu Met Gly Ser Leu Ile Asn
                   325                 330                 335
   Gln Ala Gln Leu Asp Arg Val Ser Thr Phe Val Asp Gln Ala Lys Ala
               340                 345                 350
   Ala Gly Ala Thr Val Leu Cys Gly Gly Lys Ser Arg Pro Asp Ile Gly
           355                 360                 365
   Pro Phe Phe Tyr Glu Pro Thr Val Leu Ala Asp Val Pro Glu Gly Thr
       370                 375                 380
   Pro Leu Leu Thr Glu Glu Val Phe Gly Pro Val Val Phe Ile Glu Lys
   385                 390                 395                 400
   Val Ala Thr Leu Glu Glu Ala Val Asp Lys Ala Asn Gly Thr Pro Tyr
                   405                 410                 415
   Gly Leu Asn Ala Ser Val Phe Gly Ser Ser Glu Thr Gly Asn Leu Val
               420                 425                 430
   Ala Gly Gln Leu Glu Ala Gly Gly Ile Gly Ile Asn Asp Gly Tyr Ala
           435                 440                 445
   Ala Thr Trp Ala Ser Val Ser Thr Pro Leu Gly Gly Met Lys Gln Ser
       450                 455                 460
   Gly Leu Gly His Arg His Gly Ala Glu Gly Ile Thr Lys Tyr Ala Glu
   465                 470                 475                 480
   Ile Arg Asn Ile Ala Glu Gln Arg Trp Met Ser Met Arg Gly Pro Ala
                   485                     490                 495
   Lys Met Pro Arg Lys Val Tyr Ser Asp Thr Val Ala Thr Ala Leu Lys
               500                     505                 510
   Leu Gly Lys Ile Phe Lys Val Leu Pro
           515                 520
<210> 7
   <211> 1473
   <212> DNA
   <213> Corynebacterium glutamicum
<400> 7
   atgactatta atgtctccga actacttgcc aaagtcccca cgggtctact gattggtgat      60
   tcctgggtgg aagcatccga cggcggtact ttcgatgtgg aaaacccagc gacgggtgaa     120
   acaatcgcaa cgctcgcgtc tgctacttcc gaggatgcac tggctgctct tgatgctgca     180
   tgcgctgttc aggccgagtg ggctaggatg ccagcgcgcg agcgttctaa tattttacgc     240
   cgcggttttg agctcgtagc agaacgtgca gaagagttcg ccaccctcat gaccttggaa     300
   atgggcaagc ctttggctga agctcgcggc gaagtcacct acggcaacga attcctgcgc     360
   tggttctctg aggaagcagt tcgtctgtat ggccgttacg gaaccacacc agaaggcaac     420
   ttgcggatgc tgaccgccct caagccagtt ggcccgtgcc tcctgatcac cccatggaac     480
   ttcccactag caatggctac ccgcaaggtc gcacctgcga tcgctgcagg ttgtgtcatg     540
   gtgctcaagc cagctcgact taccccgctg acctcccagt attttgctca gaccatgctt     600
   gatgccggtc ttccagcagg tgtcctcaat gtggtctccg gtgcttccgc ctctgcgatt     660
   tccaacccga ttatggaaga cgatcgcctt cgtaaagtct ccttcaccgg ctccacccca     720
   gttggccagc agctgctcaa aaaggctgcc gataaagttc tgcgcacctc catggaactt     780
   ggtggcaacg cacctttcat tgtcttcgag gacgccgacc tagatctcgc gatcgaaggt     840
   gccatgggtg ccaaaatgcg caacatcggc gaagcttgca ccgcagccaa ccgtttctta     900
   gtccacgaat ccgtcgccga tgaattcggc cgtcgcttcg ctgcccgcct tgaagagcaa     960
   gtcctaggca acggcctcga cgaaggcgtc accgtgggcc ccctggttga ggaaaaagca    1020
   cgagacagcg ttgcatcgct tgtcgacgcc gccgtcgccg aaggtgccac cgtcctcacc    1080
   ggcggcaagg ccggcacagg tgcaggctac ttctacgaac caacggtgct cacgggagtt    1140
   tcaacagatg cggctatcct gaacgaagag atcttcggtc ccgtcgcacc gatcgtcacc    1200
   ttccaaaccg aggaagaagc cctgcgtcta gccaactcca ccgaatacgg actggcctcc    1260
   tatgtgttca cccaggacac ctcacgtatt ttccgcgtct ccgatggtct cgagttcggc    1320
   ctagtgggcg tcaattccgg tgtcatctct aacgctgctg caccttttgg tggcgtaaaa    1380
   caatccggaa tgggccgcga aggtggtctc gaaggaatcg aggagtacac ctccgtgcag    1440
   tacatcggta tccgggatcc ttacgccggc tag                                 1473
<210> 8
   <211> 1362
   <212> DNA
   <213> Corynebacterium glutamicum
<400> 8
   gtgtctttga ccttcccagt aatcaacccc agcgatggct ccaccatcac cgagctagaa 60
   aaccacgatt ccacccagtg gatgtccgcg ctctctgatg cagttgcagc tggtccttca 120
   tgggctgcga aaactccccg cgaaagatcc gtggtactca ccgcaatctt cgaagcactg 180
   accgaacgcg cccaagaact tgcagagatc atccacctgg aagctggaaa atccgttgca 240
   gaagctcttg gtgaagtcgc ttatggtgca gaatacttcc gttggtttgc ggaagaagca 300
   gtgcgcctgc ccggccgcta cggacagtca ccttccggaa tcggtcacat cgccgtcacc 360
   cgcgcacccg tgggaccagt gctggcgatc accccatgga atttccccat cgccatggcc 420
   acccgcaaaa tcgccccagc cctggccgct ggttgccccg tgttggtgaa acctgcttcc 480
   gaaaccccac tgaccatggt caaagtgggg gagatcatcg cctccgtctt tgataccttt 540
   aatatcccgc agggcttggt ctcaatcatc accaccactc gagatgcaga gctatcggca 600
   gaactcatgg ctgatcctcg cttggctaaa gtcaccttca ctggatcaac caacgtggga 660
   cgcatcctgg tccgccaatc cgcggaccga ctgctgcgca cctccatgga actcggcgga 720
   aatgcagctt ttgttatcga cgaagccgca gacctcgacg aagccgtatc cggtgccatc 780
   gccgcaaaac tccgcaacgc cggccaagta tgcatcgcag ctaaccgttt cttggttcat 840
   gaatcccgcg ctgccgaatt cacctcaaag ctggcgacag ccatgcagaa cactcccatt 900
   gggccggtga tttctgcccg ccaacgcgac cggatcgcag cactagtgga tgaagccatc 960
   accgacggcg cccgcctcat catcggtggg gaggtccccg acggctccgg cttcttctat 1020
   ccagccacca tcttggccga tgtccctgca cagtcacgga ttgtgcatga ggaaatcttc 1080
   ggacctgtgg ccaccattgc cactttcacc gacttggccg aaggcgttgc acaagcaaat 1140
   tccaccgaat tcggcctcgc agcctacgga ttcagcaaca atgtgaaagc aacacagtac 1200
   atggcggaac acttggaagc cggaatggtc ggaatcaaca gaggcgccat ctctgaccca 1260
   gcagcacctt ttggcggcat cggacaatcc ggcttcggca gagaaggcgg aaccgaagga 1320
   atcgaagaat atctctccgt gcgttacctc gctttgccgt ga 1362
<210> 9
   <211> 1566
   <212> DNA
   <213> Corynebacterium glutamicum
<400> 9
   atgatcaaac gtcttccttt aggtccgctg cctaaagaac ttcatcagac tctgcttgat 60
   ctgaccgcaa atgcccaaga tgcggcgaaa gtggaggtta tagcgccatt tactggcgag 120
   accctcggat ttgtttttga tggtgatgag caagacgtcg agcatgcttt tgcactttca      180
   agggcagccc agaaaaagtg ggtgcacacc acggcagtgg aacggaagaa gatcttcctg      240
   aagtttcatg atctggtatt gaaaaaccgt gagctgctca tggacatcgt gcagttggaa      300
   acaggcaaaa atcgagcatc ggctgccgat gaggtgttgg acgttgcgat caccacccgc      360
   ttctacgcaa acaatgcagg aaagttttta aatgacaaga aacgccccgg cgcgcttccg      420
   atcatcacga aaaacacaca acagtatgtg cccaagggag tggtcgggca gatcacgccg      480
   tggaattacc ctttaacttt gggagtatct gatgctgttc cggcgctgct ggcaggaaac      540
   gcagtggtgg ctaaacctga cctcgcgaca cctttctcct gcttgatcat ggtgcacctg      600
   ctcattgaag ccggtctgcc gcgtgatttg atgcaggttg tcaccggccc tggcgatatt      660
   gttggcggtg cgattgcagc tcagtgtgat ttcctcatgt tcactggatc cacggccacg      720
   ggccggatct tgggtcggac aatgggtgag cgtttggtgg gtttctctgc ggaattaggc      780
   ggaaagaacc ctcttattgt ggccaaggat gcagatctgg acaaggtgga agctgagctt      840
   ccgcaggcgt gtttttccaa ctcggggcaa ttgtgtgtct ccactgaacg tatttatgtc      900
   gaggaagacg tgtacgagga ggtgattgca cggtttagca aggcggcgaa agccatgtcc      960
   attggtgccg gatttgagtg gaaatatgag atgggttcgt tgatcaatca ggcgcagctg     1020
   gatcgggtga gcacctttgt tgatcaggct aaagctgcgg gcgccacggt gctgtgcggt     1080
   ggcaagtcac gccctgatat tggtcccttc ttctatgagc ccacggtatt ggcggatgtc     1140
   ccagagggca ccccactgct cacggaggaa gtcttcgggc cggtggtgtt catcgaaaag     1200
   gtagccacac tggaagaagc cgtcgataag gcaaatggca cgccctacgg cctgaatgcg     1260
   tccgtctttg ggtcgtcgga aaccggcaat cttgttgcag gccagctgga agctggcggt     1320
   atcggtatta atgatggcta cgccgcgacg tgggcgagcg tgtccacgcc tctgggtggc     1380
   atgaagcagt cggggctggg gcaccgccat ggtgcggagg gaattacaaa atatgcggag     1440
   atccgaaaca tcgcggagca gcgctggatg tctatgcgtg ggccggccaa aatgccgcga     1500
   aaggtgtact cagacaccgt ggccacagcg ctaaagctgg gcaaaatctt taaagttttg     1560
   ccgtag                                                                  1566
<210> 10
   <211> 371
   <212> PRT
   <213> Porphyromonas gingivalis
<400> 10
   Met Gln Leu Phe Lys Leu Lys Ser Val Thr His His Phe Asp Thr Phe
   1               5                   10                  15
   Ala Glu Phe Ala Lys Glu Phe Cys Leu Gly Glu Arg Asp Leu Val Ile
               20                  25                  30
   Thr Asn Glu Phe Ile Tyr Glu Pro Tyr Met Lys Ala Cys Gln Leu Pro
           35                  40                  45
   Cys His Phe Val Met Gln Glu Lys Tyr Gly Gln Gly Glu Pro Ser Asp
       50                  55                  60
   Glu Met Met Asn Asn Ile Leu Ala Asp Ile Arg Asn Ile Gln Phe Asp
   65                  70                  75                  80
   Arg Val Ile Gly Ile Gly Gly Gly Thr Val Ile Asp Ile Ser Lys Leu
                   85                  90                  95
   Phe Val Leu Lys Gly Leu Asn Asp Val Leu Asp Ala Phe Asp Arg Lys
               100                 105                 110
   Ile Pro Leu Ile Lys Glu Lys Glu Leu Ile Ile Val Pro Thr Thr Cys
           115                 120                 125
   Gly Thr Gly Ser Glu Val Thr Asn Ile Ser Ile Ala Glu Ile Lys Ser
       130                 135                 140
   Arg His Thr Lys Met Gly Leu Ala Asp Asp Ala Ile Val Ala Asp His
   145                 150                 155                 160
   Ala Ile Ile Ile Pro Glu Leu Leu Lys Ser Leu Pro Phe His Phe Tyr
                   165                 170                 175
   Ala Cys Ser Ala Ile Asp Ala Leu Ile His Ala Ile Glu Ser Tyr Val
               180                 185                 190
   Ser Pro Lys Ala Ser Pro Tyr Ser Arg Leu Phe Ser Glu Ala Ala Trp
           195                 200                 205
   Asp Ile Ile Leu Glu Val Phe Lys Lys Ile Ala Glu His Gly Pro Glu
       210                 215                 220
   Tyr Arg Phe Glu Lys Leu Gly Glu Met Ile Met Ala Ser Asn Tyr Ala
   225                 230                 235                 240
   Gly Ile Ala Phe Gly Asn Ala Gly Val Gly Ala Val His Ala Leu Ser
                   245                 250                 255
   Tyr Pro Leu Gly Gly Asn Tyr His Val Pro His Gly Glu Ala Asn Tyr
               260                 265                 270
   Gln Phe Phe Thr Glu Val Phe Lys Val Tyr Gln Lys Lys Asn Pro Phe
           275                 280                 285
   Gly Tyr Ile Val Glu Leu Asn Trp Lys Leu Ser Lys Ile Leu Asn Cys
       290                 295                 300
   Gln Pro Glu Tyr Val Tyr Pro Lys Leu Asp Glu Leu Leu Gly Cys Leu
   305                 310                 315                 320
   Leu Thr Lys Lys Pro Leu His Glu Tyr Gly Met Lys Asp Glu Glu Val
                   325                 330                 335
   Arg Gly Phe Ala Glu Ser Val Leu Lys Thr Gln Gln Arg Leu Leu Ala
               340                 345                 350
   Asn Asn Tyr Val Glu Leu Thr Val Asp Glu Ile Glu Gly Ile Tyr Arg
           355                 360                 365
   Arg Leu Tyr
       370
<210> 11
   <211> 1116
   <212> DNA
   <213> Porphyromonas gingivalis
<400> 11
   atgcagcttt tcaagctcaa gagcgtcaca catcactttg atacttttgc agagtttgcc      60
   aaggagttct gtctcggtga acgcgacttg gtaattacca acgagttcat ctacgaaccg     120
   tatatgaagg catgccagct gccttgtcat tttgtgatgc aggagaaata cggccaaggc     180
   gagccttctg acgagatgat gaacaacatc ctagcagata tccgtaatat ccagttcgac     240
   cgcgtgatcg ggatcggagg tggtacggtt attgacatct caaaactctt tgttctgaag     300
   ggattaaatg atgttctcga cgcgttcgat cgcaagattc cccttatcaa agagaaagaa     360
   ctgatcattg tgcccaccac ctgcggaacc ggctcggagg tgacgaacat ttccatcgcc     420
   gagatcaagt cccggcacac caagatgggt ttggctgacg atgcaattgt tgctgaccac     480
   gccataatca tccctgaact tctgaagagc ttgcccttcc acttctatgc atgctccgca     540
   atcgacgctc ttattcatgc catcgagtca tacgtttctc caaaagcgtc tccatactcc     600
   cgtctgttca gtgaggcggc gtgggacatt atcctggaag ttttcaagaa aatcgccgaa     660
   cacggcccag agtaccgctt cgagaagctg ggggaaatga tcatggccag caactatgcc     720
   ggtatcgctt tcggcaacgc aggcgttggc gccgtccacg ctctatccta cccgttgggc     780
   ggcaactatc acgtgccgca tggagaagca aactatcagt tcttcaccga ggtctttaaa     840
   gtataccaaa agaagaatcc gttcggctat attgtcgaac tcaactggaa gctctccaag     900
   attctgaact gccagccaga gtacgtgtac ccgaagctgg atgaactgct cggttgcctt     960
   cttaccaaga aacctttgca cgaatacggc atgaaggacg aagaggttcg tggcttcgcg    1020
   gaatcggtcc tgaagaccca gcaacgcttg ctcgccaaca actacgtcga acttactgtc    1080
   gatgagatcg aaggtatcta ccgacgtctc tactag                              1116
<210> 12
   <211> 538
   <212> PRT
   <213> Clostridium kluyveri
<400> 12
   Met Ser Lys Gly Ile Lys Asn Ser Gln Leu Lys Lys Lys Asn Val Lys
   1               5                   10                  15
   Ala Ser Asn Val Ala Glu Lys Ile Glu Glu Lys Val Glu Lys Thr Asp
               20                  25                  30
   Lys Val Val Glu Lys Ala Ala Glu Val Thr Glu Lys Arg Ile Arg Asn
           35                  40                  45
   Leu Lys Leu Gln Glu Lys Val Val Thr Ala Asp Val Ala Ala Asp Met
       50                  55                  60
   Ile Glu Asn Gly Met Ile Val Ala Ile Ser Gly Phe Thr Pro Ser Gly
   65                  70                  75                  80
   Tyr Pro Lys Glu Val Pro Lys Ala Leu Thr Lys Lys Val Asn Ala Leu
                   85                  90                  95
   Glu Glu Glu Phe Lys Val Thr Leu Tyr Thr Gly Ser Ser Thr Gly Ala
               100                 105                 110
   Asp Ile Asp Gly Glu Trp Ala Lys Ala Gly Ile Ile Glu Arg Arg Ile
           115                 120                 125
   Pro Tyr Gln Thr Asn Ser Asp Met Arg Lys Lys Ile Asn Asp Gly Ser
       130                 135                 140
   Ile Lys Tyr Ala Asp Met His Leu Ser His Met Ala Gln Tyr Ile Asn
   145                 150                 155                 160
   Tyr Ser Val Ile Pro Lys Val Asp Ile Ala Ile Ile Glu Ala Val Ala
                   165                 170                 175
   Ile Thr Glu Glu Gly Asp Ile Ile Pro Ser Thr Gly Ile Gly Asn Thr
               180                 185                 190
   Ala Thr Phe Val Glu Asn Ala Asp Lys Val Ile Val Glu Ile Asn Glu
           195                 200                 205
   Ala Gln Pro Leu Glu Leu Glu Gly Met Ala Asp Ile Tyr Thr Leu Lys
       210                 215                 220
   Asn Pro Pro Arg Arg Glu Pro Ile Pro Ile Val Asn Ala Gly Asn Arg
   225                 230                 235                 240
   Ile Gly Thr Thr Tyr Val Thr Cys Gly Ser Glu Lys Ile Cys Ala Ile
                           245                 250                 255
   Val Met Thr Asn Thr Gln Asp Lys Thr Arg Pro Leu Thr Glu Val Ser
                   260                 265                 270
   Pro Val Ser Gln Ala Ile Ser Asp Asn Leu Ile Gly Phe Leu Asn Lys
           275                 280                 285
   Glu Val Glu Glu Gly Lys Leu Pro Lys Asn Leu Leu Pro Ile Gln Ser
       290                 295                 300
   Gly Val Gly Ser Val Ala Asn Ala Val Leu Ala Gly Leu Cys Glu Ser
   305                 310                 315                 320
   Asn Phe Lys Asn Leu Ser Cys Tyr Thr Glu Val Ile Gln Asp Ser Met
                   325                 330                 335
   Leu Lys Leu Ile Lys Cys Gly Lys Ala Asp Val Val Ser Gly Thr Ser
               340                 345                 350
   Ile Ser Pro Ser Pro Glu Met Leu Pro Glu Phe Ile Lys Asp Ile Asn
           355                 360                 365
   Phe Phe Arg Glu Lys Ile Val Leu Arg Pro Gln Glu Ile Ser Asn Asn
       370                 375                 380
   Pro Glu Ile Ala Arg Arg Ile Gly Val Ile Ser Ile Asn Thr Ala Leu
   385                 390                 395                 400
   Glu Val Asp Ile Tyr Gly Asn Val Asn Ser Thr His Val Met Gly Ser
                   405                 410                 415
   Lys Met Met Asn Gly Ile Gly Gly Ser Gly Asp Phe Ala Arg Asn Ala
               420                 425                 430
   Tyr Leu Thr Ile Phe Thr Thr Glu Ser Ile Ala Lys Lys Gly Asp Ile
           435                 440                 445
   Ser Ser Ile Val Pro Met Val Ser His Val Asp His Thr Glu His Asp
       450                 455                 460
   Val Met Val Ile Val Thr Glu Gln Gly Val Ala Asp Leu Arg Gly Leu
   465                 470                 475                 480
   Ser Pro Arg Glu Lys Ala Val Ala Ile Ile Glu Asn Cys Val His Pro
                   485                 490                 495
   Asp Tyr Lys Asp Met Leu Met Glu Tyr Phe Glu Glu Ala Cys Lys Ser
               500                 505                 510
   Ser Gly Gly Asn Thr Pro His Asn Leu Glu Lys Ala Leu Ser Trp His
           515                 520                 525
   Thr Lys Phe Ile Lys Thr Gly Ser Met Lys
       530                 535
<210> 13
   <211> 1617
   <212> DNA
   <213> Clostridium kluyveri
<400> 13
   atgtctaaag gaatcaagaa tagccaattg aaaaaaaaga acgtcaaggc cagtaacgtt      60
   gctgagaaga tcgaagagaa ggtggaaaag accgacaagg tcgttgagaa ggctgctgag     120
   gtgaccgaaa agcgcattcg aaacttaaag ctccaggaaa aagttgtgac cgcagatgtc     180
   gcagctgaca tgatcgagaa tggcatgatc gtcgcaatta gcggcttcac gccatccggg     240
   tatccaaagg aggttccaaa agcccttact aagaaggtta atgcgctgga ggaggagttc     300
   aaggtgacgc tgtataccgg ttctagcaca ggcgctgata ttgacggaga atgggcgaag     360
   gcaggaataa tcgaacggcg tatcccatac cagaccaact ctgacatgag gaaaaaaata     420
   aacgatggtt caatcaagta cgcagatatg cacctgagcc acatggctca atacattaac     480
   tattctgtga ttcctaaggt tgacattgcc atcatcgagg cggtggccat taccgaggaa     540
   ggggatatta ttcctagtac tggaatcggc aacacagcta cgtttgtcga gaatgcggat     600
   aaggtaattg tggaaataaa cgaggctcag ccgcttgagt tggaaggcat ggcagatatc     660
   tataccctga agaaccctcc acgtcgcgag cccatcccga tagtcaacgc aggcaaccgc     720
   atagggacca cttacgtcac ctgtggctct gaaaaaatct gcgcgatcgt catgaccaac     780
   acccaagaca aaacccgccc actcaccgaa gtttctcctg tcagtcaggc aatctccgat     840
   aacctgattg gcttcctgaa caaagaagta gaggagggta aactcccaaa aaacctgctc     900
   cccatacagt caggtgtcgg ttcggttgct aacgccgttc tagccggact ctgcgaatca     960
   aacttcaaaa atttgagctg ctacacagaa gtgatccagg attcgatgtt gaagctcatc    1020
   aaatgtggaa aggcagatgt ggtgtccggc acctcgatct cgccatcacc ggaaatgctg    1080
   cccgagttca taaaggacat aaattttttt cgcgagaaga tagtactgcg cccccaggaa    1140
   atatctaata atccggaaat agctcgtcgt ataggagtga tctccataaa cactgctttg    1200
   gaagtagaca tctacggtaa tgtgaactcc acgcatgtca tgggctccaa gatgatgaac    1260
   ggcatcggcg gcagcggcga ctttgcccgc aacgcatacc tcaccatatt cactacggag    1320
   tccatcgcga agaagggcga catttcctct atcgttccta tggtttccca cgtggaccac    1380
   accgagcatg acgtaatggt catcgttacc gaacaggggg ttgcggatct gcgcggtctt    1440
   tcccctcggg aaaaggccgt ggcgataatt gagaattgcg tccacccgga ttacaaggat    1500
   atgctcatgg agtacttcga ggaggcttgt aagtcctcag gtggcaacac cccacacaac    1560
   cttgaaaaag ccctatcctg gcacactaag ttcataaaaa ctggctcgat gaagtaa       1617
<210> 14
   <211> 431
   <212> PRT
   <213> Porphyromonas gingivalis
<400> 14
   Met Lys Asp Val Leu Ala Glu Tyr Ala Ser Arg Ile Val Ser Ala Glu
   1               5                   10                  15
   Glu Ala Val Lys His Ile Lys Asn Gly Glu Arg Val Ala Leu Ser His
               20                  25                  30
   Ala Ala Gly Val Pro Gln Ser Cys Val Asp Ala Leu Val Gln Gln Ala
           35                  40                  45
   Asp Leu Phe Gln Asn Val Glu Ile Tyr His Met Leu Cys Leu Gly Glu
       50                  55                  60
   Gly Lys Tyr Met Ala Pro Glu Met Ala Pro His Phe Arg His Ile Thr
   65                  70                  75                  80
   Asn Phe Val Gly Gly Asn Ser Arg Lys Ala Val Glu Glu Asn Arg Ala
                   85                  90                  95
   Asp Phe Ile Pro Val Phe Phe Tyr Glu Val Pro Ser Met Ile Arg Lys
               100                 105                 110
   Asp Ile Leu His Ile Asp Val Ala Ile Val Gln Leu Ser Met Pro Asp
           115                 120                 125
   Glu Asn Gly Tyr Cys Ser Phe Gly Val Ser Cys Asp Tyr Ser Lys Pro
       130                 135                 140
   Ala Ala Glu Ser Ala His Leu Val Ile Gly Glu Ile Asn Arg Gln Met
   145                 150                 155                 160
   Pro Tyr Val His Gly Asp Asn Leu Ile His Ile Ser Lys Leu Asp Tyr
                   165                 170                 175
   Ile Val Met Ala Asp Tyr Pro Ile Tyr Ser Leu Ala Lys Pro Lys Ile
               180                 185                 190
   Gly Glu Val Glu Glu Ala Ile Gly Arg Asn Cys Ala Glu Leu Ile Glu
           195                 200                 205
   Asp Gly Ala Thr Leu Gln Leu Gly Ile Gly Ala Ile Pro Asp Ala Ala
       210                 215                 220
   Leu Leu Phe Leu Lys Asp Lys Lys Asp Leu Gly Ile His Thr Glu Met
   225                 230                 235                 240
   Phe Ser Asp Gly Val Val Glu Leu Val Arg Ser Gly Val Ile Thr Gly
                   245                 250                 255
   Lys Lys Lys Thr Leu His Pro Gly Lys Met Val Ala Thr Phe Leu Met
               260                 265                 270
   Gly Ser Glu Asp Val Tyr His Phe Ile Asp Lys Asn Pro Asp Val Glu
           275                 280                 285
   Leu Tyr Pro Val Asp Tyr Val Asn Asp Pro Arg Val Ile Ala Gln Asn
       290                 295                 300
   Asp Asn Met Val Ser Ile Asn Ser Cys Ile Glu Ile Asp Leu Met Gly
   305                 310                 315                 320
   Gln Val Val Ser Glu Cys Ile Gly Ser Lys Gln Phe Ser Gly Thr Gly
                   325                 330                 335
   Gly Gln Val Asp Tyr Val Arg Gly Ala Ala Trp Ser Lys Asn Gly Lys
               340                 345                 350
   Ser Ile Met Ala Ile Pro Ser Thr Ala Lys Asn Gly Thr Ala Ser Arg
           355                 360                 365
   Ile Val Pro Ile Ile Ala Glu Gly Ala Ala Val Thr Thr Leu Arg Asn
       370                 375                 380
   Glu Val Asp Tyr Val Val Thr Glu Tyr Gly Ile Ala Gln Leu Lys Gly
   385                 390                 395                 400
   Lys Ser Leu Arg Gln Arg Ala Glu Ala Leu Ile Ala Ile Ala His Pro
                   405                 410                 415
   Asp Phe Arg Glu Glu Leu Thr Lys His Leu Arg Lys Arg Phe Gly
               420                 425                 430
<210> 15
   <211> 1296
   <212> DNA
   <213> Porphyromonas gingivalis
<400> 15
   atgaaggatg tactggcgga atacgcctcc cgcattgttt cggcggagga ggccgttaag      60
   cacatcaaaa acggtgaacg ggtagctttg tcacacgctg ccggcgtgcc tcagagttgc     120
   gttgacgcac tggtgcagca ggccgacctt ttccagaatg tggaaatcta tcacatgctg     180
   tgcctcggtg agggtaagta tatggcgcct gagatggccc ctcacttccg ccacatcacc     240
   aactttgtcg gtggtaactc ccgtaaggcg gtcgaagaaa accgggccga tttcattccg     300
   gtattctttt acgaggtgcc aagcatgatt cgcaaagaca tcctccacat tgatgtcgcc     360
   atcgttcagc tttcaatgcc tgacgaaaat ggttactgtt cctttggagt atcttgcgat     420
   tactccaagc cggcagcaga gagcgctcac ctggttatcg gagaaatcaa ccgtcaaatg     480
   ccatacgtac acggcgacaa cttgattcat atctccaagt tggattacat cgtgatggca     540
   gactacccca tctactctct tgcaaagccc aagatcgggg aagtcgagga agctatcggg     600
   aggaattgtg ccgagcttat tgaagatggt gccactctcc agctgggaat cggcgcgatt     660
   cctgatgcgg ccctgttatt tctcaaggac aaaaaggatc tgggcatcca taccgaaatg     720
   ttctccgatg gtgttgtcga attggttcgc tccggcgtta tcacaggcaa gaaaaagact     780
   cttcaccccg gaaagatggt cgcaaccttc ctgatgggaa gcgaggacgt gtatcatttc     840
   atcgataaaa accccgatgt agaactgtat ccagtagatt acgtgaatga cccgcgtgtg     900
   atcgcccaaa acgacaatat ggtctcgatt aacagctgca tcgaaatcga ccttatggga     960
   caggtcgtgt ccgagtgcat cggctcaaag caattcagcg gcaccggcgg ccaagttgac    1020
   tacgtgcgtg gcgcagcatg gtctaaaaac ggcaaatcga tcatggcaat cccgtccact    1080
   gcaaaaaacg gtacggcatc tcgaattgta cctatcatcg cggagggcgc tgctgtcacc    1140
   accctgcgca acgaggtcga ttacgttgta accgagtacg gtatcgctca gctcaagggc    1200
   aagagcctgc gccagcgcgc agaggctttg atcgcgatag cccaccccga cttccgtgag    1260
   gaactaacga aacatctccg caagcgattc ggataa                              1296
<210> 16
   <211> 858
   <212> PRT
   <213> Clostridium acetobutylicum
<400> 16
   Met Lys Val Thr Asn Gln Lys Glu Leu Lys Gln Lys Leu Asn Glu Leu
   1               5                   10                  15
   Arg Glu Ala Gln Lys Lys Phe Ala Thr Tyr Thr Gln Glu Gln Val Asp
               20                  25                  30
   Lys Ile Phe Lys Gln Cys Ala Ile Ala Ala Ala Lys Glu Arg Ile Asn
           35                  40                  45
   Leu Ala Lys Leu Ala Val Glu Glu Thr Gly Ile Gly Leu Val Glu Asp
       50                  55                  60
   Lys Ile Ile Lys Asn His Phe Ala Ala Glu Tyr Ile Tyr Asn Lys Tyr
   65                  70                  75                  80
   Lys Asn Glu Lys Thr Cys Gly Ile Ile Asp His Asp Asp Ser Leu Gly
                   85                  90                  95
   Ile Thr Lys Val Ala Glu Pro Ile Gly Ile Val Ala Ala Ile Val Pro
               100                 105                 110
   Thr Thr Asn Pro Thr Ser Thr Ala Ile Phe Lys Ser Leu Ile Ser Leu
           115                 120                 125
   Lys Thr Arg Asn Ala Ile Phe Phe Ser Pro His Pro Arg Ala Lys Lys
       130                 135                 140
   Ser Thr Ile Ala Ala Ala Lys Leu Ile Leu Asp Ala Ala Val Lys Ala
   145                 150                 155                 160
   Gly Ala Pro Lys Asn Ile Ile Gly Trp Ile Asp Glu Pro Ser Ile Glu
                   165                 170                 175
   Leu Ser Gln Asp Leu Met Ser Glu Ala Asp Ile Ile Leu Ala Thr Gly
               180                 185                 190
   Gly Pro Ser Met Val Lys Ala Ala Tyr Ser Ser Gly Lys Pro Ala Ile
           195                 200                 205
   Gly Val Gly Ala Gly Asn Thr Pro Ala Ile Ile Asp Glu Ser Ala Asp
       210                 215                 220
   Ile Asp Met Ala Val Ser Ser Ile Ile Leu Ser Lys Thr Tyr Asp Asn
   225                 230                 235                 240
   Gly Val Ile Cys Ala Ser Glu Gln Ser Ile Leu Val Met Asn Ser Ile
                   245                 250                 255
   Tyr Glu Lys Val Lys Glu Glu Phe Val Lys Arg Gly Ser Tyr Ile Leu
               260                 265                 270
   Asn Gln Asn Glu Ile Ala Lys Ile Lys Glu Thr Met Phe Lys Asn Gly
           275                 280                 285
   Ala Ile Asn Ala Asp Ile Val Gly Lys Ser Ala Tyr Ile Ile Ala Lys
       290                 295                 300
   Met Ala Gly Ile Glu Val Pro Gln Thr Thr Lys Ile Leu Ile Gly Glu
   305                 310                 315                 320
   Val Gln Ser Val Glu Lys Ser Glu Leu Phe Ser His Glu Lys Leu Ser
                   325                 330                 335
   Pro Val Leu Ala Met Tyr Lys Val Lys Asp Phe Asp Glu Ala Leu Lys
               340                 345                 350
   Lys Ala Gln Arg Leu Ile Glu Leu Gly Gly Ser Gly His Thr Ser Ser
           355                 360                 365
   Leu Tyr Ile Asp Ser Gln Asn Asn Lys Asp Lys Val Lys Glu Phe Gly
       370                 375                 380
   Leu Ala Met Lys Thr Ser Arg Thr Phe Ile Asn Met Pro Ser Ser Gln
   385                 390                 395                 400
   Gly Ala Ser Gly Asp Leu Tyr Asn Phe Ala Ile Ala Pro Ser Phe Thr
                   405                 410                 415
   Leu Gly Cys Gly Thr Trp Gly Gly Asn Ser Val Ser Gln Asn Val Glu
               420                 425                 430
   Pro Lys His Leu Leu Asn Ile Lys Ser Val Ala Glu Arg Arg Glu Asn
           435                 440                 445
   Met Leu Trp Phe Lys Val Pro Gln Lys Ile Tyr Phe Lys Tyr Gly Cys
       450                 455                 460
   Leu Arg Phe Ala Leu Lys Glu Leu Lys Asp Met Asn Lys Lys Arg Ala
   465                 470                 475                 480
   Phe Ile Val Thr Asp Lys Asp Leu Phe Lys Leu Gly Tyr Val Asn Lys
                   485                 490                 495
   Ile Thr Lys Val Leu Asp Glu Ile Asp Ile Lys Tyr Ser Ile Phe Thr
               500                 505                 510
   Asp Ile Lys Ser Asp Pro Thr Ile Asp Ser Val Lys Lys Gly Ala Lys
           515                 520                 525
   Glu Met Leu Asn Phe Glu Pro Asp Thr Ile Ile Ser Ile Gly Gly Gly
       530                 535                 540
   Ser Pro Met Asp Ala Ala Lys Val Met His Leu Leu Tyr Glu Tyr Pro
   545                 550                 555                 560
   Glu Ala Glu Ile Glu Asn Leu Ala Ile Asn Phe Met Asp Ile Arg Lys
                   565                 570                 575
   Arg Ile Cys Asn Phe Pro Lys Leu Gly Thr Lys Ala Ile Ser Val Ala
               580                 585                 590
   Ile Pro Thr Thr Ala Gly Thr Gly Ser Glu Ala Thr Pro Phe Ala Val
           595                 600                 605
   Ile Thr Asn Asp Glu Thr Gly Met Lys Tyr Pro Leu Thr Ser Tyr Glu
       610                 615                 620
   Leu Thr Pro Asn Met Ala Ile Ile Asp Thr Glu Leu Met Leu Asn Met
   625                 630                 635                 640
   Pro Arg Lys Leu Thr Ala Ala Thr Gly Ile Asp Ala Leu Val His Ala
                   645                 650                 655
   Ile Glu Ala Tyr Val Ser Val Met Ala Thr Asp Tyr Thr Asp Glu Leu
               660                 665                 670
   Ala Leu Arg Ala Ile Lys Met Ile Phe Lys Tyr Leu Pro Arg Ala Tyr
           675                 680                 685
   Lys Asn Gly Thr Asn Asp Ile Glu Ala Arg Glu Lys Met Ala His Ala
       690                 695                 700
   Ser Asn Ile Ala Gly Met Ala Phe Ala Asn Ala Phe Leu Gly Val Cys
   705                 710                 715                 720
   His Ser Met Ala His Lys Leu Gly Ala Met His His Val Pro His Gly
                   725                 730                 735
   Ile Ala Cys Ala Val Leu Ile Glu Glu Val Ile Lys Tyr Asn Ala Thr
               740                 745                 750
   Asp Cys Pro Thr Lys Gln Thr Ala Phe Pro Gln Tyr Lys Ser Pro Asn
           755                 760                 765
   Ala Lys Arg Lys Tyr Ala Glu Ile Ala Glu Tyr Leu Asn Leu Lys Gly
       770                 775                 780
   Thr Ser Asp Thr Glu Lys Val Thr Ala Leu Ile Glu Ala Ile Ser Lys
   785                 790                 795                 800
   Leu Lys Ile Asp Leu Ser Ile Pro Gln Asn Ile Ser Ala Ala Gly Ile
                   805                 810                 815
   Asn Lys Lys Asp Phe Tyr Asn Thr Leu Asp Lys Met Ser Glu Leu Ala
               820                 825                 830
   Phe Asp Asp Gln Cys Thr Thr Ala Asn Pro Arg Tyr Pro Leu Ile Ser
           835                 840                 845
   Glu Leu Lys Asp Ile Tyr Ile Lys Ser Phe
       850                 855
<210> 17
   <211> 2577
   <212> DNA
   <213> Clostridium acetobutylicum
<400> 17
   atgaaagtaa ccaatcagaa agagttgaag cagaagttga acgagctgcg agaggctcag 60
   aagaagttcg caacctacac ccaggaacag gtggacaaga tctttaagca gtgtgccatt 120
   gcagccgcga aagaacgtat taatctcgcg aaacttgcgg tcgaggaaac cggtattggg 180
   ctggtagaag acaagatcat caagaaccac ttcgccgctg aatacatcta caacaagtac 240
   aaaaacgaaa agacatgtgg tatcatcgac cacgacgaca gcttgggcat caccaaggta 300
   gcggagccaa tcggtatcgt cgcagctatc gtgcccacta ctaaccctac ctccactgct 360
   attttcaagt cactcatctc cctgaaaacc cgcaatgcta tcttcttctc acctcaccca 420
   cgcgctaaga aatcaactat cgctgcagct aaacttatcc tggatgcagc cgtgaaagcc 480
   ggggctccga aaaacatcat cggttggatc gacgaacctt ccattgaact ctctcaagac 540
   ctcatgtccg aggcagacat tatcctggca accggaggcc catccatggt taaagcagct 600
   tacagctcag gcaagccggc tatcggcgtt ggagctggta acactccagc aatcatcgac 660
   gagtcggccg atatcgacat ggcagtgtcc tctattatcc tgtccaaaac ttatgacaac 720
   ggcgttattt gcgcgtccga gcagtctatt ctcgtcatga actctattta cgagaaggta 780
   aaggaggagt ttgtgaagcg ggggtcgtac attctgaacc agaacgagat cgctaagatc 840
   aaagagacta tgtttaaaaa cggagccatc aacgcagata tcgtagggaa gtccgcgtac 900
   atcattgcta agatggctgg aatcgaagtc cctcaaacca cgaaaattct gatcggcgag 960
   gtgcaatcgg tcgaaaagtc cgagctgttc tcgcatgaaa agttgtcccc ggtcctcgcg 1020
   atgtataaag ttaaggattt tgatgaagca ctcaagaaag ctcagcgcct gatcgaattg 1080
   ggtggctcgg gtcacacctc ttccctctac attgactccc agaacaataa agataaggtg 1140
   aaagagttcg gcctggctat gaagacgtct cgtaccttca tcaatatgcc ctcttcacag 1200
   ggcgccagcg gtgaccttta caatttcgct atcgctccta gctttaccct cggctgcggc 1260
   acctggggcg gtaattctgt gtcccaaaac gtcgaaccaa agcatctgct caacattaaa 1320
   agcgtcgccg aacgtcgcga gaacatgttg tggttcaagg tcccgcaaaa aatctacttc 1380
   aagtatggtt gcttgcgctt tgcacttaaa gagcttaagg acatgaataa aaagcgggcg 1440
   ttcatcgtca ctgataagga tctgttcaaa ctgggctatg ttaacaagat taccaaggtc 1500
   ctggatgaga tcgatatcaa gtattccatc ttcaccgata ttaagtccga tccgaccatt 1560
   gattccgtga agaagggcgc gaaggagatg ctcaactttg aacccgacac gattatttct 1620
   attggcggag gcagcccaat ggacgcagct aaggttatgc acctgctgta tgagtaccca 1680
   gaagcagaga tcgagaacct tgcaatcaat ttcatggata ttcgcaaacg catttgcaac 1740
   tttcctaagc ttggtacaaa agctatctct gttgcgatcc ctaccaccgc aggaaccggc 1800
   agcgaagcga caccattcgc cgttattacc aacgatgaaa caggtatgaa gtacccactt 1860
   acctcttatg aacttacccc gaacatggct atcattgata cggaattgat gctgaacatg 1920
   ccgcggaagt tgaccgcagc tacgggaatc gatgcattgg ttcatgcaat cgaggcatac 1980
   gtttccgtca tggcaaccga ttacaccgac gagctcgcgt tgcgtgcgat taaaatgatc 2040
   ttcaagtacc ttccacgcgc atacaagaat ggcacaaacg atattgaagc ccgagaaaag 2100
   atggcacacg cttcgaacat cgctggtatg gccttcgcga atgcgtttct cggagtgtgt 2160
   cactccatgg cgcacaaact gggagccatg catcacgtgc cccacggtat cgcatgcgcc 2220
   gttcttattg aagaggtgat caagtataat gccaccgatt gccccactaa gcagacggcc 2280
   ttccctcagt acaaatcgcc caatgccaag cgtaaatacg cggaaattgc cgagtacttg 2340
   aaccttaagg ggaccagcga cacggaaaag gtgaccgcac tgattgaagc catctccaag 2400
   cttaagatcg acctgagcat cccacaaaac atctcagcag ccggcattaa caagaaggac 2460
   ttctacaaca ctctcgacaa gatgtcagag ctcgccttcg atgatcagtg cactaccgca 2520
   aacccacgtt atccgctcat ctctgaactg aaggatatct acatcaagtc gttttaa 2577
<210> 18
   <211> 43
   <212> DNA
   <213> Artificial Sequence
<220>
   <223> ldhA_5'_HindIII
<400> 18
   catgattacg ccaagcttga gagcccacca cattgcgatt tcc 43
<210> 19
   <211> 42
   <212> DNA
   <213> Artificial Sequence
<220>
   <223> ldhA up_3' XhoI
<400> 19
   tcgaaactcg agtttcgatc ccacttcctg atttccctaa cc 42
<210> 20
   <211> 39
   <212> DNA
   <213> Artificial Sequence
<220>
   <223> ldhA_dn_5'_XhoI
<400> 20
   tcgaaactcg agtaaatctt tggcgcctag ttggcgacg 39
<210> 21
   <211> 46
   <212> DNA
   <213> Artificial Sequence
<220>
   <223> ldhA_3_EcoRI
<400> 21
   acgacggcca gtgaattcga cgacatctga gggtggataa agtggg 46
<210> 22
   <211> 20
   <212> DNA
   <213> Artificial Sequence
<220>
   <223> ldhA up
<400> 22
   atcgggcata attaaaggtg 20
<210> 23
   <211> 22
   <212> DNA
   <213> Artificial Sequence
<220>
   <223> ldhA down
<400> 23
   gtcacctcat caagttctag aa 22
<210> 24
   <211> 36
   <212> DNA
   <213> Artificial Sequence
<220>
   <223> 0049-1 for
<400> 24
   gcaggcatgc aagcttaaag tccccacggg tctact 36
<210> 25
   <211> 35
   <212> DNA
   <213> Artificial Sequence
<220>
   <223> 0049-1 rev
<400> 25
   gagctcagtc agtcacggag accacattga ggaca 35
<210> 26
   <211> 35
   <212> DNA
   <213> Artificial Sequence
<220>
   <223> 0049-2 for
<400> 26
   tgactgactg agctcccaaa atgcgcaaca tcggc 35
<210> 27
   <211> 36
   <212> DNA
   <213> Artificial Sequence
<220>
   <223> 0049-2 rev
<400> 27
   ggccagtgcc aagctttacc gatgtactgc acggag 36
<210> 28
   <211> 36
   <212> DNA
   <213> Artificial Sequence
<220>
   <223> 0463-1 for
<400> 28
   gcaggcatgc aagcttgcac cagaaggtgg gctaaa 36
<210> 29
   <211> 35
   <212> DNA
   <213> Artificial Sequence
<220>
   <223> 0463-1 rev
<400> 29
   gagctcagtc agtcatgccg atagctctgc atctc 35
<210> 30
   <211> 35
   <212> DNA
   <213> Artificial Sequence
<220>
   <223> 0463-2 for
<400> 30
   tgactgactg agctcgtatg catcgcagct aaccg 35
<210> 31
   <211> 36
   <212> DNA
   <213> Artificial Sequence
<220>
   <223> 0463-2 rev
<400> 31
   ggccagtgcc aagctttacc tgcagcattg tgcgca 36
<210> 32
   <211> 36
   <212> DNA
   <213> Artificial Sequence
<220>
   <223> 2619-1 for
<400> 32
   gcaggcatgc aagcttgtct tcctttaggt ccgctg 36
<210> 33
   <211> 35
   <212> DNA
   <213> Artificial Sequence
<220>
   <223> 2619-1 rev
<400> 33
   gagctcagtc agtcaccggt gacaacctgc atcaa 35
<210> 34
   <211> 35
   <212> DNA
   <213> Artificial Sequence
<220>
   <223> 2619-2 for
<400> 34
   tgactgactg agctcggtgc cggatttgag tggaa 35
<210> 35
   <211> 36
   <212> DNA
   <213> Artificial Sequence
<220>
   <223> 2619-2 rev
<400> 35
   ggccagtgcc aagcttttgc ccagctttag cgctgt 36
<210> 36
   <211> 20
   <212> DNA
   <213> Artificial Sequence
<220>
   <223> 0049for
<400> 36
   gtgatgctgg taaacctgtg 20
<210> 37
   <211> 19
   <212> DNA
   <213> Artificial Sequence
<220>
   <223> 0049rev
<400> 37
   ttgcgtttgg gaaatacgg 19
<210> 38
   <211> 20
   <212> DNA
   <213> Artificial Sequence
<220>
   <223> 0463for
<400> 38
   gcaccagaag gtgggctaaa 20
<210> 39
   <211> 20
   <212> DNA
   <213> Artificial Sequence
<220>
   <223> 0463rev
<400> 39
   tacctgcagc attgtgcgca 20
<210> 40
   <211> 20
   <212> DNA
   <213> Artificial Sequence
<220>
   <223> 2619for
<400> 40
   gtcttccttt aggtccgctg 20
<210> 41
   <211> 20
   <212> DNA
   <213> Artificial Sequence
<220>
   <223> 2619rev
<400> 41
   ttgcccagct ttagcgctgt 20
<210> 42
   <211> 4990
   <212> DNA
   <213> Artificial Sequence
<220>
   <223> pGSK+
<400> 42
   ttgagatcct ttttttctgc gcgtaatctg ctgcttgcaa acaaaaaaac caccgctacc     60
   agcggtggtt tgtttgccgg atcaagagct accaactctt tttccgaagg taactggctt    120
   cagcagagcg cagataccaa atactgttct tctagtgtag ccgtagttag gccaccactt    180
   caagaactct gtagcaccgc ctacatacct cgctctgcta atcctgttac cagtggctgc    240
   tgccagtggc gataagtcgt gtcttaccgg gttggactca agacgatagt taccggataa    300
   ggcgcagcgg tcgggctgaa cggggggttc gtgcacacag cccagcttgg agcgaacgac    360
   ctacaccgaa ctgagatacc tacagcgtga gctatgagaa agcgccacgc ttcccgaagg    420
   gagaaaggcg gacaggtatc cggtaagcgg cagggtcgga acaggagagc gcacgaggga    480
   gcttccaggg ggaaacgcct ggtatcttta tagtcctgtc gggtttcgcc acctctgact    540
   tgagcgtcga tttttgtgat gctcgtcagg ggggcggagc ctatggaaaa acgcccctct    600
   tcgctattac gccagctggc gaaaggggga tgtgctgcaa ggcgattaag ttgggtaacg    660
   ccagggtttt cccagtcacg acgttgtaaa acgacggcca gtgaattgta atacgactca    720
   ctatagggcg aattgggtac cgggcccccc ctcgaggtcg acggtatcga taagcttgat    780
   atcgaattcc tgcagcccgg gggatccact agttctagag cggccgccac cgcggtggag    840
   ctccagcttt tgttcccttt agtgagggtt aatttcgagc ttggcgtaat catggtcata    900
   gctgtttcct gtgtgaaatt gttatccgct cacaattcca cacaacatac gagccggaag    960
   cataaagtgt aaagcctggg aacaacaaga cccatcatag tttgcccccg cgacattgac   1020
   cataaattca tcgcacaaaa tatcgaacgg ggtttatgcc gcttttagtg ggtgcgaaga   1080
   atagtctgct cattacccgc gaacaccgcc gcattcagat cacgcttagt agcgtcccca   1140
   tgagtaggca gaaccgcgtc caagtccaca tcatccataa cgatcatgca cggggtggaa   1200
   tccacaccca gacttgccag cacctcatta gcgacacgtt gcgcagcggc cacgtcctta 1260
   gccttatcca cgcaatctag gacgtactgc ctaaccgcga aatcagactg aatcagtttc 1320
   caatcatcgg gcttcaccaa agcaacagca acgcgggttg attcgacccg ttccggtgct 1380
   tccagaccgg cgagcttgta cagttcttct tccatttcac gacgtacatc agcgtctatg 1440
   taatcaatgc ccaaagcacg cttagcccca cgtgaccagg acgaacgcag gtttttagaa 1500
   ccaacctcat actcacgcca ccgagccacc aaaacagcgt ccatatcctc gccggcgtcg 1560
   ctttgatcgg ccaacatatc caacatctga aacggcgtgt acgacccctt agacgcggtt 1620
   ttagtagcgg agccagtcag ttcctgagac atgcccttag cgaggtaggt tgccattttc 1680
   gcagcgtctc caccccaggt agacacctga tcaagtttga ccccgtgctc acgcagtggc 1740
   gcgtccatac cggccttaac cacaccagca gaccagcggg aaaacatgga atcctcaaac 1800
   gccttgagtt catcgtcaga cagtggacga tccaagaaca acagcatgtt gcggtgcaag 1860
   tgccaaccgt tcgcccaaga gtctgtgacc tcatagtcac tataggtgtg ctccaccccg 1920
   taccgtgcac gttctttctt ccactgagat gttttcacca tcgaagagta cgcagtctta 1980
   atacccgctt caacctgcgc aaatgactgt gagcggttgt gtcgaacagt gcccacaaac 2040
   atcatgagcg cgccacccgc cgccaagtga ttcttagtag caatagccag ctcaatgcgg 2100
   cgttcgccca tgacttccaa ttcagccaga ggtgaccccc agcgagagtg agagttttgc 2160
   agaccctcaa actgcgaagc accgttagac gaccaggaca ccgcaacagc ttcgtccctg 2220
   cgccacctat ggcaccccgc cagagcctta ctattggtga tcttgtacat gacgttttgc 2280
   ctacgccacg ccctagcgcg agtgacctta gaaccctcat tgacctgcgg ttccttagag 2340
   gtgttcactt ctatttcagt gttacctaga cccgatgttg tgcggggttg cgcagtgcga 2400
   gtttgtgcgg gtgttgtgcc cgttgtctta gctagtgcta tggttgtcaa ttgaaacccc 2460
   ttcgggttat gtggcccccg tgcatatgag ttggtagctc gcacgggggt ttgtcttgtc 2520
   taggaactat taatttttag tggtgtttgg tggccgccta gcttggctat gcgtgccagc 2580
   ttacccgtac tcaatgttaa agatttgcat cgacatggga gggttacgtg tccgatacct 2640
   agggggggta tccgcgacta ggtgccccgg tgctcactgt ctgtaccggc ggggcaagcc 2700
   ccacaccccg catggacagg gtggctccgc cccctgcacc cccagcaatc tgcatgtaca 2760
   tgttttacac attagcacga catgactgca tgtgcatgca ctgcatgcag actaggtaaa 2820
   tatgagtatg tacgactagt aacaggagca ctgcacataa tgaatgagtt gcaggacaat 2880
   gtttgctacg catgcgcatg acatatcgca ggaaagctac tagagtctta aagcatggca 2940
   accaaggcac agctagaaca gcaactacaa gaagctcaac aggcactaca ggcgcagcaa 3000
   gcgcaggcac aagccaccat cgaagcacta gaagcgcagg caaaggctaa gcccgtcgtg 3060
   gtcaccgcac gcgttccttt ggcactacgt gaggacatga agcgcgcagg catgcagaac 3120
   ggtgaaaacc tccaagagtt catgatcgcc gcgtttaccg agcggctaga aaagctcacc 3180
   accaccgaca acgaggaaaa caatgtctaa cccactagtt ctctttgccc accgtgaccc 3240
   ggtaaatgac gtgacgttcg agtgcattga gcacgccacc tacgacacac tttcacacgc 3300
   taaagaccag atcaccgccc aaatgcaagc cctagacgaa gaagccgccc tactgcccta 3360
   atgggtgttt catgggtgtt tccctagtgt ttcatggtgt tttcacctaa gctagggaat 3420
   tgcgcgagaa gtctcgcaaa aatcagcaac ccccggaacc acacagttca cgggggttct 3480
   tctatgccag aaatcagaaa ggggaaccag tgaacgaccc cgaatattgg atcacagcgc 3540
   agcaggtcgc cgcccgcgta gctctcaccc cggccaccat taaaaagtgg gcaaacgagg 3600
   gaaaaatcac cgcatacaag atcggcaagt ccgtccgatt caaagcatca gacgtagaca 3660
   agctaggggg gggggggcgc tgaggtctgc ctcgtgaaga aggtgttgct gactcatacc 3720
   aggcctgaat cgccccatca tccagccaga aagtgaggga gccacggttg atgagagctt 3780
   tgttgtaggt ggaccagttg gtgattttga acttttgctt tgccacggaa cggtctgcgt 3840
   tgtcgggaag atgcgtgatc tgatccttca actcagcaaa agttcgattt attcaacaaa 3900
   gccgccgtcc cgtcaagtca gcgtaatgct ctgccagtgt tacaaccaat taaccaattc 3960
   tgattagaag aactcgtcaa gaaggcgata gaaggcgatg cgctgcgaat cgggagcggc 4020
   gataccgtaa agcacgagga agcggtcagc ccattcgccg ccaagctctt cagcaatatc 4080
   acgggtagcc aacgctatgt cctgatagcg gtccgccaca cccagccggc cacagtcgat 4140
   gaatccagaa aagcggccat tttccaccat gatattcggc aagcaggcat cgccatgggt 4200
   cacgacgaga tcctcgccgt cgggcatgct cgccttgagc ctggcgaaca gttcggctgg 4260
   cgcgagcccc tgatgctctt cgtccagatc atcctgatcg acaagaccgg cttccatccg 4320
   agtacgtgct cgctcgatgc gatgtttcgc ttggtggtcg aatgggcagg tagccggatc 4380
   aagcgtatgc agccgccgca ttgcatcagc catgatggat actttctcgg caggagcaag 4440
   gtgagatgac aggagatcct gccccggcac ttcgcccaat agcagccagt cccttcccgc 4500
   ttcagtgaca acgtcgagca cagctgcgca aggaacgccc gtcgtggcca gccacgatag 4560
   ccgcgctgcc tcgtcttgca gttcattcag ggcaccggac aggtcggtct tgacaaaaag 4620
   aaccgggcgc ccctgcgctg acagccggaa cacggcggca tcagagcagc cgattgtctg 4680
   ttgtgcccag tcatagccga atagcctctc cacccaagcg gccggagaac ctgcgtgcaa 4740
   tccatcttgt tcaatcataa caccccttgt attactgttt atgtaagcag acagttttat 4800
   tgttcatgat gatatatttt tatcttgtgc aatgtaacat cagagatttt gagacacaac 4860
   gtggctttcc cccccccccc aaaaggatct aggtgaagat cctttttgat aatctcatga 4920
   ccaaaatccc ttaacgtgag ttttcgttcc actgagcgtc agaccccgta gaaaagatca 4980
   aaggatcttc 4990
<210> 43
   <211> 5342
   <212> DNA
   <213> Artificial Sequence
<220>
   <223> pGT1
<400> 43
   ttgagatcct ttttttctgc gcgtaatctg ctgcttgcaa acaaaaaaac caccgctacc      60
   agcggtggtt tgtttgccgg atcaagagct accaactctt tttccgaagg taactggctt     120
   cagcagagcg cagataccaa atactgttct tctagtgtag ccgtagttag gccaccactt     180
   caagaactct gtagcaccgc ctacatacct cgctctgcta atcctgttac cagtggctgc     240
   tgccagtggc gataagtcgt gtcttaccgg gttggactca agacgatagt taccggataa     300
   ggcgcagcgg tcgggctgaa cggggggttc gtgcacacag cccagcttgg agcgaacgac     360
   ctacaccgaa ctgagatacc tacagcgtga gctatgagaa agcgccacgc ttcccgaagg     420
   gagaaaggcg gacaggtatc cggtaagcgg cagggtcgga acaggagagc gcacgaggga     480
   gcttccaggg ggaaacgcct ggtatcttta tagtcctgtc gggtttcgcc acctctgact     540
   tgagcgtcga tttttgtgat gctcgtcagg ggggcggagc ctatggaaaa acgcccctct    600
   tcgctattac gccagctggc gaaaggggga tgtgctgcaa ggcgattaag ttgggtaacg     660
   ccagggtttt cccagtcacg acgttgtaaa acgacggcca gtgaattgta atacgactca     720
   ctatagggcg aattgggtac cgggcccccc ctcgaggtcg acggtatcga taagcttgat     780
   atcgaattcc tgcagcccgg gggatccact agttctagag cggccgccac cgcggtggag     840
   ctcatttagc ggatgattct cgttcaactt cggccgaagc cacttcgtct gtcataatga     900
   cagggatggt ttcggccgtt tttgcaaata aaacgaaagg ctcagtcgaa agactgggcc     960
   tttcgtttta tctgttgttt gtcggtgaac gctctcctga gtaggacaaa tccgccggga    1020
   gcggatttga acgttgcgaa gcaacggccc ggagggtggc gggcaggacg cccgccataa    1080
   actgccaggc atcaaattaa gcagaaggcc atcctgacgg atggcctttt tgcgtttcta    1140
   caaactcttc ctgtcgtcat atctacaagc catcccccca cagatacggt agctccagct    1200
   tttgttccct ttagtgaggg ttaatttcga gcttggcgta atcatggtca tagctgtttc    1260
   ctgtgtgaaa ttgttatccg ctcacaattc cacacaacat acgagccgga agcataaagt    1320
   gtaaagcctg gggtgcctaa tgagtgagct aactcacatt aattgcgttg cgctcactgc   1380
   catcgcacaa aatatcgaac ggggtttatg ccgcttttag tgggtgcgaa gaatagtctg    1440
   ctcattaccc gcgaacaccg ccgcattcag atcacgctta gtagcgtccc catgagtagg    1500
   cagaaccgcg tccaagtcca catcatccat aacgatcatg cacggggtgg aatccacacc    1560
   cagacttgcc agcacctcat tagcgacacg ttgcgcagcg gccacgtcct tagccttatc 1620
   cacgcaatct aggacgtact gcctaaccgc gaaatcagac tgaatcagtt tccaatcatc 1680
   gggcttcacc aaagcaacag caacgcgggt tgattcgacc cgttccggtg cttccagacc 1740
   ggcgagcttg tacagttctt cttccatttc acgacgtaca tcagcgtcta tgtaatcaat 1800
   gcccaaagca cgcttagccc cacgtgacca ggacgaacgc aggtttttag aaccaacctc 1860
   atactcacgc caccgagcca ccaaaacagc gtccatatcc tcgccggcgt cgctttgatc 1920
   ggccaacata tccaacatct gaaacggcgt gtacgacccc ttagacgcgg ttttagtagc 1980
   ggagccagtc agttcctgag acatgccctt agcgaggtag gttgccattt tcgcagcgtc 2040
   tccaccccag gtagacacct gatcaagttt gaccccgtgc tcacgcagtg gcgcgtccat 2100
   accggcctta accacaccag cagaccagcg ggaaaacatg gaatcctcaa acgccttgag 2160
   ttcatcgtca gacagtggac gatccaagaa caacagcatg ttgcggtgca agtgccaacc 2220
   gttcgcccaa gagtctgtga cctcatagtc actataggtg tgctccaccc cgtaccgtgc 2280
   acgttctttc ttccactgag atgttttcac catcgaagag tacgcagtct taatacccgc 2340
   ttcaacctgc gcaaatgact gtgagcggtt gtgtcgaaca gtgcccacaa acatcatgag 2400
   cgcgccaccc gccgccaagt gattcttagt agcaatagcc agctcaatgc ggcgttcgcc 2460
   catgacttcc aattcagcca gaggtgaccc ccagcgagag tgagagtttt gcagaccctc 2520
   aaactgcgaa gcaccgttag acgaccagga caccgcaaca gcttcgtccc tgcgccacct 2580
   atggcacccc gccagagcct tactattggt gatcttgtac atgacgtttt gcctacgcca 2640
   cgccctagcg cgagtgacct tagaaccctc attgacctgc ggttccttag aggtgttcac 2700
   ttctatttca gtgttaccta gacccgatgt tgtgcggggt tgcgcagtgc gagtttgtgc 2760
   gggtgttgtg cccgttgtct tagctagtgc tatggttgtc aattgaaacc ccttcgggtt 2820
   atgtggcccc cgtgcatatg agttggtagc tcgcacgggg gtttgtcttg tctaggaact 2880
   attaattttt agtggtgttt ggtggccgcc tagcttggct atgcgtgcca gcttacccgt 2940
   actcaatgtt aaagatttgc atcgacatgg gagggttacg tgtccgatac ctaggggggg 3000
   tatccgcgac taggtgcccc ggtgctcact gtctgtaccg gcggggcaag ccccacaccc 3060
   cgcatggaca gggtggctcc gccccctgca cccccagcaa tctgcatgta catgttttac 3120
   acattagcac gacatgactg catgtgcatg cactgcatgc agactaggta aatatgagta 3180
   tgtacgacta gtaacaggag cactgcacat aatgaatgag ttgcaggaca atgtttgcta 3240
   cgcatgcgca tgacatatcg caggaaagct actagagtct taaagcatgg caaccaaggc 3300
   acagctagaa cagcaactac aagaagctca acaggcacta caggcgcagc aagcgcaggc 3360
   acaagccacc atcgaagcac tagaagcgca ggcaaaggct aagcccgtcg tggtcaccgc 3420
   acgcgttcct ttggcactac gtgaggacat gaagcgcgca ggcatgcaga acggtgaaaa 3480
   cctccaagag ttcatgatcg ccgcgtttac cgagcggcta gaaaagctca ccaccaccga 3540
   caacgaggaa aacaatgtct aacccactag ttctctttgc ccaccgtgac ccggtaaatg 3600
   acgtgacgtt cgagtgcatt gagcacgcca cctacgacac actttcacac gctaaagacc 3660
   agatcaccgc ccaaatgcaa gccctagacg aagaagccgc cctactgccc taatgggtgt 3720
   ttcatgggtg tttccctagt gtttcatggt gttttcacct aagctaggga attgcgcgag 3780
   aagtctcgca aaaatcagca acccccggaa ccacacagtt cacgggggtt cttctatgcc 3840
   agaaatcaga aaggggaacc agtgaacgac cccgaatatt ggatcacagc gcagcaggtc 3900
   gccgcccgcg tagctctcac cccggccacc attaaaaagt gggcaaacga gggaaaaatc 3960
   accgcataca agatcggcaa gtccgtccga ttcaaagcat cagacgtaga caagctaggg 4020
   gggggggggc gctgaggtct gcctcgtgaa gaaggtgttg ctgactcata ccaggcctga 4080
   atcgccccat catccagcca gaaagtgagg gagccacggt tgatgagagc tttgttgtag 4140
   gtggaccagt tggtgatttt gaacttttgc tttgccacgg aacggtctgc gttgtcggga 4200
   agatgcgtga tctgatcctt caactcagca aaagttcgat ttattcaaca aagccgccgt 4260
   cccgtcaagt cagcgtaatg ctctgccagt gttacaacca attaaccaat tctgattaga 4320
   agaactcgtc aagaaggcga tagaaggcga tgcgctgcga atcgggagcg gcgataccgt 4380
   aaagcacgag gaagcggtca gcccattcgc cgccaagctc ttcagcaata tcacgggtag 4440
   ccaacgctat gtcctgatag cggtccgcca cacccagccg gccacagtcg atgaatccag 4500
   aaaagcggcc attttccacc atgatattcg gcaagcaggc atcgccatgg gtcacgacga 4560
   gatcctcgcc gtcgggcatg ctcgccttga gcctggcgaa cagttcggct ggcgcgagcc 4620
   cctgatgctc ttcgtccaga tcatcctgat cgacaagacc ggcttccatc cgagtacgtg 4680
   ctcgctcgat gcgatgtttc gcttggtggt cgaatgggca ggtagccgga tcaagcgtat 4740
   gcagccgccg cattgcatca gccatgatgg atactttctc ggcaggagca aggtgagatg 4800
   acaggagatc ctgccccggc acttcgccca atagcagcca gtcccttccc gcttcagtga 4860
   caacgtcgag cacagctgcg caaggaacgc ccgtcgtggc cagccacgat agccgcgctg 4920
   cctcgtcttg cagttcattc agggcaccgg acaggtcggt cttgacaaaa agaaccgggc 4980
   gcccctgcgc tgacagccgg aacacggcgg catcagagca gccgattgtc tgttgtgccc 5040
   agtcatagcc gaatagcctc tccacccaag cggccggaga acctgcgtgc aatccatctt 5100
   gttcaatcat aacacccctt gtattactgt ttatgtaagc agacagtttt attgttcatg 5160
   atgatatatt tttatcttgt gcaatgtaac atcagagatt ttgagacaca acgtggcttt 5220
   cccccccccc ccaaaaggat ctaggtgaag atcctttttg ataatctcat gaccaaaatc 5280
   ccttaacgtg agttttcgtt ccactgagcg tcagaccccg tagaaaagat caaaggatct 5340
   tc 5342
<210> 44
   <211> 5693
   <212> DNA
   <213> Artificial Sequence
<220>
   <223> synthetic 4G cassette
<400> 44
   gtcgacatga ttttgcatct gctgcgaaat ctttgtttcc ccgctaaagt tgaggacagg       60
   ttgacacgga gttgactcga cgaattatcc aatgtgagta ggtttggtgc gtgagttgga      120
   aaaattcgcc atactcgccc ttgggttctg tcagctcaag aattcttgag tgaccgatgc      180
   tctgattgac ctaactgctt gacacattgc atttcctaca atctttagag gagacacaac      240
   atgtctaaag gaatcaagaa tagccaattg aaaaaaaaga acgtcaaggc cagtaacgtt      300
   gctgagaaga tcgaagagaa ggtggaaaag accgacaagg tcgttgagaa ggctgctgag      360
   gtgaccgaaa agcgaattcg aaacttaaag ctccaggaaa aagttgtgac cgcagatgtc      420
   gcagctgaca tgatcgagaa tggcatgatc gtcgcaatta gcggcttcac gccatccggg      480
   tatccaaagg aggttccaaa agcccttact aagaaggtta atgcgctgga ggaggagttc      540
   aaggtgacgc tgtataccgg ttctagcaca ggcgctgata ttgacggaga atgggcgaag      600
   gcaggaataa tcgaacggcg tatcccatac cagaccaact ctgacatgag gaaaaaaata      660
   aacgatggtt caatcaagta cgcagatatg cacctgagcc atatggctca atacattaac      720
   tattctgtga ttcctaaggt tgacattgcc atcatcgagg cggtggccat taccgaggaa      780
   ggggatatta ttcctagtac tggaatcggc aacacagcta cgtttgtcga gaatgcggat      840
   aaggtaattg tggaaataaa cgaggctcag ccgcttgagt tggaaggcat ggcagatatc      900
   tataccctga agaaccctcc acgtcgcgag cccatcccga tagtcaacgc aggcaaccgc      960
   atagggacca cttacgtcac ctgtggctct gaaaaaatct gcgcgatcgt catgaccaac     1020
   acccaagaca aaacccgccc actcaccgaa gtttctcctg tcagtcaggc aatctccgat     1080
   aacctgattg gcttcctgaa caaagaagta gaggagggta aactcccaaa aaacctgctc     1140
   cccatacagt caggtgtcgg ttcggttgct aacgccgtgc tagccggact ctgcgaatca     1200
   aacttcaaaa atttgagctg ctacacagaa gtgatccagg attcgatgtt gaagcttatc     1260
   aaatgtggaa aggcagatgt ggtgtccggc acctcgatct cgccatcacc ggaaatgctg     1320
   cccgagttca taaaggacat aaattttttt cgcgagaaga tagtactgcg cccccaggaa     1380
   atatctaata atccggaaat agctcgtcgt ataggagtga tctccataaa cactgctttg     1440
   gaagtagaca tctacggtaa tgtgaactcc acgcatgtca tgggctccaa gatgatgaac     1500
   ggcatcggcg gcagcggcga ctttgcccgc aacgcatacc tcaccatatt cactacggag 1560
   tccatcgcga agaagggcga catttcctct atcgttccta tggtttccca cgtggaccac 1620
   accgagcatg acgtaatggt catcgttacc gaacaggggg ttgcggatct ccgcggtctt 1680
   tcccctcggg aaaaggccgt ggcgataatt gagaattgcg tccacccgga ttacaaggat 1740
   atgctcatgg agtacttcga ggaggcttgt aagtcctcag gtggcaacac cccacacaac 1800
   cttgaaaaag ccctatcctg gcacactaag ttcataaaaa ctggctcgat gaagtaatta 1860
   gaggagacac aacatggaga ttaaagagat ggtcagtctt gcgcgcaaag ctcagaagga 1920
   gtatcaggcc acccataacc aagaagctgt ggacaacatc tgccgagctg cagcgaaggt 1980
   tatttacgaa aatgcagcaa ttctggcccg cgaggcagtg gacgaaaccg gcatgggtgt 2040
   ttacgagcac aaggtggcca agaatcaagg caagtccaaa ggtgtttggt acaacctgca 2100
   taacaagaag tcgattggca tcctcaatat cgatgagcgt accggcatga tcgagatcgc 2160
   aaaacctatc ggggttgtag gcgccgttac gccaaccacc aaccctatcg ttactccgat 2220
   gagcaacatc atctttgctc ttaagacctg caacgccatc attatcgccc cacacccgcg 2280
   ctccaaaaag tgctctgccc acgcagttcg gctgatcaaa gaggctatcg ctccgttcaa 2340
   cgtgcccgaa ggtatggttc agatcatcga ggagcctagc atcgagaaga cgcaggaatt 2400
   gatgggcgcc gtagacgtgg tcgttgctac cgggggcatg ggcatggtca agtctgccta 2460
   ctcctcaggg aagccttctt tcggtgtcgg agccggcaat gttcaggtga tagtggacag 2520
   caacatcgat ttcgaagcgg ctgcagaaaa gatcatcacc ggacgtgcct tcgacaacgg 2580
   tatcatctgc tcaggcgaac agtccatcat ctacaacgag gctgacaagg aagcagtttt 2640
   cacagcattc cgcaaccacg gtgcgtactt ttgcgacgag gccgagggag atcgggctcg 2700
   tgcagcgatc ttcgaaaatg gagccatcgc gaaagatgtt gtgggccagt ccgttgcctt 2760
   tattgccaag aaggcgaaca ttaatatccc cgagggtact cgtattctcg tggtcgaagc 2820
   tcgcggagta ggcgccgaag atgtcatctg taaagaaaag atgtgtccag tcatgtgcgc 2880
   cctctcctac aagcacttcg aagagggggt agagatcgca aggacgaacc tcgcaaacga 2940
   aggcaatggc catacctgtg ctatccactc caacaaccaa gcacacatca tcttggcagg 3000
   ctcggagctg accgtgtctc gcatcgtggt caacgcgcca agtgctacca cagcaggcgg 3060
   tcacatccag aacggtcttg ccgtcaccaa tactctaggc tgcggctctt ggggtaacaa 3120
   ctcgatctcc gaaaacttca cttataaaca cctgctcaac atttcacgca tcgccccgtt 3180
   gaactccagc attcatatcc cagatgataa ggaaatctgg gaactctaat tagaggagac 3240
   acaacatgca gcttttcaag ctcaagagcg tcacacatca ctttgatact tttgcagagt 3300
   ttgccaagga attctgtctc ggtgaacgcg acttggtaat taccaacgag ttcatctacg 3360
   aaccgtatat gaaggcatgc cagctgcctt gtcattttgt gatgcaggag aaatacggcc 3420
   aaggcgagcc ttctgacgag atgatgaaca acatcctagc agatatccgt aatatccagt 3480
   tcgaccgcgt gatcgggatc ggaggtggta cggttattga catctcaaaa ctctttgttc 3540
   tgaagggatt aaatgatgtt ctcgacgcgt tcgatcgcaa gattcccctt atcaaagaga 3600
   aagaactgat cattgtgccc accacctgcg gaaccggctc ggaggtgacg aacatttcca 3660
   tcgccgagat caagtcccgg cacaccaaga tgggtttggc tgacgatgca attgttgctg 3720
   accacgccat aatcatccct gaacttctga agagcttgcc cttccacttc tatgcatgct 3780
   ccgcaatcga tgctcttatt catgccatcg agtcatacgt ttctccaaaa gcgtctccat 3840
   actcccgtct gttcagtgag gcggcgtggg acattatcct ggaagttttc aagaaaatcg 3900
   ccgaacacgg cccagagtac cgcttcgaga agctggggga aatgatcatg gccagcaact 3960
   atgccggtat cgctttcggc aacgcaggcg ttggcgccgt ccacgctcta tcctacccgt 4020
   tgggcggcaa ctatcacgtg ccgcatggag aagcaaacta tcagttcttc accgaggtct 4080
   ttaaagtata ccaaaagaag aatccgttcg gctatattgt cgaactcaac tggaagctct 4140
   ccaagattct gaactgccag ccagagtacg tgtacccgaa gctggatgaa ctgctcggtt 4200
   gccttcttac caagaaacct ttgcacgaat acggcatgaa ggacgaagag gttcgtggct 4260
   tcgcggaatc ggtcctgaag acccagcaac gcttgctcgc caacaactac gtcgaactta 4320
   ctgtcgatga gatcgaaggt atctaccgac gtctctacta attagaggag acacaacatg 4380
   aaggatgtac tggcggaata cgcctcccgc attgtttcgg cggaggaggc cgttaagcac 4440
   atcaaaaacg gtgaacgggt agctttgtca cacgctgccg gcgtgcctca gagttgcgtt 4500
   gacgcactgg tgcagcaggc cgaccttttc cagaatgtgg aaatctatca catgctgtgc 4560
   ctcggtgagg gtaagtatat ggcgcctgag atggcccctc acttccgcca catcaccaac 4620
   tttgtcggtg gtaactcccg taaggcggtc gaagaaaacc gggccgattt cattccggta 4680
   ttcttttacg aggtgccaag catgattcgc aaagacatcc tccacattga tgtcgccatc 4740
   gttcagcttt caatgcctga cgaaaatggt tactgttcct ttggagtatc ttgcgattac 4800
   tccaagccgg cagcagagag cgctcacctg gttatcggag aaatcaaccg tcaaatgcca 4860
   tacgtacacg gcgacaactt gattcatatc tccaagttgg attacatcgt gatggcagac 4920
   taccccatct actctcttgc aaagcccaag atcggggaag tcgaggaagc tatcgggagg 4980
   aattgtgccg agcttattga agatggtgcc actctccagc tgggaatcgg cgcgattcct 5040
   gatgcggccc tgttatttct caaggacaaa aaggatctgg gcatccatac cgaaatgttc 5100
   tccgatggtg ttgtcgaatt ggttcgctcc ggcgttatca caggcaagaa aaagactctt 5160
   caccccggaa agatggtcgc aaccttcctg atgggaagcg aggacgtgta tcatttcatc 5220
   gataaaaacc ccgatgtaga actgtatcca gtagattacg tgaatgaccc gcgtgtgatc 5280
   gcccaaaacg acaatatggt ctcgattaac agctgcatcg aaatcgacct tatgggacag 5340
   gtcgtgtccg agtgcatcgg ctcaaagcaa ttcagcggca ccggcggcca agttgactac 5400
   gtgcgtggcg cagcatggtc taaaaacggc aaatcgatca tggcaatccc gtccactgca 5460
   aaaaacggta cggcatctcg aattgtacct atcatcgcgg agggcgctgc tgtcaccacc 5520
   ctgcgcaacg aggtcgatta cgttgtaacc gagtacggta tcgctcagct caagggcaag 5580
   agcctgcgcc agcgcgcaga ggctttgatc gcgatagccc accccgactt ccgtgaggaa 5640
   ctaacgaaac atctccgcaa gcgattcgga taagcggccg cagatctgga tcc 5693
<210> 45
   <211> 44
   <212> DNA
   <213> Artificial Sequence
<220>
   <223> 3G up
<400> 45
   gatatcgaat tcctgcagat gattttgcat ctgctgcgaa atct 44
<210> 46
   <211> 47
   <212> DNA
   <213> Artificial Sequence
<220>
   <223> 3G down
<400> 46
   gcggtggcgg ccgctctaga ttagtagaga cgtcggtaga taccttc 47
<210> 47
   <211> 5567
   <212> DNA
   <213> Artificial Sequence
<220>
   <223> pGSX1
<400> 47
   ttgagatcct ttttttctgc gcgtaatctg ctgcttgcaa acaaaaaaac caccgctacc        60
   agcggtggtt tgtttgccgg atcaagagct accaactctt tttccgaagg taactggctt       120
   cagcagagcg cagataccaa atactgttct tctagtgtag ccgtagttag gccaccactt       180
   caagaactct gtagcaccgc ctacatacct cgctctgcta atcctgttac cagtggctgc       240
   tgccagtggc gataagtcgt gtcttaccgg gttggactca agacgatagt taccggataa       300
   ggcgcagcgg tcgggctgaa cggggggttc gtgcacacag cccagcttgg agcgaacgac       360
   ctacaccgaa ctgagatacc tacagcgtga gctatgagaa agcgccacgc ttcccgaagg       420
   gagaaaggcg gacaggtatc cggtaagcgg cagggtcgga acaggagagc gcacgaggga       480
   gcttccaggg ggaaacgcct ggtatcttta tagtcctgtc gggtttcgcc acctctgact 540
   tgagcgtcga tttttgtgat gctcgtcagg ggggcggagc ctatggaaaa acgcccctct 600
   tcgctattac gccagctggc gaaaggggga tgtgctgcaa ggcgattaag ttgggtaacg 660
   ccagggtttt cccagtcacg acgttgtaaa acgacggcca gtgaattgta atacgactca 720
   ctatagggcg aattgggtac catgattttg catctgctgc gaaatctttg tttccccgct 780
   aaagttgagg acaggttgac acggagttga ctcgacgaat tatccaatgt gagtaggttt 840
   ggtgcgtgag ttggaaaaat tcgccatact cgcccttggg ttctgtcagc tcaagaattc 900
   ttgagtgacc gatgctctga ttgacctaac tgcttgacac attgcatttc ctacaatctt 960
   tagaggagac acaacctcga ggtcgacggt atcgataagc ttgatatcga attcctgcag 1020
   cccgggggat ccactagttc tagagcggcc gccaccgcgg tggagctcat ttagcggatg 1080
   attctcgttc aacttcggcc gaagccactt cgtctgtcat aatgacaggg atggtttcgg 1140
   ccgtttttgc aaataaaacg aaaggctcag tcgaaagact gggcctttcg ttttatctgt 1200
   tgtttgtcgg tgaacgctct cctgagtagg acaaatccgc cgggagcgga tttgaacgtt 1260
   gcgaagcaac ggcccggagg gtggcgggca ggacgcccgc cataaactgc caggcatcaa 1320
   attaagcaga aggccatcct gacggatggc ctttttgcgt ttctacaaac tcttcctgtc 1380
   gtcatatcta caagccatcc ccccacagat acggtagctc cagcttttgt tccctttagt 1440
   gagggttaat ttcgagcttg gcgtaatcat ggtcatagct gtttcctgtg tgaaattgtt 1500
   atccgctcac aattccacac aacatacgag ccggaagcat aaagtgtaaa gcctgggaac 1560
   aacaagaccc atcatagttt gcccccgcga cattgaccat aaattcatcg cacaaaatat 1620
   cgaacggggt ttatgccgct tttagtgggt gcgaagaata gtctgctcat tacccgcgaa 1680
   caccgccgca ttcagatcac gcttagtagc gtccccatga gtaggcagaa ccgcgtccaa 1740
   gtccacatca tccataacga tcatgcacgg ggtggaatcc acacccagac ttgccagcac 1800
   ctcattagcg acacgttgcg cagcggccac gtccttagcc ttatccacgc aatctaggac 1860
   gtactgccta accgcgaaat cagactgaat cagtttccaa tcatcgggct tcaccaaagc 1920
   aacagcaacg cgggttgatt cgacccgttc cggtgcttcc agaccggcga gcttgtacag 1980
   ttcttcttcc atttcacgac gtacatcagc gtctatgtaa tcaatgccca aagcacgctt 2040
   agccccacgt gaccaggacg aacgcaggtt tttagaacca acctcatact cacgccaccg 2100
   agccaccaaa acagcgtcca tatcctcgcc ggcgtcgctt tgatcggcca acatatccaa 2160
   catctgaaac ggcgtgtacg accccttaga cgcggtttta gtagcggagc cagtcagttc 2220
   ctgagacatg cccttagcga ggtaggttgc cattttcgca gcgtctccac cccaggtaga 2280
   cacctgatca agtttgaccc cgtgctcacg cagtggcgcg tccataccgg ccttaaccac 2340
   accagcagac cagcgggaaa acatggaatc ctcaaacgcc ttgagttcat cgtcagacag 2400
   tggacgatcc aagaacaaca gcatgttgcg gtgcaagtgc caaccgttcg cccaagagtc 2460
   tgtgacctca tagtcactat aggtgtgctc caccccgtac cgtgcacgtt ctttcttcca 2520
   ctgagatgtt ttcaccatcg aagagtacgc agtcttaata cccgcttcaa cctgcgcaaa 2580
   tgactgtgag cggttgtgtc gaacagtgcc cacaaacatc atgagcgcgc cacccgccgc 2640
   caagtgattc ttagtagcaa tagccagctc aatgcggcgt tcgcccatga cttccaattc 2700
   agccagaggt gacccccagc gagagtgaga gttttgcaga ccctcaaact gcgaagcacc 2760
   gttagacgac caggacaccg caacagcttc gtccctgcgc cacctatggc accccgccag 2820
   agccttacta ttggtgatct tgtacatgac gttttgccta cgccacgccc tagcgcgagt 2880
   gaccttagaa ccctcattga cctgcggttc cttagaggtg ttcacttcta tttcagtgtt 2940
   acctagaccc gatgttgtgc ggggttgcgc agtgcgagtt tgtgcgggtg ttgtgcccgt 3000
   tgtcttagct agtgctatgg ttgtcaattg aaaccccttc gggttatgtg gcccccgtgc 3060
   atatgagttg gtagctcgca cgggggtttg tcttgtctag gaactattaa tttttagtgg 3120
   tgtttggtgg ccgcctagct tggctatgcg tgccagctta cccgtactca atgttaaaga 3180
   tttgcatcga catgggaggg ttacgtgtcc gatacctagg gggggtatcc gcgactaggt 3240
   gccccggtgc tcactgtctg taccggcggg gcaagcccca caccccgcat ggacagggtg 3300
   gctccgcccc ctgcaccccc agcaatctgc atgtacatgt tttacacatt agcacgacat 3360
   gactgcatgt gcatgcactg catgcagact aggtaaatat gagtatgtac gactagtaac 3420
   aggagcactg cacataatga atgagttgca ggacaatgtt tgctacgcat gcgcatgaca 3480
   tatcgcagga aagctactag agtcttaaag catggcaacc aaggcacagc tagaacagca 3540
   actacaagaa gctcaacagg cactacaggc gcagcaagcg caggcacaag ccaccatcga 3600
   agcactagaa gcgcaggcaa aggctaagcc cgtcgtggtc accgcacgcg ttcctttggc 3660
   actacgtgag gacatgaagc gcgcaggcat gcagaacggt gaaaacctcc aagagttcat 3720
   gatcgccgcg tttaccgagc ggctagaaaa gctcaccacc accgacaacg aggaaaacaa 3780
   tgtctaaccc actagttctc tttgcccacc gtgacccggt aaatgacgtg acgttcgagt 3840
   gcattgagca cgccacctac gacacacttt cacacgctaa agaccagatc accgcccaaa 3900
   tgcaagccct agacgaagaa gccgccctac tgccctaatg ggtgtttcat gggtgtttcc 3960
   ctagtgtttc atggtgtttt cacctaagct agggaattgc gcgagaagtc tcgcaaaaat 4020
   cagcaacccc cggaaccaca cagttcacgg gggttcttct atgccagaaa tcagaaaggg 4080
   gaaccagtga acgaccccga atattggatc acagcgcagc aggtcgccgc ccgcgtagct 4140
   ctcaccccgg ccaccattaa aaagtgggca aacgagggaa aaatcaccgc atacaagatc 4200
   ggcaagtccg tccgattcaa agcatcagac gtagacaagc tagggggggg ggggcgctga 4260
   ggtctgcctc gtgaagaagg tgttgctgac tcataccagg cctgaatcgc cccatcatcc      4320
   agccagaaag tgagggagcc acggttgatg agagctttgt tgtaggtgga ccagttggtg      4380
   attttgaact tttgctttgc cacggaacgg tctgcgttgt cgggaagatg cgtgatctga      4440
   tccttcaact cagcaaaagt tcgatttatt caacaaagcc gccgtcccgt caagtcagcg      4500
   taatgctctg ccagtgttac aaccaattaa ccaattctga ttagaagaac tcgtcaagaa      4560
   ggcgatagaa ggcgatgcgc tgcgaatcgg gagcggcgat accgtaaagc acgaggaagc      4620
   ggtcagccca ttcgccgcca agctcttcag caatatcacg ggtagccaac gctatgtcct      4680
   gatagcggtc cgccacaccc agccggccac agtcgatgaa tccagaaaag cggccatttt      4740
   ccaccatgat attcggcaag caggcatcgc catgggtcac gacgagatcc tcgccgtcgg      4800
   gcatgctcgc cttgagcctg gcgaacagtt cggctggcgc gagcccctga tgctcttcgt      4860
   ccagatcatc ctgatcgaca agaccggctt ccatccgagt acgtgctcgc tcgatgcgat      4920
   gtttcgcttg gtggtcgaat gggcaggtag ccggatcaag cgtatgcagc cgccgcattg      4980
   catcagccat gatggatact ttctcggcag gagcaaggtg agatgacagg agatcctgcc      5040
   ccggcacttc gcccaatagc agccagtccc ttcccgcttc agtgacaacg tcgagcacag      5100
   ctgcgcaagg aacgcccgtc gtggccagcc acgatagccg cgctgcctcg tcttgcagtt      5160
   cattcagggc accggacagg tcggtcttga caaaaagaac cgggcgcccc tgcgctgaca      5220
   gccggaacac ggcggcatca gagcagccga ttgtctgttg tgcccagtca tagccgaata      5280
   gcctctccac ccaagcggcc ggagaacctg cgtgcaatcc atcttgttca atcataacac      5340
   cccttgtatt actgtttatg taagcagaca gttttattgt tcatgatgat atatttttat      5400
   cttgtgcaat gtaacatcag agattttgag acacaacgtg gctttccccc cccccccaaa      5460
   aggatctagg tgaagatcct ttttgataat ctcatgacca aaatccctta acgtgagttt      5520
   tcgttccact gagcgtcaga ccccgtagaa aagatcaaag gatcttc                    5567
<210> 48
   <211> 48
   <212> DNA
   <213> Artificial Sequence
<220>
   <223> MD-625
<400> 48
   tagggcgaat tgggtaccat gattttgcat ctgctgcgaa atctttgt 48
<210> 49
   <211> 55
   <212> DNA
   <213> Artificial Sequence
<220>
   <223> MD-626
<400> 49
   gataccgtcg acctcgaggt tgtgtctcct ctaaagattg taggaaatgc aatgt 55
<210> 50
   <211> 48
   <212> DNA
   <213> Artificial Sequence
<220>
   <223> ncgl0049_up F
<400> 50
   catgattacg ccaagcttca atgcctttga tttgaactaa gcacagga 48
<210> 51
   <211> 43
   <212> DNA
   <213> Artificial Sequence
<220>
   <223> ncgl0049_up R
<400> 51
   tgcaaaatca ttagagctca aaaccgcggc gtaaaatatt aga 43
<210> 52
   <211> 31
   <212> DNA
   <213> Artificial Sequence
<220>
   <223> ncgl0049_down F
<400> 52
   tacggtgcta gcatctgccc ctttacaaat c 31
<210> 53
   <211> 48
   <212> DNA
   <213> Artificial Sequence
<220>
   <223> ncgl0049_down R
<400> 53
   acgacggcca gtgaattcgg atcagcccta aatacaacta tgagacag 48
<210> 54
   <211> 42
   <212> DNA
   <213> Artificial Sequence
<220>
   <223> G3G F
<400> 54
   tgagctctaa tgattttgca tctgctgcga aatctttgtt tc 42
<210> 55
   <211> 32
   <212> DNA
   <213> Artificial Sequence
<220>
   <223> G3G R
<400> 55
   cagatgctag caccgtatct gtggggggat gg 32
<210> 56
   <211> 24
   <212> DNA
   <213> Artificial Sequence
<220>
   <223> cat1-seq1F
<400> 56
   agcccttact aagaaggtta atgc 24
<210> 57
   <211> 22
   <212> DNA
   <213> Artificial Sequence
<220>
   <223> cat1-seq2R
<400> 57
   cgtacttgat tgaaccatcg tt 22
<210> 58
   <211> 22
   <212> DNA
   <213> Artificial Sequence
<220>
   <223> sucD-seq1F
<400> 58
   caatgttcag gtgatagtgg ac 22
<210> 59
   <211> 20
   <212> DNA
   <213> Artificial Sequence
<220>
   <223> sucD-seq2R
<400> 59
   cacaacatct ttcgcgatgg 20
<210> 60
   <211> 21
   <212> DNA
   <213> Artificial Sequence
<220>
   <223> 4hbD-seq1F
<400> 60
   ctgaccacgc cataatcatc c 21
<210> 61
   <211> 20
   <212> DNA
   <213> Artificial Sequence
<220>
   <223> 4hbD-seq2R
<400> 61
   cgataccggc atagttgctg 20
<210> 62
   <211> 37
   <212> DNA
   <213> Artificial Sequence
<220>
   <223> cat1 up
<400> 62
   ttgatatcga attccatgtc taaaggaatc aagaata 37
<210> 63
   <211> 35
   <212> DNA
   <213> Artificial Sequence
<220>
   <223> cat1 down
<400> 63
   tggcggccgc tctagttact tcatcgagcc agttt 35
<210> 64
   <211> 35
   <212> DNA
   <213> Artificial Sequence
<220>
   <223> sucD-up-IF
<400> 64
   tggcggccgc tctagttact tcatcgagcc agttt 35
<210> 65
   <211> 35
   <212> DNA
   <213> Artificial Sequence
<220>
   <223> sucD-down-IF
<400> 65
   tggcggccgc tctagttaga gttcccagat ttcct 35
<210> 66
   <211> 35
   <212> DNA
   <213> Artificial Sequence
<220>
   <223> 4hbD-up-IF
<400> 66
   ttgatatcga attccatgca gcttttcaag ctcaa 35
<210> 67
   <211> 34
   <212> DNA
   <213> Artificial Sequence
<220>
   <223> 4hbD-down-IF
<400> 67
   tggcggccgc tctagctagt agagacgtcg gtag 34
<210> 68
   <211> 9334
   <212> DNA
   <213> Artificial Sequence
<220>
   <223> pC2E2
<400> 68
   ttgagatcct ttttttctgc gcgtaatctg ctgcttgcaa acaaaaaaac caccgctacc      60
   agcggtggtt tgtttgccgg atcaagagct accaactctt tttccgaagg taactggctt     120
   cagcagagcg cagataccaa atactgttct tctagtgtag ccgtagttag gccaccactt     180
   caagaactct gtagcaccgc ctacatacct cgctctgcta atcctgttac cagtggctgc     240
   tgccagtggc gataagtcgt gtcttaccgg gttggactca agacgatagt taccggataa     300
   ggcgcagcgg tcgggctgaa cggggggttc gtgcacacag cccagcttgg agcgaacgac     360
   ctacaccgaa ctgagatacc tacagcgtga gctatgagaa agcgccacgc ttcccgaagg     420
   gagaaaggcg gacaggtatc cggtaagcgg cagggtcgga acaggagagc gcacgaggga     480
   gcttccaggg ggaaacgcct ggtatcttta tagtcctgtc gggtttcgcc acctctgact     540
   tgagcgtcga tttttgtgat gctcgtcagg ggggcggagc ctatggaaaa acgcccctct     600
   tcgctattac gccagctggc gaaaggggga tgtgctgcaa ggcgattaag ttgggtaacg 660
   ccagggtttt cccagtcacg acgttgtaaa acgacggcca gtgaattgta atacgactca 720
   ctatagggcg aattggtgcg ttaataaagg tggagaataa gttgtttcca agatcaattc 780
   aaggaaagtt gcattttcgc aggtcagtgt taccccctaa gactacccct ttccattgca 840
   tacaaaggaa atacatatag acttttgggc attagattac ctcgataaaa gtttagggaa 900
   tctaaactcg agaggagaca caacatgaag gatgtactgg cggaatacgc ctcccgcatt 960
   gtttcggcgg aggaggccgt taagcacatc aaaaacggtg aacgggtagc tttgtcacac 1020
   gctgccggcg tgcctcagag ttgcgttgac gcactggtgc agcaggccga ccttttccag 1080
   aatgtggaaa tctatcacat gctgtgcctc ggtgagggta agtatatggc gcctgagatg 1140
   gcccctcact tccgccacat caccaacttt gtcggtggta actcccgtaa ggcggtcgaa 1200
   gaaaaccggg ccgatttcat tccggtattc ttttacgagg tgccaagcat gattcgcaaa 1260
   gacatcctcc acattgatgt cgccatcgtt cagctttcaa tgcctgacga aaatggttac 1320
   tgttcctttg gagtatcttg cgattactcc aagccggcag cagagagcgc tcacctggtt 1380
   atcggagaaa tcaaccgtca aatgccatac gtacacggcg acaacttgat tcatatctcc 1440
   aagttggatt acatcgtgat ggcagactac cccatctact ctcttgcaaa gcccaagatc 1500
   ggggaagtcg aggaagctat cgggaggaat tgtgccgagc ttattgaaga tggtgccact 1560
   ctccagctgg gaatcggcgc gattcctgat gcggccctgt tatttctcaa ggacaaaaag 1620
   gatctgggca tccataccga aatgttctcc gatggtgttg tcgaattggt tcgctccggc 1680
   gttatcacag gcaagaaaaa gactcttcac cccggaaaga tggtcgcaac cttcctgatg 1740
   ggaagcgagg acgtgtatca tttcatcgac aaaaaccccg atgtagaact gtatccagta 1800
   gattacgtga atgacccgcg tgtgatcgcc caaaacgaca atatggtctc gattaacagc 1860
   tgcatcgaaa tcgaccttat gggacaggtc gtgtccgagt gcatcggctc aaagcaattc 1920
   agcggcaccg gcggccaagt tgactacgtg cgtggcgcag catggtctaa aaacggcaag 1980
   tccatcatgg caatcccgtc cactgcaaaa aacggtacgg catctcgaat tgtacctatc 2040
   atcgcggagg gcgctgctgt caccaccctg cgcaacgagg tcgattacgt tgtaaccgag 2100
   tacggtatcg ctcagctcaa gggcaagagc ctgcgccagc gcgcagaggc tttgatcgcg 2160
   atagcccacc ccgacttccg tgaggaacta acgaaacatc tccgcaagcg attcggagtc 2220
   tagatttcct acaatcttta gaggagacac aacatgaaag taaccaatca gaaagagttg 2280
   aagcagaagt tgaacgagct gcgagaggct cagaagaagt tcgcaaccta cacccaggaa 2340
   caggtggaca agatctttaa gcagtgtgcc attgcagccg cgaaagaacg tattaatctc 2400
   gcgaaacttg cggtcgagga aaccggtatt gggctggtag aagacaagat catcaagaac 2460
   cacttcgccg ctgaatacat ctacaacaag tacaaaaacg aaaagacatg tggtatcatc 2520
   gaccacgacg acagcttggg catcaccaag gtagcggagc caatcggtat cgtcgcagct 2580
   atcgtgccca ctactaaccc tacctccact gctattttca agtcactcat ctccctgaaa 2640
   acccgcaatg ctatcttctt ctcacctcac ccacgcgcta agaaatcaac tatcgcagca 2700
   gctaaactta tcctggatgc agccgtgaaa gccggggctc cgaaaaacat catcggttgg 2760
   atcgacgaac cttccattga actctctcaa gacctcatgt ccgaggcaga cattatcctg 2820
   gcaaccggag gcccatccat ggttaaagca gcttacagct caggcaagcc ggctatcggc 2880
   gttggagctg gtaacactcc agcaatcatc gacgagtcgg ccgatatcga catggcagtg 2940
   tcctctatta tcctgtccaa aacttatgac aacggcgtta tttgcgcgtc cgagcagtct 3000
   attctcgtca tgaactctat ttacgagaag gtaaaggagg agtttgtgaa gcgggggtcg 3060
   tacattctga accagaacga gatcgctaag atcaaagaga ctatgtttaa aaacggagcc 3120
   atcaacgcag atatcgtagg gaagtccgcg tacatcattg ctaagatggc tggaatcgaa 3180
   gtccctcaaa ccacgaaaat tctgatcggc gaggtgcaat cggtcgaaaa gtccgagctg 3240
   ttctcgcatg aaaagttgtc cccggtcctc gcgatgtata aagttaagga ttttgatgaa 3300
   gcactcaaga aagctcagcg cctgatcgaa ttgggtggct cgggtcacac ctcttccctc 3360
   tacattgact cccagaacaa taaagataag gtgaaagagt tcggcctggc tatgaagacg 3420
   tctcgtacct tcatcaatat gccctcttca cagggcgcca gcggtgacct ttacaatttc 3480
   gctatcgctc ctagctttac cctcggctgc ggcacctggg gcggtaattc tgtgtcccaa 3540
   aacgtcgaac caaagcatct gctcaacatt aaaagcgtcg ccgaacgtcg cgagaacatg 3600
   ttgtggttca aggtcccgca aaaaatctac ttcaagtatg gttgcttgcg ctttgcactt 3660
   aaagagctta aggacatgaa taaaaagcgg gcgttcatcg tcactgataa ggatctgttc 3720
   aaactgggct atgttaacaa gattaccaag gtcctggatg agatcgacat caagtattcc 3780
   atcttcaccg atattaagtc cgatccgacc attgattccg tgaagaaggg cgcgaaggag 3840
   atgctcaact ttgaacccga cacgattatt tctattggcg gaggcagccc aatggacgca 3900
   gctaaggtta tgcacctgct gtatgagtac ccagaagcag agatcgagaa ccttgcaatc 3960
   aatttcatgg atattcgcaa acgcatttgc aactttccta agctcggtac aaaagctatc 4020
   tctgttgcga tccctaccac cgcaggaacc ggcagcgaag cgacaccatt cgccgttatt 4080
   accaacgatg aaacaggtat gaagtaccca cttacctctt atgaacttac cccgaacatg 4140
   gctatcattg atacggaatt gatgctgaac atgccacgca agttgaccgc agctacggga 4200
   atcgacgcat tggttcatgc aatcgaggca tacgtttccg tcatggcaac cgattacacc 4260
   gacgaactcg cgttgcgtgc gattaaaatg atcttcaagt accttccacg cgcatacaag 4320
   aatggcacaa acgatattga agcccgagaa aagatggcac acgcttcgaa catcgctggt 4380
   atggccttcg cgaatgcgtt tctcggagtg tgtcactcca tggcgcacaa actgggagcc 4440
   atgcatcacg tgccccacgg tatcgcatgc gccgttctta ttgaagaggt gatcaagtat 4500
   aatgccaccg attgccccac taagcagacg gccttccctc agtacaaatc gcccaatgcc 4560
   aagcgtaaat acgcggaaat tgccgagtac ttgaacctta aggggaccag cgacacggaa 4620
   aaggtgaccg cactgattga agccatctcc aagctcaaga tcgacctgag catcccacaa 4680
   aacatctcag cagccggcat taacaagaag gacttctaca acactctcga caagatgtca 4740
   gaactcgcct tcgatgatca gtgcactacc gcaaacccac gttatccgct catctctgaa 4800
   ctgaaggata tctacatcaa gtcgttttag aattcattta gcggatgatt ctcgttcaac 4860
   ttcggccgaa gccacttcgt ctgtcataat gacagggatg gtttcggccg tttttgcaaa 4920
   taaaacgaaa ggctcagtcg aaagactggg cctttcgttt tatctgttgt ttgtcggtga 4980
   acgctctcct gagtaggaca aatccgccgg gagcggattt gaacgttgcg aagcaacggc 5040
   ccggagggtg gcgggcagga cgcccgccat aaactgccag gcatcaaatt aagcagaagg 5100
   ccatcctgac ggatggcctt tttgcgtttc tacaaactct tcctgtcgtc atatctacaa 5160
   gccatccccc cacagatacg gtagctccag cttttgttcc ctttagtgag ggttaatttc 5220
   gagcttggcg taatcatggt catagctgtt tcctgtgtga aattgttatc cgctcacaat 5280
   tccacacaac atacgagccg gaagcataaa gtgtaaagcc tgggaacaac aagacccatc 5340
   atagtttgcc cccgcgacat tgaccataaa ttcatcgcac aaaatatcga acggggttta 5400
   tgccgctttt agtgggtgcg aagaatagtc tgctcattac ccgcgaacac cgccgcattc 5460
   agatcacgct tagtagcgtc cccatgagta ggcagaaccg cgtccaagtc cacatcatcc 5520
   ataacgatca tgcacggggt ggaatccaca cccagacttg ccagcacctc attagcgaca 5580
   cgttgcgcag cggccacgtc cttagcctta tccacgcaat ctaggacgta ctgcctaacc 5640
   gcgaaatcag actgaatcag tttccaatca tcgggcttca ccaaagcaac agcaacgcgg 5700
   gttgattcga cccgttccgg tgcttccaga ccggcgagct tgtacagttc ttcttccatt 5760
   tcacgacgta catcagcgtc tatgtaatca atgcccaaag cacgcttagc cccacgtgac 5820
   caggacgaac gcaggttttt agaaccaacc tcatactcac gccaccgagc caccaaaaca 5880
   gcgtccatat cctcgccggc gtcgctttga tcggccaaca tatccaacat ctgaaacggc 5940
   gtgtacgacc ccttagacgc ggttttagta gcggagccag tcagttcctg agacatgccc 6000
   ttagcgaggt aggttgccat tttcgcagcg tctccacccc aggtagacac ctgatcaagt 6060
   ttgaccccgt gctcacgcag tggcgcgtcc ataccggcct taaccacacc agcagaccag 6120
   cgggaaaaca tggaatcctc aaacgccttg agttcatcgt cagacagtgg acgatccaag 6180
   aacaacagca tgttgcggtg caagtgccaa ccgttcgccc aagagtctgt gacctcatag 6240
   tcactatagg tgtgctccac cccgtaccgt gcacgttctt tcttccactg agatgttttc 6300
   accatcgaag agtacgcagt cttaataccc gcttcaacct gcgcaaatga ctgtgagcgg 6360
   ttgtgtcgaa cagtgcccac aaacatcatg agcgcgccac ccgccgccaa gtgattctta 6420
   gtagcaatag ccagctcaat gcggcgttcg cccatgactt ccaattcagc cagaggtgac 6480
   ccccagcgag agtgagagtt ttgcagaccc tcaaactgcg aagcaccgtt agacgaccag 6540
   gacaccgcaa cagcttcgtc cctgcgccac ctatggcacc ccgccagagc cttactattg 6600
   gtgatcttgt acatgacgtt ttgcctacgc cacgccctag cgcgagtgac cttagaaccc 6660
   tcattgacct gcggttcctt agaggtgttc acttctattt cagtgttacc tagacccgat 6720
   gttgtgcggg gttgcgcagt gcgagtttgt gcgggtgttg tgcccgttgt cttagctagt 6780
   gctatggttg tcaattgaaa ccccttcggg ttatgtggcc cccgtgcata tgagttggta 6840
   gctcgcacgg gggtttgtct tgtctaggaa ctattaattt ttagtggtgt ttggtggccg 6900
   cctagcttgg ctatgcgtgc cagcttaccc gtactcaatg ttaaagattt gcatcgacat 6960
   gggagggtta cgtgtccgat acctaggggg ggtatccgcg actaggtgcc ccggtgctca 7020
   ctgtctgtac cggcggggca agccccacac cccgcatgga cagggtggct ccgccccctg 7080
   cacccccagc aatctgcatg tacatgtttt acacattagc acgacatgac tgcatgtgca 7140
   tgcactgcat gcagactagg taaatatgag tatgtacgac tagtaacagg agcactgcac 7200
   ataatgaatg agttgcagga caatgtttgc tacgcatgcg catgacatat cgcaggaaag 7260
   ctactagagt cttaaagcat ggcaaccaag gcacagctag aacagcaact acaagaagct 7320
   caacaggcac tacaggcgca gcaagcgcag gcacaagcca ccatcgaagc actagaagcg 7380
   caggcaaagg ctaagcccgt cgtggtcacc gcacgcgttc ctttggcact acgtgaggac 7440
   atgaagcgcg caggcatgca gaacggtgaa aacctccaag agttcatgat cgccgcgttt 7500
   accgagcggc tagaaaagct caccaccacc gacaacgagg aaaacaatgt ctaacccact 7560
   agttctcttt gcccaccgtg acccggtaaa tgacgtgacg ttcgagtgca ttgagcacgc 7620
   cacctacgac acactttcac acgctaaaga ccagatcacc gcccaaatgc aagccctaga 7680
   cgaagaagcc gccctactgc cctaatgggt gtttcatggg tgtttcccta gtgtttcatg 7740
   gtgttttcac ctaagctagg gaattgcgcg agaagtctcg caaaaatcag caacccccgg 7800
   aaccacacag ttcacggggg ttcttctatg ccagaaatca gaaaggggaa ccagtgaacg 7860
   accccgaata ttggatcaca gcgcagcagg tcgccgcccg cgtagctctc accccggcca 7920
   ccattaaaaa gtgggcaaac gagggaaaaa tcaccgcata caagatcggc aagtccgtcc 7980
   gattcaaagc atcagacgta gacaagctag gggggggggg gcgctgaggt ctgcctcgtg 8040
   aagaaggtgt tgctgactca taccaggcct gaatcgcccc atcatccagc cagaaagtga 8100
   gggagccacg gttgatgaga gctttgttgt aggtggacca gttggtgatt ttgaactttt 8160
   gctttgccac ggaacggtct gcgttgtcgg gaagatgcgt gatctgatcc ttcaactcag 8220
   caaaagttcg atttattcaa caaagccgcc gtcccgtcaa gtcagcgtaa tgctctgcca 8280
   gtgttacaac caattaacca attctgatta gaagaactcg tcaagaaggc gatagaaggc 8340
   gatgcgctgc gaatcgggag cggcgatacc gtaaagcacg aggaagcggt cagcccattc 8400
   gccgccaagc tcttcagcaa tatcacgggt agccaacgct atgtcctgat agcggtccgc 8460
   cacacccagc cggccacagt cgatgaatcc agaaaagcgg ccattttcca ccatgatatt 8520
   cggcaagcag gcatcgccat gggtcacgac gagatcctcg ccgtcgggca tgctcgcctt 8580
   gagcctggcg aacagttcgg ctggcgcgag cccctgatgc tcttcgtcca gatcatcctg 8640
   atcgacaaga ccggcttcca tccgagtacg tgctcgctcg atgcgatgtt tcgcttggtg 8700
   gtcgaatggg caggtagccg gatcaagcgt atgcagccgc cgcattgcat cagccatgat 8760
   ggatactttc tcggcaggag caaggtgaga tgacaggaga tcctgccccg gcacttcgcc 8820
   caatagcagc cagtcccttc ccgcttcagt gacaacgtcg agcacagctg cgcaaggaac 8880
   gcccgtcgtg gccagccacg atagccgcgc tgcctcgtct tgcagttcat tcagggcacc 8940
   ggacaggtcg gtcttgacaa aaagaaccgg gcgcccctgc gctgacagcc ggaacacggc 9000
   ggcatcagag cagccgattg tctgttgtgc ccagtcatag ccgaatagcc tctccaccca 9060
   agcggccgga gaacctgcgt gcaatccatc ttgttcaatc ataacacccc ttgtattact 9120
   gtttatgtaa gcagacagtt ttattgttca tgatgatata tttttatctt gtgcaatgta 9180
   acatcagaga ttttgagaca caacgtggct ttcccccccc ccccaaaagg atctaggtga 9240
   agatcctttt tgataatctc atgaccaaaa tcccttaacg tgagttttcg ttccactgag 9300
   cgtcagaccc cgtagaaaag atcaaaggat cttc 9334
<210> 69
   <211> 50
   <212> DNA
   <213> Artificial Sequence
<220>
   <223> P1
<400> 69
   ctatagggcg aattggtgcg ttaataaagg tggagaataa gttgtttcca 50
<210> 70
   <211> 45
   <212> DNA
   <213> Artificial Sequence
<220>
   <223> P2
<400> 70
   gtctcctctc gagtttagat tccctaaact tttatcgagg taatc 45
<210> 71
   <211> 6773
   <212> DNA
   <213> Artificial Sequence
<220>
   <223> MD0294
<400> 71
   ttgagatcct ttttttctgc gcgtaatctg ctgcttgcaa acaaaaaaac caccgctacc      60
   agcggtggtt tgtttgccgg atcaagagct accaactctt tttccgaagg taactggctt    120
   cagcagagcg cagataccaa atactgttct tctagtgtag ccgtagttag gccaccactt    180
   caagaactct gtagcaccgc ctacatacct cgctctgcta atcctgttac cagtggctgc    240
   tgccagtggc gataagtcgt gtcttaccgg gttggactca agacgatagt taccggataa    300
   ggcgcagcgg tcgggctgaa cggggggttc gtgcacacag cccagcttgg agcgaacgac    360
   ctacaccgaa ctgagatacc tacagcgtga gctatgagaa agcgccacgc ttcccgaagg    420
   gagaaaggcg gacaggtatc cggtaagcgg cagggtcgga acaggagagc gcacgaggga    480
   gcttccaggg ggaaacgcct ggtatcttta tagtcctgtc gggtttcgcc acctctgact    540
   tgagcgtcga tttttgtgat gctcgtcagg ggggcggagc ctatggaaaa acgcccctct    600
   tcgctattac gccagctggc gaaaggggga tgtgctgcaa ggcgattaag ttgggtaacg    660
   ccagggtttt cccagtcacg acgttgtaaa acgacggcca gtgaattgta atacgactca    720
   ctatagggcg aattgggtac catgattttg catctgctgc gaaatctttg tttccccgct    780
   aaagttgagg acaggttgac acggagttga ctcgacgaat tatccaatgt gagtaggttt    840
   ggtgcgtgag ttggaaaaat tcgccatact cgcccttggg ttctgtcagc tcaagaattc    900
   ttgagtgacc gatgctctga ttgacctaac tgcttgacac attgcatttc ctacaatctt    960
   tagaggagac acaacatgaa ggatgtactg gcggaatacg cctcccgcat tgtttcggcg   1020
   gaggaggccg ttaagcacat caaaaacggt gaacgggtag ctttgtcaca cgctgccggc   1080
   gtgcctcaga gttgcgttga cgcactggtg cagcaggccg accttttcca gaatgtggaa   1140
   atctatcaca tgctgtgcct cggtgagggt aagtatatgg cgcctgagat ggcccctcac   1200
   ttccgccaca tcaccaactt tgtcggtggt aactcccgta aggcggtcga agaaaaccgg   1260
   gccgatttca ttccggtatt cttttacgag gtgccaagca tgattcgcaa agacatcctc   1320
   cacattgatg tcgccatcgt tcagctttca atgcctgacg aaaatggtta ctgttccttt   1380
   ggagtatctt gcgattactc caagccggca gcagagagcg ctcacctggt tatcggagaa   1440
   atcaaccgtc aaatgccata cgtacacggc gacaacttga ttcatatctc caagttggat   1500
   tacatcgtga tggcagacta ccccatctac tctcttgcaa agcccaagat cggggaagtc   1560
   gaggaagcta tcgggaggaa ttgtgccgag cttattgaag atggtgccac tctccagctg 1620
   ggaatcggcg cgattcctga tgcggccctg ttatttctca aggacaaaaa ggatctgggc 1680
   atccataccg aaatgttctc cgatggtgtt gtcgaattgg ttcgctccgg cgttatcaca 1740
   ggcaagaaaa agactcttca ccccggaaag atggtcgcaa ccttcctgat gggaagcgag 1800
   gacgtgtatc atttcatcga caaaaacccc gatgtagaac tgtatccagt agattacgtg 1860
   aatgacccgc gtgtgatcgc ccaaaacgac aatatggtct cgattaacag ctgcatcgaa 1920
   atcgacctta tgggacaggt cgtgtccgag tgcatcggct caaagcaatt cagcggcacc 1980
   ggcggccaag ttgactacgt gcgtggcgca gcatggtcta aaaacggcaa gtccatcatg 2040
   gcaatcccgt ccactgcaaa aaacggtacg gcatctcgaa ttgtacctat catcgcggag 2100
   ggcgctgctg tcaccaccct gcgcaacgag gtcgattacg ttgtaaccga gtacggtatc 2160
   gctcagctca agggcaagag cctgcgccag cgcgcagagg ctttgatcgc gatagcccac 2220
   cccgacttcc gtgaggaact aacgaaacat ctccgcaagc gattcggata gctcatttag 2280
   cggatgattc tcgttcaact tcggccgaag ccacttcgtc tgtcataatg acagggatgg 2340
   tttcggccgt ttttgcaaat aaaacgaaag gctcagtcga aagactgggc ctttcgtttt 2400
   atctgttgtt tgtcggtgaa cgctctcctg agtaggacaa atccgccggg agcggatttg 2460
   aacgttgcga agcaacggcc cggagggtgg cgggcaggac gcccgccata aactgccagg 2520
   catcaaatta agcagaaggc catcctgacg gatggccttt ttgcgtttct acaaactctt 2580
   cctgtcgtca tatctacaag ccatcccccc acagatacgg tagctccagc ttttgttccc 2640
   tttagtgagg gttaatttcg agcttggcgt aatcatggtc atagctgttt cctgtgtgaa 2700
   attgttatcc gctcacaatt ccacacaaca tacgagccgg aagcataaag tgtaaagcct 2760
   gggaacaaca agacccatca tagtttgccc ccgcgacatt gaccataaat tcatcgcaca 2820
   aaatatcgaa cggggtttat gccgctttta gtgggtgcga agaatagtct gctcattacc 2880
   cgcgaacacc gccgcattca gatcacgctt agtagcgtcc ccatgagtag gcagaaccgc 2940
   gtccaagtcc acatcatcca taacgatcat gcacggggtg gaatccacac ccagacttgc 3000
   cagcacctca ttagcgacac gttgcgcagc ggccacgtcc ttagccttat ccacgcaatc 3060
   taggacgtac tgcctaaccg cgaaatcaga ctgaatcagt ttccaatcat cgggcttcac 3120
   caaagcaaca gcaacgcggg ttgattcgac ccgttccggt gcttccagac cggcgagctt 3180
   gtacagttct tcttccattt cacgacgtac atcagcgtct atgtaatcaa tgcccaaagc 3240
   acgcttagcc ccacgtgacc aggacgaacg caggttttta gaaccaacct catactcacg 3300
   ccaccgagcc accaaaacag cgtccatatc ctcgccggcg tcgctttgat cggccaacat 3360
   atccaacatc tgaaacggcg tgtacgaccc cttagacgcg gttttagtag cggagccagt 3420
   cagttcctga gacatgccct tagcgaggta ggttgccatt ttcgcagcgt ctccacccca 3480
   ggtagacacc tgatcaagtt tgaccccgtg ctcacgcagt ggcgcgtcca taccggcctt 3540
   aaccacacca gcagaccagc gggaaaacat ggaatcctca aacgccttga gttcatcgtc 3600
   agacagtgga cgatccaaga acaacagcat gttgcggtgc aagtgccaac cgttcgccca 3660
   agagtctgtg acctcatagt cactataggt gtgctccacc ccgtaccgtg cacgttcttt 3720
   cttccactga gatgttttca ccatcgaaga gtacgcagtc ttaatacccg cttcaacctg 3780
   cgcaaatgac tgtgagcggt tgtgtcgaac agtgcccaca aacatcatga gcgcgccacc 3840
   cgccgccaag tgattcttag tagcaatagc cagctcaatg cggcgttcgc ccatgacttc 3900
   caattcagcc agaggtgacc cccagcgaga gtgagagttt tgcagaccct caaactgcga 3960
   agcaccgtta gacgaccagg acaccgcaac agcttcgtcc ctgcgccacc tatggcaccc 4020
   cgccagagcc ttactattgg tgatcttgta catgacgttt tgcctacgcc acgccctagc 4080
   gcgagtgacc ttagaaccct cattgacctg cggttcctta gaggtgttca cttctatttc 4140
   agtgttacct agacccgatg ttgtgcgggg ttgcgcagtg cgagtttgtg cgggtgttgt 4200
   gcccgttgtc ttagctagtg ctatggttgt caattgaaac cccttcgggt tatgtggccc 4260
   ccgtgcatat gagttggtag ctcgcacggg ggtttgtctt gtctaggaac tattaatttt 4320
   tagtggtgtt tggtggccgc ctagcttggc tatgcgtgcc agcttacccg tactcaatgt 4380
   taaagatttg catcgacatg ggagggttac gtgtccgata cctagggggg gtatccgcga 4440
   ctaggtgccc cggtgctcac tgtctgtacc ggcggggcaa gccccacacc ccgcatggac 4500
   agggtggctc cgccccctgc acccccagca atctgcatgt acatgtttta cacattagca 4560
   cgacatgact gcatgtgcat gcactgcatg cagactaggt aaatatgagt atgtacgact 4620
   agtaacagga gcactgcaca taatgaatga gttgcaggac aatgtttgct acgcatgcgc 4680
   atgacatatc gcaggaaagc tactagagtc ttaaagcatg gcaaccaagg cacagctaga 4740
   acagcaacta caagaagctc aacaggcact acaggcgcag caagcgcagg cacaagccac 4800
   catcgaagca ctagaagcgc aggcaaaggc taagcccgtc gtggtcaccg cacgcgttcc 4860
   tttggcacta cgtgaggaca tgaagcgcgc aggcatgcag aacggtgaaa acctccaaga 4920
   gttcatgatc gccgcgttta ccgagcggct agaaaagctc accaccaccg acaacgagga 4980
   aaacaatgtc taacccacta gttctctttg cccaccgtga cccggtaaat gacgtgacgt 5040
   tcgagtgcat tgagcacgcc acctacgaca cactttcaca cgctaaagac cagatcaccg 5100
   cccaaatgca agccctagac gaagaagccg ccctactgcc ctaatgggtg tttcatgggt 5160
   gtttccctag tgtttcatgg tgttttcacc taagctaggg aattgcgcga gaagtctcgc 5220
   aaaaatcagc aacccccgga accacacagt tcacgggggt tcttctatgc cagaaatcag 5280
   aaaggggaac cagtgaacga ccccgaatat tggatcacag cgcagcaggt cgccgcccgc 5340
   gtagctctca ccccggccac cattaaaaag tgggcaaacg agggaaaaat caccgcatac 5400
   aagatcggca agtccgtccg attcaaagca tcagacgtag acaagctagg gggggggggg 5460
   cgctgaggtc tgcctcgtga agaaggtgtt gctgactcat accaggcctg aatcgcccca 5520
   tcatccagcc agaaagtgag ggagccacgg ttgatgagag ctttgttgta ggtggaccag 5580
   ttggtgattt tgaacttttg ctttgccacg gaacggtctg cgttgtcggg aagatgcgtg 5640
   atctgatcct tcaactcagc aaaagttcga tttattcaac aaagccgccg tcccgtcaag 5700
   tcagcgtaat gctctgccag tgttacaacc aattaaccaa ttctgattag aagaactcgt 5760
   caagaaggcg atagaaggcg atgcgctgcg aatcgggagc ggcgataccg taaagcacga 5820
   ggaagcggtc agcccattcg ccgccaagct cttcagcaat atcacgggta gccaacgcta 5880
   tgtcctgata gcggtccgcc acacccagcc ggccacagtc gatgaatcca gaaaagcggc 5940
   cattttccac catgatattc ggcaagcagg catcgccatg ggtcacgacg agatcctcgc 6000
   cgtcgggcat gctcgccttg agcctggcga acagttcggc tggcgcgagc ccctgatgct 6060
   cttcgtccag atcatcctga tcgacaagac cggcttccat ccgagtacgt gctcgctcga 6120
   tgcgatgttt cgcttggtgg tcgaatgggc aggtagccgg atcaagcgta tgcagccgcc 6180
   gcattgcatc agccatgatg gatactttct cggcaggagc aaggtgagat gacaggagat 6240
   cctgccccgg cacttcgccc aatagcagcc agtcccttcc cgcttcagtg acaacgtcga 6300
   gcacagctgc gcaaggaacg cccgtcgtgg ccagccacga tagccgcgct gcctcgtctt 6360
   gcagttcatt cagggcaccg gacaggtcgg tcttgacaaa aagaaccggg cgcccctgcg 6420
   ctgacagccg gaacacggcg gcatcagagc agccgattgt ctgttgtgcc cagtcatagc 6480
   cgaatagcct ctccacccaa gcggccggag aacctgcgtg caatccatct tgttcaatca 6540
   taacacccct tgtattactg tttatgtaag cagacagttt tattgttcat gatgatatat 6600
   ttttatcttg tgcaatgtaa catcagagat tttgagacac aacgtggctt tccccccccc 6660
   cccaaaagga tctaggtgaa gatccttttt gataatctca tgaccaaaat cccttaacgt 6720
   gagttttcgt tccactgagc gtcagacccc gtagaaaaga tcaaaggatc ttc 6773
<210> 72
   <211> 37
   <212> DNA
   <213> Artificial Sequence
<220>
   <223> P3
<400> 72
   ctaaactcga gaggagacac aacatgaagg atgtact 37
<210> 73
   <211> 35
   <212> DNA
   <213> Artificial Sequence
<220>
   <223> P4
<400> 73
   aggaaatcta gactccgaat cgcttgcgga gatgt 35
<210> 74
   <211> 8054
   <212> DNA
   <213> Artificial Sequence
<220>
   <223> MD0295
<400> 74
   ttgagatcct ttttttctgc gcgtaatctg ctgcttgcaa acaaaaaaac caccgctacc 60
   agcggtggtt tgtttgccgg atcaagagct accaactctt tttccgaagg taactggctt 120
   cagcagagcg cagataccaa atactgttct tctagtgtag ccgtagttag gccaccactt 180
   caagaactct gtagcaccgc ctacatacct cgctctgcta atcctgttac cagtggctgc 240
   tgccagtggc gataagtcgt gtcttaccgg gttggactca agacgatagt taccggataa 300
   ggcgcagcgg tcgggctgaa cggggggttc gtgcacacag cccagcttgg agcgaacgac 360
   ctacaccgaa ctgagatacc tacagcgtga gctatgagaa agcgccacgc ttcccgaagg 420
   gagaaaggcg gacaggtatc cggtaagcgg cagggtcgga acaggagagc gcacgaggga 480
   gcttccaggg ggaaacgcct ggtatcttta tagtcctgtc gggtttcgcc acctctgact 540
   tgagcgtcga tttttgtgat gctcgtcagg ggggcggagc ctatggaaaa acgcccctct 600
   tcgctattac gccagctggc gaaaggggga tgtgctgcaa ggcgattaag ttgggtaacg 660
   ccagggtttt cccagtcacg acgttgtaaa acgacggcca gtgaattgta atacgactca 720
   ctatagggcg aattgggtac catgattttg catctgctgc gaaatctttg tttccccgct 780
   aaagttgagg acaggttgac acggagttga ctcgacgaat tatccaatgt gagtaggttt 840
   ggtgcgtgag ttggaaaaat tcgccatact cgcccttggg ttctgtcagc tcaagaattc 900
   ttgagtgacc gatgctctga ttgacctaac tgcttgacac attgcatttc ctacaatctt 960
   tagaggagac acaacatgaa agtaaccaat cagaaagagt tgaagcagaa gttgaacgag 1020
   ctgcgagagg ctcagaagaa gttcgcaacc tacacccagg aacaggtgga caagatcttt 1080
   aagcagtgtg ccattgcagc cgcgaaagaa cgtattaatc tcgcgaaact tgcggtcgag 1140
   gaaaccggta ttgggctggt agaagacaag atcatcaaga accacttcgc cgctgaatac 1200
   atctacaaca agtacaaaaa cgaaaagaca tgtggtatca tcgaccacga cgacagcttg 1260
   ggcatcacca aggtagcgga gccaatcggt atcgtcgcag ctatcgtgcc cactactaac 1320
   cctacctcca ctgctatttt caagtcactc atctccctga aaacccgcaa tgctatcttc 1380
   ttctcacctc acccacgcgc taagaaatca actatcgcag cagctaaact tatcctggat 1440
   gcagccgtga aagccggggc tccgaaaaac atcatcggtt ggatcgacga accttccatt 1500
   gaactctctc aagacctcat gtccgaggca gacattatcc tggcaaccgg aggcccatcc 1560
   atggttaaag cagcttacag ctcaggcaag ccggctatcg gcgttggagc tggtaacact 1620
   ccagcaatca tcgacgagtc ggccgatatc gacatggcag tgtcctctat tatcctgtcc 1680
   aaaacttatg acaacggcgt tatttgcgcg tccgagcagt ctattctcgt catgaactct 1740
   atttacgaga aggtaaagga ggagtttgtg aagcgggggt cgtacattct gaaccagaac 1800
   gagatcgcta agatcaaaga gactatgttt aaaaacggag ccatcaacgc agatatcgta 1860
   gggaagtccg cgtacatcat tgctaagatg gctggaatcg aagtccctca aaccacgaaa 1920
   attctgatcg gcgaggtgca atcggtcgaa aagtccgagc tgttctcgca tgaaaagttg 1980
   tccccggtcc tcgcgatgta taaagttaag gattttgatg aagcactcaa gaaagctcag 2040
   cgcctgatcg aattgggtgg ctcgggtcac acctcttccc tctacattga ctcccagaac 2100
   aataaagata aggtgaaaga gttcggcctg gctatgaaga cgtctcgtac cttcatcaat 2160
   atgccctctt cacagggcgc cagcggtgac ctttacaatt tcgctatcgc tcctagcttt 2220
   accctcggct gcggcacctg gggcggtaat tctgtgtccc aaaacgtcga accaaagcat 2280
   ctgctcaaca ttaaaagcgt cgccgaacgt cgcgagaaca tgttgtggtt caaggtcccg 2340
   caaaaaatct acttcaagta tggttgcttg cgctttgcac ttaaagagct taaggacatg 2400
   aataaaaagc gggcgttcat cgtcactgat aaggatctgt tcaaactggg ctatgttaac 2460
   aagattacca aggtcctgga tgagatcgac atcaagtatt ccatcttcac cgatattaag 2520
   tccgatccga ccattgattc cgtgaagaag ggcgcgaagg agatgctcaa ctttgaaccc 2580
   gacacgatta tttctattgg cggaggcagc ccaatggacg cagctaaggt tatgcacctg 2640
   ctgtatgagt acccagaagc agagatcgag aaccttgcaa tcaatttcat ggatattcgc 2700
   aaacgcattt gcaactttcc taagctcggt acaaaagcta tctctgttgc gatccctacc 2760
   accgcaggaa ccggcagcga agcgacacca ttcgccgtta ttaccaacga tgaaacaggt 2820
   atgaagtacc cacttacctc ttatgaactt accccgaaca tggctatcat tgatacggaa 2880
   ttgatgctga acatgccacg caagttgacc gcagctacgg gaatcgacgc attggttcat 2940
   gcaatcgagg catacgtttc cgtcatggca accgattaca ccgacgaact cgcgttgcgt 3000
   gcgattaaaa tgatcttcaa gtaccttcca cgcgcataca agaatggcac aaacgatatt 3060
   gaagcccgag aaaagatggc acacgcttcg aacatcgctg gtatggcctt cgcgaatgcg 3120
   tttctcggag tgtgtcactc catggcgcac aaactgggag ccatgcatca cgtgccccac 3180
   ggtatcgcat gcgccgttct tattgaagag gtgatcaagt ataatgccac cgattgcccc 3240
   actaagcaga cggccttccc tcagtacaaa tcgcccaatg ccaagcgtaa atacgcggaa     3300
   attgccgagt acttgaacct taaggggacc agcgacacgg aaaaggtgac cgcactgatt     3360
   gaagccatct ccaagctcaa gatcgacctg agcatcccac aaaacatctc agcagccggc     3420
   attaacaaga aggacttcta caacactctc gacaagatgt cagaactcgc cttcgatgat     3480
   cagtgcacta ccgcaaaccc acgttatccg ctcatctctg aactgaagga tatctacatc     3540
   aagtcgtttt agctcattta gcggatgatt ctcgttcaac ttcggccgaa gccacttcgt     3600
   ctgtcataat gacagggatg gtttcggccg tttttgcaaa taaaacgaaa ggctcagtcg     3660
   aaagactggg cctttcgttt tatctgttgt ttgtcggtga acgctctcct gagtaggaca     3720
   aatccgccgg gagcggattt gaacgttgcg aagcaacggc ccggagggtg gcgggcagga     3780
   cgcccgccat aaactgccag gcatcaaatt aagcagaagg ccatcctgac ggatggcctt     3840
   tttgcgtttc tacaaactct tcctgtcgtc atatctacaa gccatccccc cacagatacg     3900
   gtagctccag cttttgttcc ctttagtgag ggttaatttc gagcttggcg taatcatggt     3960
   catagctgtt tcctgtgtga aattgttatc cgctcacaat tccacacaac atacgagccg     4020
   gaagcataaa gtgtaaagcc tgggaacaac aagacccatc atagtttgcc cccgcgacat     4080
   tgaccataaa ttcatcgcac aaaatatcga acggggttta tgccgctttt agtgggtgcg     4140
   aagaatagtc tgctcattac ccgcgaacac cgccgcattc agatcacgct tagtagcgtc     4200
   cccatgagta ggcagaaccg cgtccaagtc cacatcatcc ataacgatca tgcacggggt     4260
   ggaatccaca cccagacttg ccagcacctc attagcgaca cgttgcgcag cggccacgtc     4320
   cttagcctta tccacgcaat ctaggacgta ctgcctaacc gcgaaatcag actgaatcag     4380
   tttccaatca tcgggcttca ccaaagcaac agcaacgcgg gttgattcga cccgttccgg     4440
   tgcttccaga ccggcgagct tgtacagttc ttcttccatt tcacgacgta catcagcgtc     4500
   tatgtaatca atgcccaaag cacgcttagc cccacgtgac caggacgaac gcaggttttt     4560
   agaaccaacc tcatactcac gccaccgagc caccaaaaca gcgtccatat cctcgccggc     4620
   gtcgctttga tcggccaaca tatccaacat ctgaaacggc gtgtacgacc ccttagacgc     4680
   ggttttagta gcggagccag tcagttcctg agacatgccc ttagcgaggt aggttgccat     4740
   tttcgcagcg tctccacccc aggtagacac ctgatcaagt ttgaccccgt gctcacgcag     4800
   tggcgcgtcc ataccggcct taaccacacc agcagaccag cgggaaaaca tggaatcctc     4860
   aaacgccttg agttcatcgt cagacagtgg acgatccaag aacaacagca tgttgcggtg     4920
   caagtgccaa ccgttcgccc aagagtctgt gacctcatag tcactatagg tgtgctccac     4980
   cccgtaccgt gcacgttctt tcttccactg agatgttttc accatcgaag agtacgcagt     5040
   cttaataccc gcttcaacct gcgcaaatga ctgtgagcgg ttgtgtcgaa cagtgcccac     5100
   aaacatcatg agcgcgccac ccgccgccaa gtgattctta gtagcaatag ccagctcaat 5160
   gcggcgttcg cccatgactt ccaattcagc cagaggtgac ccccagcgag agtgagagtt 5220
   ttgcagaccc tcaaactgcg aagcaccgtt agacgaccag gacaccgcaa cagcttcgtc 5280
   cctgcgccac ctatggcacc ccgccagagc cttactattg gtgatcttgt acatgacgtt 5340
   ttgcctacgc cacgccctag cgcgagtgac cttagaaccc tcattgacct gcggttcctt 5400
   agaggtgttc acttctattt cagtgttacc tagacccgat gttgtgcggg gttgcgcagt 5460
   gcgagtttgt gcgggtgttg tgcccgttgt cttagctagt gctatggttg tcaattgaaa 5520
   ccccttcggg ttatgtggcc cccgtgcata tgagttggta gctcgcacgg gggtttgtct 5580
   tgtctaggaa ctattaattt ttagtggtgt ttggtggccg cctagcttgg ctatgcgtgc 5640
   cagcttaccc gtactcaatg ttaaagattt gcatcgacat gggagggtta cgtgtccgat 5700
   acctaggggg ggtatccgcg actaggtgcc ccggtgctca ctgtctgtac cggcggggca 5760
   agccccacac cccgcatgga cagggtggct ccgccccctg cacccccagc aatctgcatg 5820
   tacatgtttt acacattagc acgacatgac tgcatgtgca tgcactgcat gcagactagg 5880
   taaatatgag tatgtacgac tagtaacagg agcactgcac ataatgaatg agttgcagga 5940
   caatgtttgc tacgcatgcg catgacatat cgcaggaaag ctactagagt cttaaagcat 6000
   ggcaaccaag gcacagctag aacagcaact acaagaagct caacaggcac tacaggcgca 6060
   gcaagcgcag gcacaagcca ccatcgaagc actagaagcg caggcaaagg ctaagcccgt 6120
   cgtggtcacc gcacgcgttc ctttggcact acgtgaggac atgaagcgcg caggcatgca 6180
   gaacggtgaa aacctccaag agttcatgat cgccgcgttt accgagcggc tagaaaagct 6240
   caccaccacc gacaacgagg aaaacaatgt ctaacccact agttctcttt gcccaccgtg 6300
   acccggtaaa tgacgtgacg ttcgagtgca ttgagcacgc cacctacgac acactttcac 6360
   acgctaaaga ccagatcacc gcccaaatgc aagccctaga cgaagaagcc gccctactgc 6420
   cctaatgggt gtttcatggg tgtttcccta gtgtttcatg gtgttttcac ctaagctagg 6480
   gaattgcgcg agaagtctcg caaaaatcag caacccccgg aaccacacag ttcacggggg 6540
   ttcttctatg ccagaaatca gaaaggggaa ccagtgaacg accccgaata ttggatcaca 6600
   gcgcagcagg tcgccgcccg cgtagctctc accccggcca ccattaaaaa gtgggcaaac 6660
   gagggaaaaa tcaccgcata caagatcggc aagtccgtcc gattcaaagc atcagacgta 6720
   gacaagctag gggggggggg gcgctgaggt ctgcctcgtg aagaaggtgt tgctgactca 6780
   taccaggcct gaatcgcccc atcatccagc cagaaagtga gggagccacg gttgatgaga 6840
   gctttgttgt aggtggacca gttggtgatt ttgaactttt gctttgccac ggaacggtct 6900
   gcgttgtcgg gaagatgcgt gatctgatcc ttcaactcag caaaagttcg atttattcaa 6960
   caaagccgcc gtcccgtcaa gtcagcgtaa tgctctgcca gtgttacaac caattaacca 7020
   attctgatta gaagaactcg tcaagaaggc gatagaaggc gatgcgctgc gaatcgggag 7080
   cggcgatacc gtaaagcacg aggaagcggt cagcccattc gccgccaagc tcttcagcaa 7140
   tatcacgggt agccaacgct atgtcctgat agcggtccgc cacacccagc cggccacagt 7200
   cgatgaatcc agaaaagcgg ccattttcca ccatgatatt cggcaagcag gcatcgccat 7260
   gggtcacgac gagatcctcg ccgtcgggca tgctcgcctt gagcctggcg aacagttcgg 7320
   ctggcgcgag cccctgatgc tcttcgtcca gatcatcctg atcgacaaga ccggcttcca 7380
   tccgagtacg tgctcgctcg atgcgatgtt tcgcttggtg gtcgaatggg caggtagccg 7440
   gatcaagcgt atgcagccgc cgcattgcat cagccatgat ggatactttc tcggcaggag 7500
   caaggtgaga tgacaggaga tcctgccccg gcacttcgcc caatagcagc cagtcccttc 7560
   ccgcttcagt gacaacgtcg agcacagctg cgcaaggaac gcccgtcgtg gccagccacg 7620
   atagccgcgc tgcctcgtct tgcagttcat tcagggcacc ggacaggtcg gtcttgacaa 7680
   aaagaaccgg gcgcccctgc gctgacagcc ggaacacggc ggcatcagag cagccgattg 7740
   tctgttgtgc ccagtcatag ccgaatagcc tctccaccca agcggccgga gaacctgcgt 7800
   gcaatccatc ttgttcaatc ataacacccc ttgtattact gtttatgtaa gcagacagtt 7860
   ttattgttca tgatgatata tttttatctt gtgcaatgta acatcagaga ttttgagaca 7920
   caacgtggct ttcccccccc ccccaaaagg atctaggtga agatcctttt tgataatctc 7980
   atgaccaaaa tcccttaacg tgagttttcg ttccactgag cgtcagaccc cgtagaaaag 8040
   atcaaaggat cttc 8054
<210> 75
   <211> 48
   <212> DNA
   <213> Artificial Sequence
<220>
   <223> P5
<400> 75
   ttcggagtct agatttccta caatctttag aggagacaca acatgaaa 48
<210> 76
   <211> 47
   <212> DNA
   <213> Artificial Sequence
<220>
   <223> P6
<400> 76
   ccgctaaatg aattctaaaa cgacttgatg tagatatcct tcagttc 47
<210> 77
   <211> 42
   <212> DNA
   <213> Artificial Sequence
<220>
   <223> P7
<400> 77
   cgttttagaa ttcatttagc ggatgattct cgttcaactt cg 42
<210> 78
   <211> 43
   <212> DNA
   <213> Artificial Sequence
<220>
   <223> P8
<400> 78
   gaacaaaagc tggagctacc gtatctgtgg ggggatggct tgt 43
<210> 79
   <211> 20
   <212> DNA
   <213> Artificial Sequence
<220>
   <223> P9 primer
<400> 79
   accgtatctg tggggggatg 20
<210> 80
   <211> 8031
   <212> DNA
   <213> Artificial Sequence
<220>
   <223> pG2G vector
<400> 80
   ttgagatcct ttttttctgc gcgtaatctg ctgcttgcaa acaaaaaaac caccgctacc      60
   agcggtggtt tgtttgccgg atcaagagct accaactctt tttccgaagg taactggctt     120
   cagcagagcg cagataccaa atactgttct tctagtgtag ccgtagttag gccaccactt     180
   caagaactct gtagcaccgc ctacatacct cgctctgcta atcctgttac cagtggctgc     240
   tgccagtggc gataagtcgt gtcttaccgg gttggactca agacgatagt taccggataa     300
   ggcgcagcgg tcgggctgaa cggggggttc gtgcacacag cccagcttgg agcgaacgac     360
   ctacaccgaa ctgagatacc tacagcgtga gctatgagaa agcgccacgc ttcccgaagg     420
   gagaaaggcg gacaggtatc cggtaagcgg cagggtcgga acaggagagc gcacgaggga     480
   gcttccaggg ggaaacgcct ggtatcttta tagtcctgtc gggtttcgcc acctctgact     540
   tgagcgtcga tttttgtgat gctcgtcagg ggggcggagc ctatggaaaa acgcccctct      600
   tcgctattac gccagctggc gaaaggggga tgtgctgcaa ggcgattaag ttgggtaacg 660
   ccagggtttt cccagtcacg acgttgtaaa acgacggcca gtgaattgta atacgactca 720
   ctatagggcg aattgggtac catgattttg catctgctgc gaaatctttg tttccccgct 780
   aaagttgagg acaggttgac acggagttga ctcgacgaat tatccaatgt gagtaggttt 840
   ggtgcgtgag ttggaaaaat tcgccatact cgcccttggg ttctgtcagc tcaagaattc 900
   ttgagtgacc gatgctctga ttgacctaac tgcttgacac attgcatttc ctacaatctt 960
   tagaggagac acaacctcga ggtcgacggt atcgataagc ttgatatcga attccatgga 1020
   gattaaagag atggtcagtc ttgcgcgcaa agctcagaag gagtatcagg ccacccataa 1080
   ccaagaagct gtggacaaca tctgccgagc tgcagcgaag gttatttacg aaaatgcagc 1140
   aattctggcc cgcgaggcag tggacgaaac cggcatgggt gtttacgagc acaaggtggc 1200
   caagaatcaa ggcaagtcca aaggtgtttg gtacaacctg cataacaaga agtcgattgg 1260
   catcctcaat atcgatgagc gtaccggcat gatcgagatc gcaaaaccta tcggggttgt 1320
   aggcgccgtt acgccaacca ccaaccctat cgttactccg atgagcaaca tcatctttgc 1380
   tcttaagacc tgcaacgcca tcattatcgc ccccacaccc cgctccaaaa agtgctctgc 1440
   ccacgcagtt cggctgatca aagaggctat cgctccgttc aacgtgcccg aaggtatggt 1500
   tcagatcatc gaggagccta gcatcgagaa gacgcaggaa ttgatgggcg ccgtagacgt 1560
   ggtcgttgct accgggggca tgggcatggt caagtctgcc tactcctcag ggaagccttc 1620
   tttcggtgtc ggagccggca atgttcaggt gatagtggac agcaacatcg atttcgaagc 1680
   ggctgcagaa aagatcatca ccggacgtgc cttcgacaac ggtatcatct gctcaggcga 1740
   acagtccatc atctacaacg aggctgacaa ggaagcagtt ttcacagcat tccgcaacca 1800
   cggtgcgtac ttttgcgacg aggccgaggg agatcgggct cgtgcagcga tcttcgaaaa 1860
   tggagccatc gcgaaagatg ttgtgggcca gtccgttgcc tttattgcca agaaggcgaa 1920
   cattaatatc cccgagggta ctcgtattct cgtggtcgaa gctcgcggag taggcgccga 1980
   agatgtcatc tgtaaagaaa agatgtgtcc agtcatgtgc gccctctcct acaagcactt 2040
   cgaagagggg gtagagatcg caaggacgaa cctcgcaaac gaaggcaatg gccatacctg 2100
   tgctatccac tccaacaacc aagcacacat catcttggca ggctcggagc tgaccgtgtc 2160
   tcgcatcgtg gtcaacgcgc caagtgctac cacagcaggc ggtcacatcc agaacggtct 2220
   tgccgtcacc aatactctag gctgcggctc ttggggtaac aactcgatct ccgaaaactt 2280
   cacttataaa cacctgctca acatttcacg catcgccccg ttgaactcca gcattcatat 2340
   cccagatgat aaggaaatct gggaactcta attagaggag acacaacatg cagcttttca 2400
   agctcaagag cgtcacacat cactttgata cttttgcaga gtttgccaag gaattctgtc 2460
   tcggtgaacg cgacttggta attaccaacg agttcatcta cgaaccgtat atgaaggcat 2520
   gccagctgcc ttgtcatttt gtgatgcagg agaaatacgg ccaaggcgag ccttctgacg 2580
   agatgatgaa caacatccta gcagatatcc gtaatatcca gttcgaccgc gtgatcggga 2640
   tcggaggtgg tacggttatt gacatctcaa aactctttgt tctgaaggga ttaaatgatg 2700
   ttctcgacgc gttcgatcgc aagattcccc ttatcaaaga gaaagaactg atcattgtgc 2760
   ccaccacctg cggaaccggc tcggaggtga cgaacatttc catcgccgag atcaagtccc 2820
   ggcacaccaa gatgggtttg gctgacgatg caattgttgc tgaccacgcc ataatcatcc 2880
   ctgaacttct gaagagcttg cccttccact tctatgcatg ctccgcaatc gatgctctta 2940
   ttcatgccat cgagtcatac gtttctccaa aagcgtctcc atactcccgt ctgttcagtg 3000
   aggcggcgtg ggacattatc ctggaagttt tcaagaaaat cgccgaacac ggcccagagt 3060
   accgcttcga gaagctgggg gaaatgatca tggccagcaa ctatgccggt atcgctttcg 3120
   gcaacgcagg cgttggcgcc gtccacgctc tatcctaccc gttgggcggc aactatcacg 3180
   tgccgcatgg agaagcaaac tatcagttct tcaccgaggt ctttaaagta taccaaaaga 3240
   agaatccgtt cggctatatt gtcgaactca actggaagct ctccaagatt ctgaactgcc 3300
   agccagagta cgtgtacccg aagctggatg aactgctcgg ttgccttctt accaagaaac 3360
   ctttgcacga atacggcatg aaggacgaag aggttcgtgg cttcgcggaa tcggtcctga 3420
   agacccagca acgcttgctc gccaacaact acgtcgaact tactgtcgat gagatcgaag 3480
   gtatctaccg acgtctctac taactagagc ggccgccacc gcggtggagc tcatttagcg 3540
   gatgattctc gttcaacttc ggccgaagcc acttcgtctg tcataatgac agggatggtt 3600
   tcggccgttt ttgcaaataa aacgaaaggc tcagtcgaaa gactgggcct ttcgttttat 3660
   ctgttgtttg tcggtgaacg ctctcctgag taggacaaat ccgccgggag cggatttgaa 3720
   cgttgcgaag caacggcccg gagggtggcg ggcaggacgc ccgccataaa ctgccaggca 3780
   tcaaattaag cagaaggcca tcctgacgga tggccttttt gcgtttctac aaactcttcc 3840
   tgtcgtcata tctacaagcc atccccccac agatacggta gctccagctt ttgttccctt 3900
   tagtgagggt taatttcgag cttggcgtaa tcatggtcat agctgtttcc tgtgtgaaat 3960
   tgttatccgc tcacaattcc acacaacata cgagccggaa gcataaagtg taaagcctgg 4020
   gaacaacaag acccatcata gtttgccccc gcgacattga ccataaattc atcgcacaaa 4080
   atatcgaacg gggtttatgc cgcttttagt gggtgcgaag aatagtctgc tcattacccg 4140
   cgaacaccgc cgcattcaga tcacgcttag tagcgtcccc atgagtaggc agaaccgcgt 4200
   ccaagtccac atcatccata acgatcatgc acggggtgga atccacaccc agacttgcca 4260
   gcacctcatt agcgacacgt tgcgcagcgg ccacgtcctt agccttatcc acgcaatcta 4320
   ggacgtactg cctaaccgcg aaatcagact gaatcagttt ccaatcatcg ggcttcacca 4380
   aagcaacagc aacgcgggtt gattcgaccc gttccggtgc ttccagaccg gcgagcttgt 4440
   acagttcttc ttccatttca cgacgtacat cagcgtctat gtaatcaatg cccaaagcac 4500
   gcttagcccc acgtgaccag gacgaacgca ggtttttaga accaacctca tactcacgcc 4560
   accgagccac caaaacagcg tccatatcct cgccggcgtc gctttgatcg gccaacatat 4620
   ccaacatctg aaacggcgtg tacgacccct tagacgcggt tttagtagcg gagccagtca 4680
   gttcctgaga catgccctta gcgaggtagg ttgccatttt cgcagcgtct ccaccccagg 4740
   tagacacctg atcaagtttg accccgtgct cacgcagtgg cgcgtccata ccggccttaa 4800
   ccacaccagc agaccagcgg gaaaacatgg aatcctcaaa cgccttgagt tcatcgtcag 4860
   acagtggacg atccaagaac aacagcatgt tgcggtgcaa gtgccaaccg ttcgcccaag 4920
   agtctgtgac ctcatagtca ctataggtgt gctccacccc gtaccgtgca cgttctttct 4980
   tccactgaga tgttttcacc atcgaagagt acgcagtctt aatacccgct tcaacctgcg 5040
   caaatgactg tgagcggttg tgtcgaacag tgcccacaaa catcatgagc gcgccacccg 5100
   ccgccaagtg attcttagta gcaatagcca gctcaatgcg gcgttcgccc atgacttcca 5160
   attcagccag aggtgacccc cagcgagagt gagagttttg cagaccctca aactgcgaag 5220
   caccgttaga cgaccaggac accgcaacag cttcgtccct gcgccaccta tggcaccccg 5280
   ccagagcctt actattggtg atcttgtaca tgacgttttg cctacgccac gccctagcgc 5340
   gagtgacctt agaaccctca ttgacctgcg gttccttaga ggtgttcact tctatttcag 5400
   tgttacctag acccgatgtt gtgcggggtt gcgcagtgcg agtttgtgcg ggtgttgtgc 5460
   ccgttgtctt agctagtgct atggttgtca attgaaaccc cttcgggtta tgtggccccc 5520
   gtgcatatga gttggtagct cgcacggggg tttgtcttgt ctaggaacta ttaattttta 5580
   gtggtgtttg gtggccgcct agcttggcta tgcgtgccag cttacccgta ctcaatgtta 5640
   aagatttgca tcgacatggg agggttacgt gtccgatacc tagggggggt atccgcgact 5700
   aggtgccccg gtgctcactg tctgtaccgg cggggcaagc cccacacccc gcatggacag 5760
   ggtggctccg ccccctgcac ccccagcaat ctgcatgtac atgttttaca cattagcacg 5820
   acatgactgc atgtgcatgc actgcatgca gactaggtaa atatgagtat gtacgactag 5880
   taacaggagc actgcacata atgaatgagt tgcaggacaa tgtttgctac gcatgcgcat 5940
   gacatatcgc aggaaagcta ctagagtctt aaagcatggc aaccaaggca cagctagaac 6000
   agcaactaca agaagctcaa caggcactac aggcgcagca agcgcaggca caagccacca 6060
   tcgaagcact agaagcgcag gcaaaggcta agcccgtcgt ggtcaccgca cgcgttcctt 6120
   tggcactacg tgaggacatg aagcgcgcag gcatgcagaa cggtgaaaac ctccaagagt 6180
   tcatgatcgc cgcgtttacc gagcggctag aaaagctcac caccaccgac aacgaggaaa 6240
   acaatgtcta acccactagt tctctttgcc caccgtgacc cggtaaatga cgtgacgttc 6300
   gagtgcattg agcacgccac ctacgacaca ctttcacacg ctaaagacca gatcaccgcc 6360
   caaatgcaag ccctagacga agaagccgcc ctactgccct aatgggtgtt tcatgggtgt 6420
   ttccctagtg tttcatggtg ttttcaccta agctagggaa ttgcgcgaga agtctcgcaa 6480
   aaatcagcaa cccccggaac cacacagttc acgggggttc ttctatgcca gaaatcagaa 6540
   aggggaacca gtgaacgacc ccgaatattg gatcacagcg cagcaggtcg ccgcccgcgt 6600
   agctctcacc ccggccacca ttaaaaagtg ggcaaacgag ggaaaaatca ccgcatacaa 6660
   gatcggcaag tccgtccgat tcaaagcatc agacgtagac aagctagggg ggggggggcg 6720
   ctgaggtctg cctcgtgaag aaggtgttgc tgactcatac caggcctgaa tcgccccatc 6780
   atccagccag aaagtgaggg agccacggtt gatgagagct ttgttgtagg tggaccagtt 6840
   ggtgattttg aacttttgct ttgccacgga acggtctgcg ttgtcgggaa gatgcgtgat 6900
   ctgatccttc aactcagcaa aagttcgatt tattcaacaa agccgccgtc ccgtcaagtc 6960
   agcgtaatgc tctgccagtg ttacaaccaa ttaaccaatt ctgattagaa gaactcgtca 7020
   agaaggcgat agaaggcgat gcgctgcgaa tcgggagcgg cgataccgta aagcacgagg 7080
   aagcggtcag cccattcgcc gccaagctct tcagcaatat cacgggtagc caacgctatg 7140
   tcctgatagc ggtccgccac acccagccgg ccacagtcga tgaatccaga aaagcggcca 7200
   ttttccacca tgatattcgg caagcaggca tcgccatggg tcacgacgag atcctcgccg 7260
   tcgggcatgc tcgccttgag cctggcgaac agttcggctg gcgcgagccc ctgatgctct 7320
   tcgtccagat catcctgatc gacaagaccg gcttccatcc gagtacgtgc tcgctcgatg 7380
   cgatgtttcg cttggtggtc gaatgggcag gtagccggat caagcgtatg cagccgccgc 7440
   attgcatcag ccatgatgga tactttctcg gcaggagcaa ggtgagatga caggagatcc 7500
   tgccccggca cttcgcccaa tagcagccag tcccttcccg cttcagtgac aacgtcgagc 7560
   acagctgcgc aaggaacgcc cgtcgtggcc agccacgata gccgcgctgc ctcgtcttgc 7620
   agttcattca gggcaccgga caggtcggtc ttgacaaaaa gaaccgggcg cccctgcgct 7680
   gacagccgga acacggcggc atcagagcag ccgattgtct gttgtgccca gtcatagccg 7740
   aatagcctct ccacccaagc ggccggagaa cctgcgtgca atccatcttg ttcaatcata 7800
   acaccccttg tattactgtt tatgtaagca gacagtttta ttgttcatga tgatatattt 7860
   ttatcttgtg caatgtaaca tcagagattt tgagacacaa cgtggctttc cccccccccc 7920
   caaaaggatc taggtgaaga tcctttttga taatctcatg accaaaatcc cttaacgtga 7980
   gttttcgttc cactgagcgt cagaccccgt agaaaagatc aaaggatctt c 8031
<210> 81
   <211> 42
   <212> DNA
   <213> Artificial Sequence
<220>
   <223> P10 primer
<400> 81
   catcccccca cagatacggt atgattttgc atctgctgcg aa 42
<210> 82
   <211> 502
   <212> PRT
   <213> Corynebacterium glutamicum
<400> 82
   Met Ser Asp Arg Ile Ala Ser Glu Lys Leu Arg Ser Lys Leu Met Ser
   1               5                   10                  15
   Ala Asp Glu Ala Ala Gln Phe Val Asn His Gly Asp Lys Val Gly Phe
               20                  25                  30
   Ser Gly Phe Thr Gly Ala Gly Tyr Pro Lys Ala Leu Pro Thr Ala Ile
           35                  40                  45
   Ala Asn Arg Ala Lys Glu Ala His Gly Ala Gly Asn Asp Tyr Ala Ile
       50                  55                  60
   Asp Leu Phe Thr Gly Ala Ser Thr Ala Pro Asp Cys Asp Gly Val Leu
   65                  70                  75                  80
   Ala Glu Ala Asp Ala Ile Arg Trp Arg Met Pro Tyr Ala Ser Asp Pro
                   85                  90                  95
   Ile Met Arg Asn Lys Ile Asn Ser Gly Ser Met Gly Tyr Ser Asp Ile
               100                 105                 110
   His Leu Ser His Ser Gly Gln Gln Val Glu Glu Gly Phe Phe Gly Gln
           115                 120                 125
   Leu Asn Val Ala Val Ile Glu Ile Thr Arg Ile Thr Glu Glu Gly Tyr
       130                 135                 140
   Ile Ile Pro Ser Ser Ser Val Gly Asn Asn Val Glu Trp Leu Asn Ala
   145                 150                 155                 160
   Ala Glu Lys Val Ile Leu Glu Val Asn Ser Trp Gln Ser Glu Asp Leu
                   165                 170                 175
   Glu Gly Met His Asp Ile Trp Ser Val Pro Ala Leu Pro Asn Arg Ile
               180                 185                 190
   Ala Val Pro Ile Asn Lys Pro Gly Asp Arg Ile Gly Lys Thr Tyr Ile
           195                 200                 205
   Glu Phe Asp Thr Asp Lys Val Val Ala Val Val Glu Thr Asn Thr Ala
       210                 215                 220
   Asp Arg Asn Ala Pro Phe Lys Pro Val Asp Asp Ile Ser Lys Lys Ile
   225                 230                 235                 240
   Ala Gly Asn Phe Leu Asp Phe Leu Glu Ser Glu Val Ala Ala Gly Arg
                   245                 250                 255
   Leu Ser Tyr Asp Gly Tyr Ile Met Gln Ser Gly Val Gly Asn Val Pro
               260                 265                 270
   Asn Ala Val Met Ala Gly Leu Leu Glu Ser Lys Phe Glu Asn Ile Gln
           275                 280                 285
   Ala Tyr Thr Glu Val Ile Gln Asp Gly Met Val Asp Leu Ile Asp Ala
       290                 295                 300
   Gly Lys Met Thr Val Ala Ser Ala Thr Ser Phe Ser Leu Ser Pro Glu
   305                 310                 315                 320
   Tyr Ala Glu Lys Met Asn Asn Glu Ala Lys Arg Tyr Arg Glu Ser Ile
                   325                 330                 335
   Ile Leu Arg Pro Gln Gln Ile Ser Asn His Pro Glu Val Ile Arg Arg
               340                 345                 350
   Val Gly Leu Ile Ala Thr Asn Gly Leu Ile Glu Ala Asp Ile Tyr Gly
           355                 360                 365
   Asn Val Asn Ser Thr Asn Val Ser Gly Ser Arg Val Met Asn Gly Ile
       370                 375                 380
   Gly Gly Ser Gly Asp Phe Thr Arg Asn Gly Tyr Ile Ser Ser Phe Ile
   385                 390                 395                 400
   Thr Pro Ser Glu Ala Lys Gly Gly Ala Ile Ser Ala Ile Val Pro Phe
                   405                 410                 415
   Ala Ser His Ile Asp His Thr Glu His Asp Val Met Val Val Ile Ser
               420                 425                 430
   Glu Tyr Gly Tyr Ala Asp Leu Arg Gly Leu Ala Pro Arg Glu Arg Val
           435                 440                 445
   Ala Lys Met Ile Gly Leu Ala His Pro Asp Tyr Arg Pro Leu Leu Glu
       450                 455                 460
   Glu Tyr Tyr Ala Arg Ala Thr Ser Gly Asp Asn Lys Tyr Met Gln Thr
   465                 470                 475                 480
   Pro His Asp Leu Ala Thr Ala Phe Asp Phe His Ile Asn Leu Ala Lys
                   485                 490                 495
   Asn Gly Ser Met Lys Ala
               500
<210> 83
   <211> 1509
   <212> DNA
   <213> Corynebacterium glutamicum
<400> 83
   atgtctgatc gcattgcttc agaaaagctg cgctccaagc tcatgtccgc cgacgaggcg      60
   gcacagtttg ttaaccacgg tgacaaggtt ggtttctccg gcttcaccgg cgctggctac     120
   ccaaaggcac tgcctacggc aatcgctaac cgggctaaag aagcacacgg tgcaggcaac     180
   gactacgcaa tcgacctgtt cactggcgca tcgaccgccc ctgactgcga tggcgtactt     240
   gcagaagctg acgctatccg ctggcgcatg ccatacgcat ctgatccaat catgcgtaac     300
   aagatcaact ccggctccat gggatactcc gatatccacc tgtcccactc cggccagcag     360
   gttgaagagg gcttcttcgg ccagctcaac gtagctgtca ttgaaatcac ccgcatcact     420
   gaagagggct acatcatccc ttcttcctcc gtgggtaaca acgttgagtg gctcaacgct     480
   gcagagaagg tcatcctcga ggttaactct tggcagtctg aagacctcga aggtatgcac     540
   gacatctggt ctgttcctgc cctgccaaac cgcattgccg tgccaatcaa caagccaggc     600
   gaccgcatcg gtaagaccta catcgagttc gacaccgaca aggttgttgc tgttgttgag     660
   accaacaccg cagaccgcaa cgcaccattc aagcctgtcg acgacatctc taagaagatc     720
   gctggcaact tcctcgactt cctggaaagc gaagttgctg caggtcgcct gtcctacgac     780
   ggctacatca tgcagtccgg cgtgggcaac gtgccaaacg cggtgatggc aggcctgctg     840
   gaatccaagt ttgagaacat ccaggcctac accgaagtta tccaggacgg catggtggac     900
   ctcatcgacg ccggcaagat gaccgttgca tccgcaactt ccttctccct gtctcctgag     960
   tacgcagaga agatgaacaa cgaggctaag cgttaccgcg agtccattat cctgcgccca    1020
   cagcagatct ctaaccaccc agaggtcatc cgccgcgttg gcctgatcgc caccaacggt    1080
   ctcatcgagg ctgacattta cggcaacgtc aactccacca acgtttctgg ctcccgcgtc    1140
   atgaacggca tcggcggctc cggcgacttc acccgtaacg gctacatctc cagcttcatc    1200
   accccttcag aggcaaaggg cggcgcaatc tctgcgatcg ttcctttcgc atcccacatc    1260
   gaccacaccg agcacgatgt catggttgtt atctctgagt acggttacgc agaccttcgt    1320
   ggtctggctc cacgtgagcg cgttgccaag atgatcggcc tggctcaccc tgattaccgc    1380
   ccactgctcg aggagtacta cgctcgcgca acctccggtg acaacaagta catgcagacc    1440
   cctcatgatc ttgcaaccgc gtttgatttc cacatcaacc tggctaagaa cggctccatg    1500
   aaggcataa                                                            1509
<210> 84
   <211> 125
   <212> PRT
   <213> Corynebacterium glutamicum
<400> 84
   Met Asn Gly Ile Gly Gly Ser Gly Asp Phe Thr Arg Asn Ala Phe Ala
   1               5                   10                  15
   Ser Thr Phe Ile Ser Pro Ser Ala Ala Lys Val Asp Ala Ile Ser Ala
               20                  25                  30
   Ile Val Pro Phe Ala Ser His Ile Asp His Thr Glu His Asp Ala Met
           35                  40                  45
   Val Val Ile Thr Glu Tyr Gly Tyr Ala Asp Leu Arg Gly Leu Ser Pro
       50                  55                  60
   Lys Gln Arg Val Pro Lys Met Ile Ala Ile Ala His Pro Asp Tyr Arg
   65                  70                  75                  80
   Pro Leu Leu Glu Ala Tyr Phe Asp Arg Ala Leu Asn Ser Ala Asp Ser
                   85                  90                  95
   Tyr Gln His Thr Leu His Asp Leu Arg Thr Ala Phe Asp Phe His Asn
               100                 105                 110
   Arg Leu Asn Ser Gln Gly Thr Met Lys Ile Glu Lys Ala
           115                 120                 125
<210> 85
   <211> 378
   <212> DNA
   <213> Corynebacterium glutamicum
<400> 85
   atgaatggta tcggcggctc gggcgatttc acgcgtaacg cctttgcttc cacatttatc      60
   tctccctcgg cagccaaagt tgatgcgatt tccgcgattg tgcctttcgc gtcccatatc     120
   gatcacacgg aacatgatgc gatggttgtc attactgaat atggctacgc agacctgcgc     180
   gggctatcgc caaaacaacg agtccccaaa atgattgcca tcgcccaccc ggactatcga     240
   ccactgctgg aagcatactt tgaccgggcg ctgaacagtg ctgattccta tcagcacacc     300
   ctgcatgatc tgcgcaccgc cttcgatttc cataatcgct tgaactcaca aggaaccatg     360
   aaaatcgaaa aagcatag                                                   378